# EUROPEAN PATENT SPECIFICATION

(11) **EP 2 978 040 B1**
(45) Date of publication and mention of the grant of the patent: **27.12.2017**
(21) Application number: 15176812.4
(22) Date of filing: 15.07.2015
(51) Int. Cl.: H01L 51/54

(54) **ORGANIC LIGHT-EMITTING DEVICE**
ORGANISCHE LICHTEMITTIERENDE VORRICHTUNG
DISPOSITIF ÉLECTROLUMINESCENT ORGANIQUE

(30) Priority: 22.07.2014 KR 20140092665; 24.02.2015 KR 20150025913
(43) Date of publication of application: 27.01.2016
(73) Proprietor: Samsung Display Co., Ltd., Gyeonggi-do (KR)
(72) Inventor: KIM, Myeongsuk, Gyeonggi-Do (KR); KIM, Sungwook, Gyeonggi-Do (KR); KIM, Jaebok, Gyeonggi-Do (KR); SONG, Wonjun, Gyeonggi-Do (KR); IM, Jahyun, Gyeonggi-Do (KR); KIM, Taekyung, Gyeonggi-Do (KR)
(74) Representative: Gulde & Partner

(56) References cited:
- EP-A1- 2 891 648
- WO-A1-2015/082056
- WO-A2-2011/133007
- WO-A2-2013/032304
- KR-A- 20140 045 154
- US-A1- 2007 082 226
- US-A1- 2014 054 559
- US-A1- 2014 175 398
- NAOKI MATSUMOTO ET AL: "Exciplex Formations at the HTL/Alq 3 Interface in an Organic Light-Emitting Diode: Influence of the Electron-Hole Recombination Zone and Electric Field", JOURNAL OF PHYSICAL CHEMISTRY C, vol. 114, no. 10, 18 March 2010 (2010-03-18), pages 4652-4658, XP055230582, ISSN: 1932-7447, DOI: 10.1021/jp9121062
- KE-FENG SHAO ET AL: "High Tg Fluorene-based Hole-transporting Materials for Organic Light-emitting Diodes", CHEMISTRY LETTERS, vol. 34, no. 12, 5 December 2005 (2005-12-05), pages 1604-1605, XP055230588, JAPAN ISSN: 0366-7022, DOI: 10.1246/cl.2005.1604

## Description

### BACKGROUND

### 1. Field

One or more aspects of embodiments of the present invention relate to an organic light-emitting device.

### 2. Description of the Related Art

Organic light-emitting devices are self-emitting devices that have wide viewing angles, high contrast ratios, short response times, and excellent luminance, driving voltage, and response speed characteristics, and can produce full-color images.

An organic light-emitting device may include a first electrode positioned on a substrate, and a hole transport region, an emission layer, an electron transport region, and a second electrode sequentially formed on the first electrode. Holes injected from the first electrode are transported to the emission layer through the hole transport region, and electrons injected from the second electrode are transported to the emission layer through the electron transport region. Carriers (e.g., holes and electrons), are then recombined in the emission layer to generate excitons. When the excitons drop from an excited state to a ground state, light is emitted.

WO 2015/082056 A1, US 2014/175398 A1, KR 2014 0045154 A, US 2007/082226 A1, US 2014/054559 A1, WO 2013/032304 A2, WO 2011/133007 A2, Naoki Matsumoto et al. (Journal of Physical Chemistry C, vol. 114, no. 10, pages 4652 - 4658), and Ke-Feng Shao et al. (Chemistry Letters, vol. 34, no. 12, pages 1604 - 1605) disclose the use of exemplary amine-based compounds in organic layers of organic light-emitting devices.

### SUMMARY

The present invention is directed to an organic light-emitting device as defined in claim 1.

Additional aspects will be set forth in part in the description which follows and, in part, will be apparent from the description, or may be learned by practice of the presented embodiments.

### BRIEF DESCRIPTION OF THE DRAWINGS

These and/or other aspects will become apparent and more readily appreciated from the following description of the embodiments, taken in conjunction with the accompanying drawings in which:
FIG. 1 is a schematic cross-sectional view of a structure of an organic light-emitting device according to one or more embodiments of the present inventive concept;
FIG. 2 is a schematic cross-sectional view of a structure of an organic light-emitting device according to one or more embodiments of the present inventive concept;
FIG. 3 is a schematic cross-sectional view of a structure of a full-color organic light-emitting device according to one or more embodiments of the present inventive concept; and
FIG. 4 shows a graph of log J verses current efficiency according to one or more embodiments of the present inventive concept.

### DETAILED DESCRIPTION

Reference will now be made in more detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of" or "at least one selected from", when preceding a list of elements, modify the entire list of elements and do not modify the individual elements of the list. Further, the use of "may" when describing embodiments of the present invention refers to "one or more embodiments of the present invention."

Like reference numerals in the drawings denote like elements, and duplicative descriptions will not be provided.

As used herein, the singular forms "a", "an", and "the" are intended to include the plural forms as well, unless the context clearly indicates otherwise.

It will be further understood that the terms "comprises" and/or "comprising" used herein specify the presence of stated features or components, but do not preclude the presence or addition of one or more other features or components. In addition, as used herein, the terms "use," "using," and "used" may be considered synonymous with the terms "utilize," "utilizing," and "utilized," respectively.

It will be understood that when a layer, region, or component is referred to as being "formed on" another layer, region, or component, it can be directly or indirectly formed on the other layer, region, or component. That is, for example, intervening layers, regions, or components may be present.

Sizes of components in the drawings may be exaggerated for convenience of explanation. In other words, since sizes and thicknesses of components in the drawings are arbitrarily illustrated for convenience of explanation, the following embodiments are not limited thereto.

As used herein, the expression " wherein the auxiliary layer comprises an amine-based compound represented by Formula 1 " herein indicates that the auxiliary layer may include one or more amine-based compounds of Formula 1.

As used herein, the term "organic layer" refers to a single layer and/or a plurality of layers between the first electrode and the second electrode in an organic light-emitting device. A material included in the "organic layer" is not limited to an organic material.

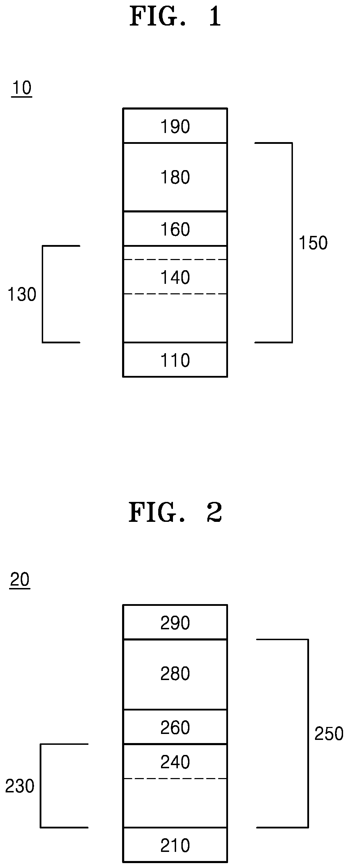

FIG. 1 is a schematic view of a structure of an organic light-emitting device according to one or more embodiments of the present inventive concept.

Referring to FIG. 1, a substrate may be positioned under a first electrode 110 or on a second electrode 190. The substrate may be a glass substrate or transparent plastic substrate, each with excellent mechanical strength, thermal stability, transparency, surface smoothness, ease of handling, and/or water resistance.

For example, the first electrode 110 may be formed by depositing or sputtering a first electrode material on the substrate. When the first electrode 110 is an anode, the first electrode material may be selected from materials with a high work function so as to facilitate hole injection. The first electrode 110 may be a reflective electrode, a semi-transmissive electrode, or a transmissive electrode. The first electrode material may be a transparent and highly conductive material, and non-limiting examples of such material may include indium tin oxide (ITO), indium zinc oxide (IZO), tin oxide (SnO₂), and zinc oxide (ZnO). When the first electrode 110 is a semi-transmissive electrode or a reflective electrode, the first electrode material may be at least one selected from magnesium (Mg), aluminum (Al), aluminum-lithium (Al-Li), calcium (Ca), magnesium-indium (Mg-In), and magnesium-silver (Mg-Ag).

The first electrode 110 may have a single-layer structure or a multi-layer structure including two or more layers. For example, the first electrode 110 may have a triple-layer structure of ITO/Ag/ITO, but embodiments of the present invention are not limited thereto. An organic layer 150 is positioned on the first electrode 110. The organic layer 150 includes an emission layer 160.

The organic layer 150 includes a hole transport region 130 between the first electrode 110 and the emission layer 160. The organic layer 150 may further include an electron transport region 180 between the emission layer 160 and the second electrode 190.

The hole transport region 130 includes a hole injection layer, a hole transport layer and an auxiliary layer 140, wherein the layers are sequentially stacked from the first electrode 110 in this stated order and wherein the emission layer 160 and the auxiliary layer 140 are adjacent to each other. The hole transport region 130 may further include an electron blocking layer (EBL). The electron transport region 180 may include at least one selected from a hole blocking layer (HBL), an electron transport layer (ETL), and an electron injection layer (EIL), but embodiments of the present invention are not limited thereto. The hole injection layer is formed on the first electrode 110 by using (utilizing) one or more suitable methods, such as vacuum deposition, spin coating, casting, a Langmuir-Blodgett (LB) method, ink-jet printing, laser-printing, and/or laser-induced thermal imaging (LITI).

When the hole injection layer is formed by vacuum deposition, the vacuum deposition may be performed at a deposition temperature in a range of about 100°C to about 500°C, at a vacuum degree in a range of about 10⁻⁸ torr (1 torr=133.3 Pa) to about 10⁻³ torr, and at a deposition rate in a range of about 0.01 Å/sec (1Å=10⁻¹⁰m) to about 100 Å/sec, depending on the compound for forming the hole injection layer and the structure of the desired hole injection layer.

When a hole injection layer is formed by spin coating, the spin coating may be performed at a coating rate in a range of about 2000 rpm to about 5000 rpm and at a temperature in a range of about 80°C to about 200°C, depending on the compound for forming the hole injection layer and the structure of the desired hole injection layer.

The hole transport layer is formed on the hole injection layer by using one or more suitable methods, such as vacuum deposition, spin coating, casting, a LB method, ink-jet printing, laser-printing, and/or LITI. When the hole transport layer is formed by vacuum deposition and/or by spin coating, the deposition conditions and the coating conditions may be similar to the deposition conditions and the coating conditions for forming the hole injection layer. In an embodiment not being part of the present invention, the hole transport region 130 may include, for example, at least one selected from m-MTDATA, TDATA, 2-TNATA, NPB, β-NPB, TPD, Spiro-TPD, Spiro-NPB, a methylated NPB, TAPC, HMTPD, 4,4',4"-tris(N-carbazolyl)triphenylamine (TCTA), polyaniline/dodecyla benzenesulfonic acid (Pani/DBSA), poly(3,4-ethylenedioxythiophene)/poly(4-styrenesulfonate) (PEDOT/PSS), polyaniline/camphor sulfonic acid (Pani/CSA), (polyaniline)/poly(4-styrenesulfonate) (PANI/PSS), and a first hole transporting compound represented by any one of Formulae 201 and 202:

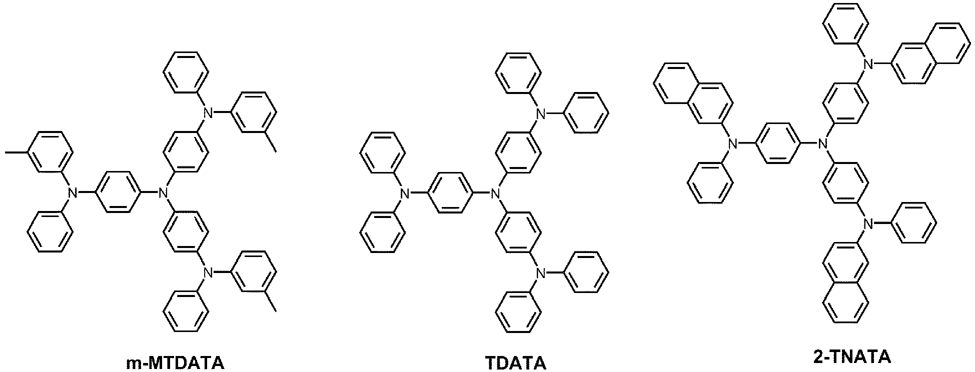

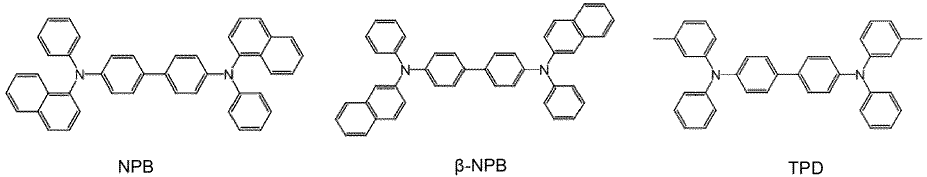

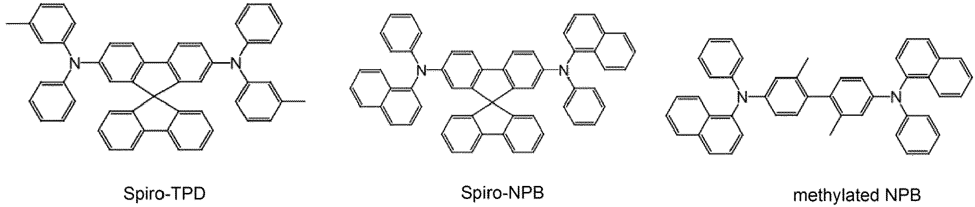

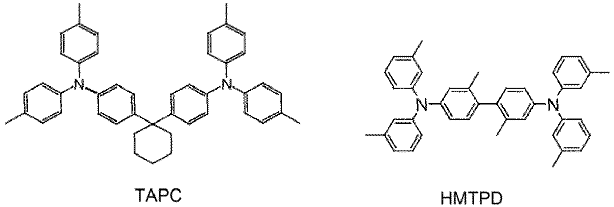

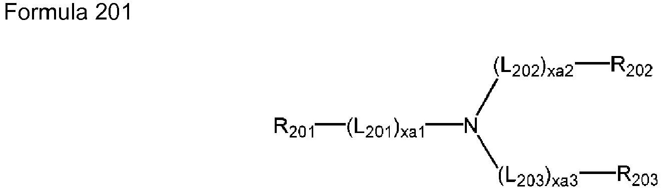

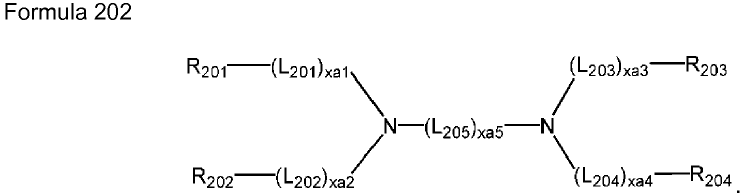

In Formulae 201 and 202,
L₂₀₁ to L₂₀₅ may be each independently the same as defined in connection with L₁₁ in the present specification;
xa1 to xa4 may each independently be selected from 0, 1, 2, and 3;
xa5 may be selected from 1, 2, 3, 4, and 5; and
R₂₀₁ to R₂₀₄ may be each independently defined the same as the definition provided in connection with R₁₁ in the present specification.

For example, in Formulae 201 and 202,

L₂₀₁ to L₂₀₅ may be each independently selected from:
a phenylene group, a naphthylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group,
a quinolinylene group, an isoquinolinylene group, a quinoxalinylene group, a quinazolinylene group, a carbazolylene group, and a triazinylene group; and
a phenylene group, a naphthalenylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, an isoindolyl group, a quinolinyl group, an isoquinolinyl group, a quinoxalinyl group, a quinazolinyl group, a carbazolyl group, and a triazinyl group;
xa1 to xa4 may be each independently 0, 1, or 2;
xa5 may be 1, 2, or 3;
R₂₀₁ to R₂₀₄ may be each independently selected from:
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, an azulenyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, but embodiments of the present invention are not limited thereto. In the present invention the hole transport layer consists of a first hole transporting compound represented by Formula 201A:

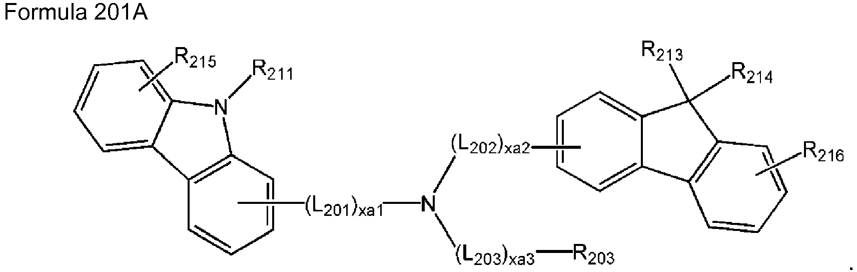

For example, the first hole transporting compound may be represented by Formula 201A-1:

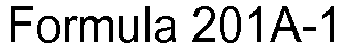

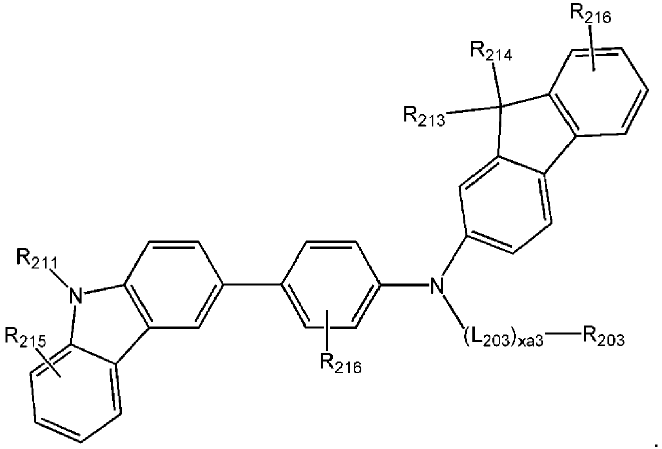

The hole transport layer may further consist of first hole transporting compound represented by Formula 202A:

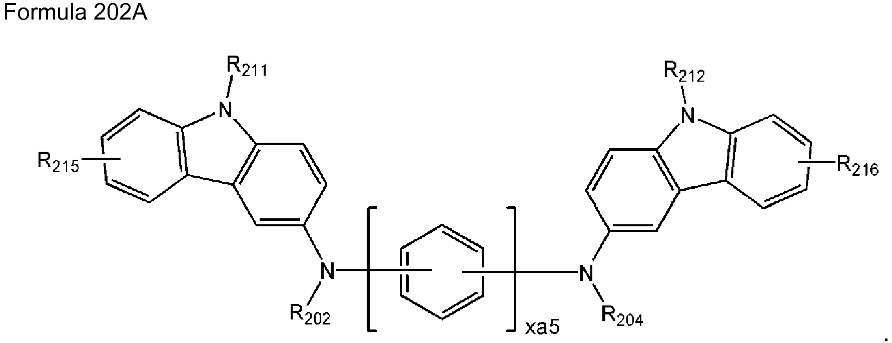

In Formulae 201A, 201A-1, and 202A, L₂₀₁ to L₂₀₃ are the same as defined in connection with L₁₁, xa1 to xa3 are independtly selected from 0, 1, 2, and 3, xa5 is slected from 1, 2, 3, 4, and 5, and R₂₀₂ to R₂₀₄ are the same as the definition provided in connection with R₁₁, R₂₁₁ and R₂₁₂ are each independently defined the same as the definition provided in connection with R₂₀₃, and
R₂₁₃ to R₂₁₆ are each independently selected from hydrogen, deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

For example, in Formulae 201A, 201A-1, and 202A,
L₂₀₁ to L₂₀₃ may be each independently selected from:
a phenylene group, a naphthylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group; and
a phenylene group, a naphthylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
xa1 to xa3 may be each independently 0 or 1;
R₂₀₃, R₂₁₁, and R₂₁₂ may be each independently selected from a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group and a triazinyl group; and
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
R₂₁₃ and R₂₁₄ may be each independently selected from:
   a C₁-C₂₀ alkyl group and a C₁-C₂₀ alkoxy group;
   a C₁-C₂₀ alkyl group and a C₁-C₂₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
R₂₁₅ and R₂₁₆ may be each independently selected from:
   hydrogen, deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, and a phosphoric acid or a salt thereof;
   a C₁-C₂₀ alkyl group and a C₁-C₂₀ alkoxy group;
   a C₁-C₂₀ alkyl group and a C₁-C₂₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, and a triazinyl group; and
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, and a triazinyl group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
   xa5 may be 1 or 2.

In Formulae 201A and 201A-1, R₂₁₃ and R₂₁₄ may be fused to each other (e.g., R₂₁₃ and R₂₁₄ may be combined) to form a saturated or unsaturated ring.

The first hole transporting compound may be one selected from Compounds HT1 to HT20 below:

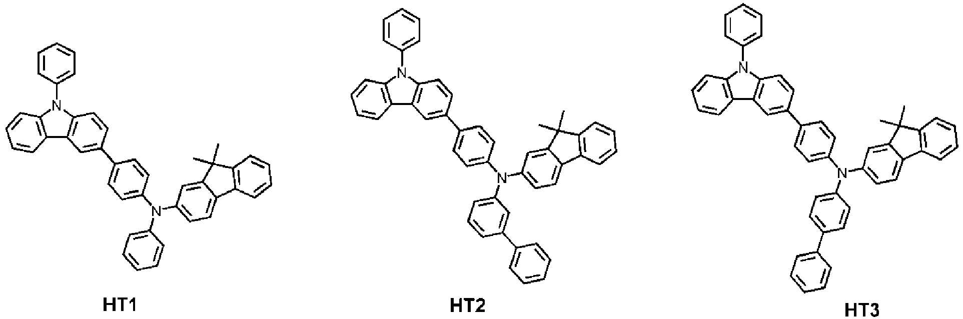

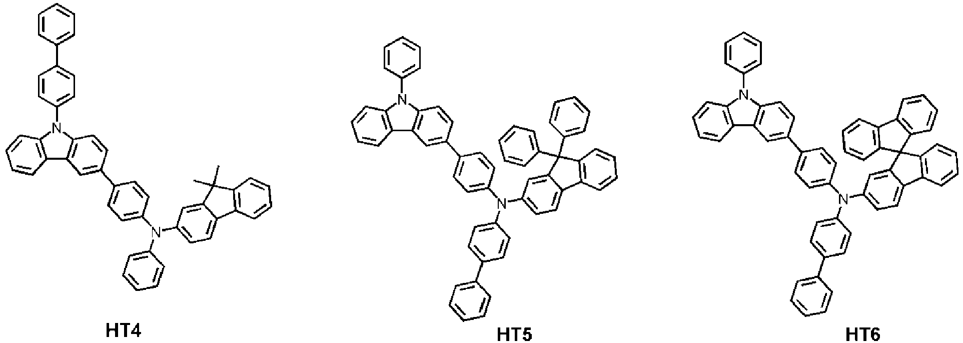

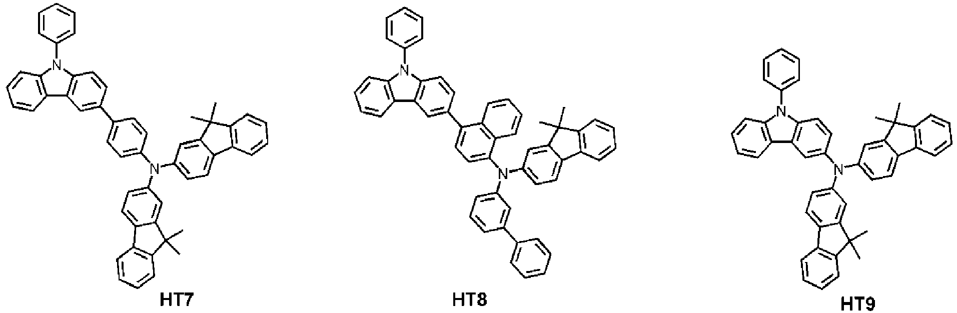

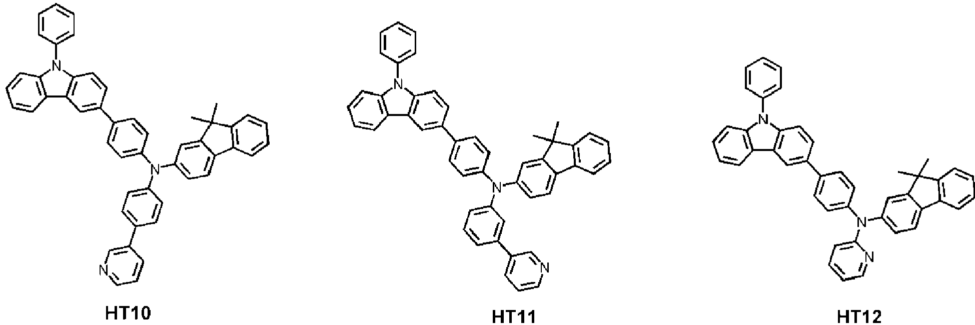

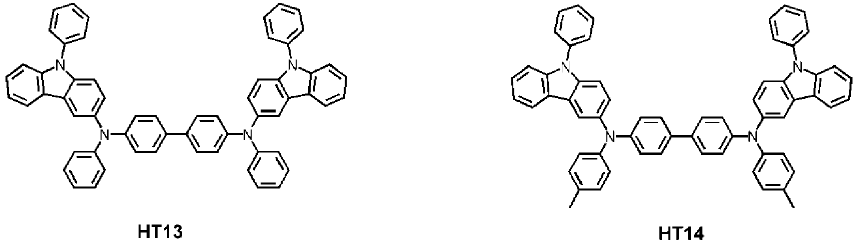

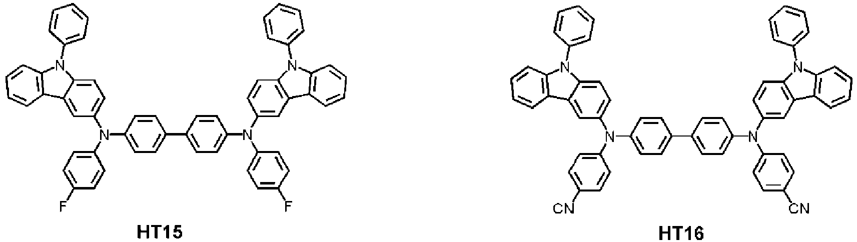

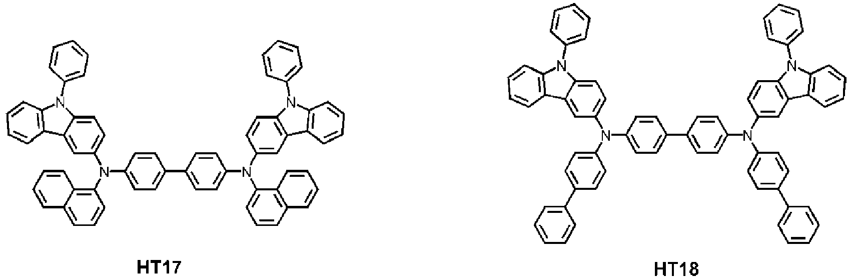

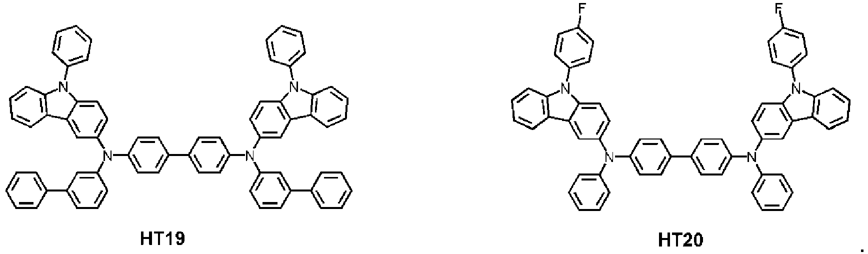

A thickness of the hole transport region 130 may be in a range of about 100 Å to about 10,000 Å, for example, about 100 Å to about 1,000 Å. In an embodiment not forming part of the present invention, when the hole transport region 130 includes a hole injection layer and a hole transport layer, a thickness of the hole injection layer may be in a range of about 100 Å to about 10,000 Å, for example, about 100 Å to about 1,000 Å, and a thickness of the hole transport layer may be in a range of about 50 Å to about 2000 Å, for example, about 100 Å to about 1500 Å. When thicknesses of the hole transport region 130, the hole injection layer, and the hole transport layer are within any of these ranges, satisfactory hole transporting properties may be obtained without a substantial increase in driving voltage.

The hole transport region 130 may further include a charge-generating material to improve conductive properties, in addition to the materials mentioned above. The charge-generating material may be homogeneously or non-homogeneously dispersed throughout the hole transport region 130.

The charge-generating material may be, for example, a p-dopant. The p-dopant may be one selected from a quinone derivative, a metal oxide, and a cyano group-containing compound, but embodiments of the present invention are not limited thereto. Non-limiting examples of the p-dopant include quinone derivatives such as tetracyanoquinonedimethane (TCNQ) and/or 2,3,5,6-tetrafluoro-tetracyano-1,4-benzoquinonedimethane (F4-TCNQ); metal oxides such as a tungsten oxide and/or a molybdenum oxide; and Compound HT-D1 illustrated below, but embodiments of the present invention are not limited thereto:

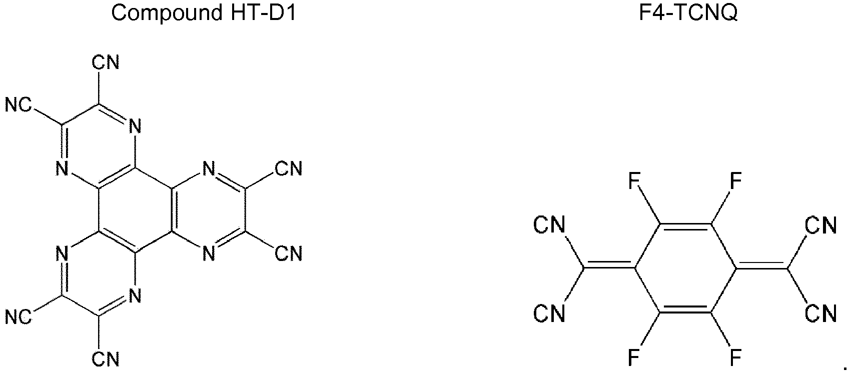

The auxiliary layer 140 includes an amine-based compound represented by Formula 1.

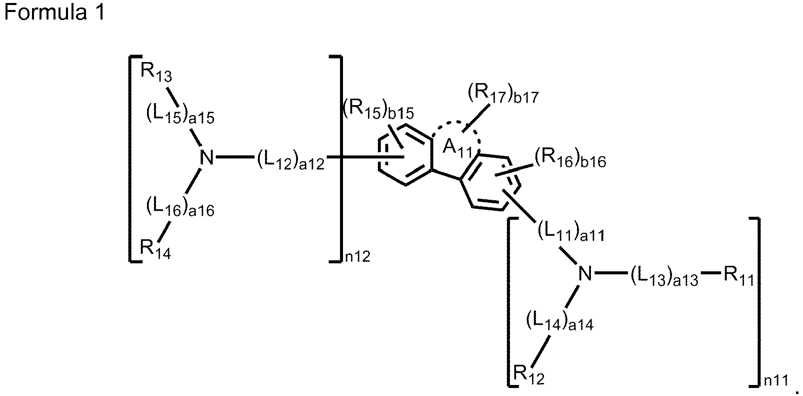

In Formula 1, A₁₁ is selected from a C₃-C₁₀ cycloalkane, a C₁-C₁₀ heterocycloalkane, a C₃-C₁₀ cycloalkene, a C₁-C₁₀ heterocycloalkene, a C₆-C₆₀ arene, a C₁-C₆₀ heteroarene, a non-aromatic condensed polycyclic hydrocarbon group, and a non-aromatic condensed heteropolycyclic hydrocarbon group.

For example, in Formula 1, A₁₁ may be represented by any one of Formulae 9-1 to 9-5:

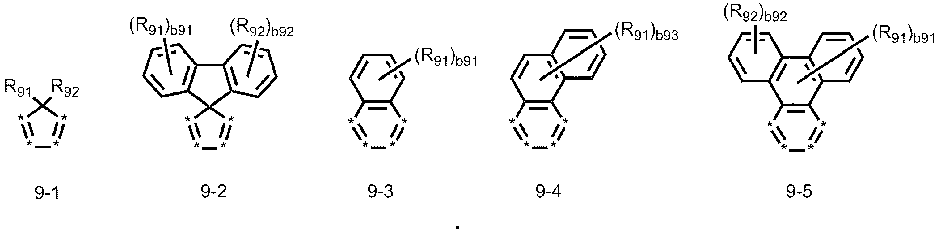

In Formulae 9-1 to 9-5,
* indicates a carbon atom (-C-) in Formula 1;
R₉₁ and R₉₂ are each independently selected from hydrogen, deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, and -N(Q₁)(Q₂);
where Q₁ and Q₂ are each independently selected from:
a C₆-C₆₀ aryl group and a monovalent non-aromatic condensed polycyclic group; and
a C₆-C₆₀ aryl group and a monovalent non-aromatic condensed polycyclic group, each substituted with a C₆-C₆₀ aryl group; and
b91 and b92 are each independently selected from 1, 2, 3, and 4;
b93 is selected from 1, 2, 3, 4, 5, and 6.
In some embodiments, in Formulae 9-1 to 9-5,
R₉₁ and R₉₂ are each independently selected from:
hydrogen, a methyl group, an ethyl group, an n-propyl group, a phenyl group, a naphthyl group, a fluorenyl group, a carbazolyl group, a dibenzofuranyl group, a dibenzothiophenyl group, and -N(Q₁)(Q₂); and
a phenyl group, a naphthyl group, a fluorenyl group, a carbazolyl group, a dibenzofuranyl group, and a dibenzothiophenyl group, each substituted with -N(Q₃₁)(Q₃₂);
where, here, Q₁, Q₂, Q₃₁, and Q₃₂ are each independently selected from:
   a phenyl group, a naphthyl group, a fluorenyl group, a carbazolyl group, a dibenzofuranyl group, and a dibenzothiophenyl group; and
   a phenyl group, a naphthyl group, a fluorenyl group, a carbazolyl group, a dibenzofuranyl group, and a dibenzothiophenyl group, each substituted with at least one selected from a phenyl group and a naphthyl group;
   b91 and b92 are each independently selected from 1, 2, 3, and 4; and
   b93 is selected from 1, 2, 3, 4, 5, and 6.

In Formula 1, L₁₁ to L₁₆ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group.

For example, in Formula 1, L₁₁ to L₁₆ may be each independently selected from:
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl group.

For example, in Formula 1, L₁₁ to L₁₆ may be each independently selected from:
a naphthylene group, a fluorenylene group, a spiro-fluorenylene group, a benzofluorenylene group, a dibenzofluorenylene group, a phenanthrenylene group, an anthracenylene group, a triphenylenylene group, a pyrenylene group, a chrysenylene group, a furanylene group, a thiophenylene group, a carbazolylene group, a dibenzofuranylene group, and a dibenzothiophenylene group; and
a phenylene group, a naphthylene group, a fluorenylene group, a spiro-fluorenylene group, a benzofluorenylene group, a dibenzofluorenylene group, a phenanthrenylene group, an anthracenylene group, a triphenylenylene group, a pyrenylene group, a chrysenylene group, a furanylene group, a thiophenylene group, a carbazolylene group, a dibenzofuranylene group, and a dibenzothiophenylene group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, a dibenzothiophenyl group, a benzocarbazolyl group, and a dibenzocarbazolyl group.

For example, in Formula 1, L₁₁ to L₁₆ may be each independently represented by one of Formulae 3-1 to 3-18:

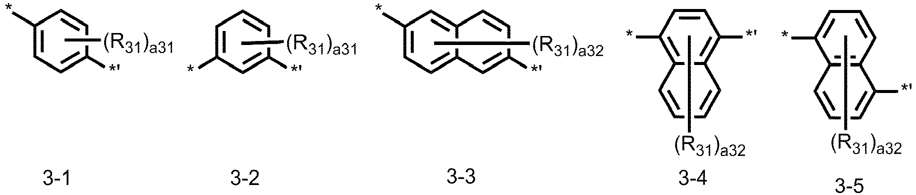

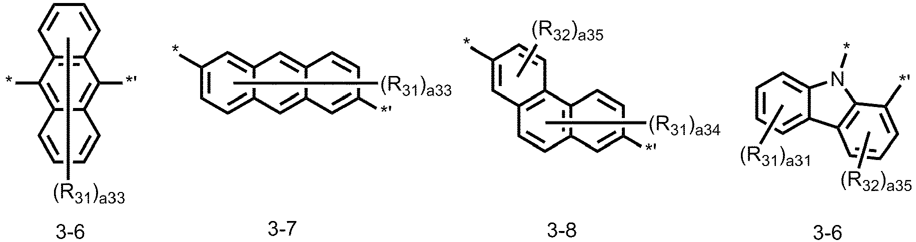

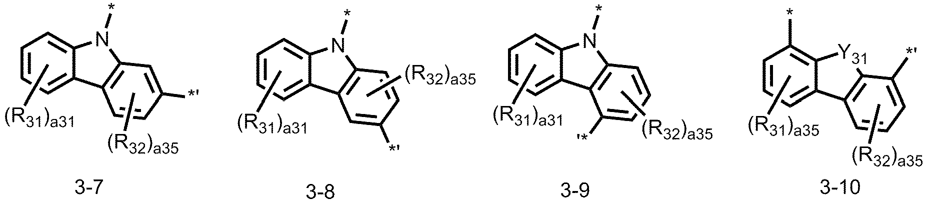

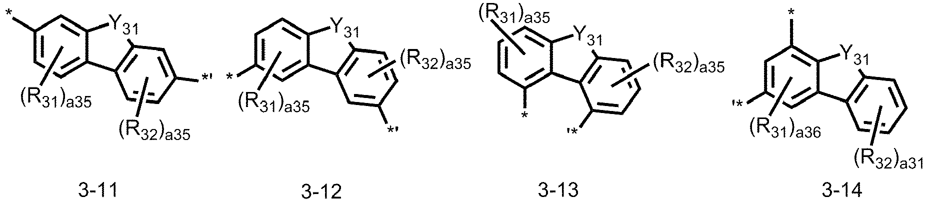

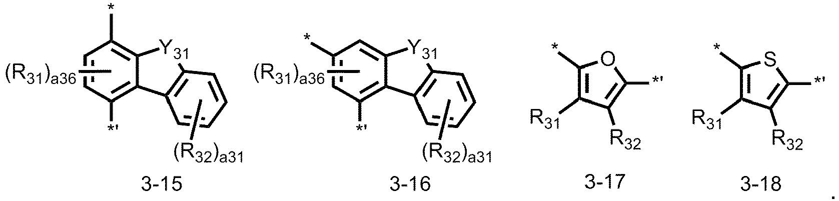

In Formulae 3-1 to 3-18,
Y₃₁ is selected from C(R₃₃)(R₃₄), N(R₃₃), O, and S;
R₃₁ to R₃₄ are each independently selected from hydrogen, deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; a31 is selected from 1, 2, 3, and 4;
a32 is selected from 1, 2, 3, 4, 5, and 6;
a33 is selected from 1, 2, 3, 4, 5, 6, 7, and 8;
a34 is selected from 1, 2, 3, 4, and 5;
a35 is selected from 1, 2, and 3;
a36 is selected from 1 and 2; and
* and *' are each independently a binding site to a neighboring atom.

For example, in Formula 1, L₁₁ to L₁₆ may be each independently represented by one of Formulae 4-1 to 4-42:

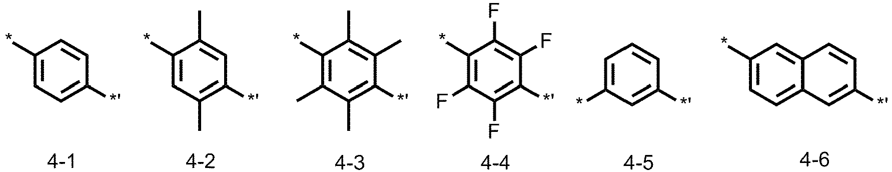

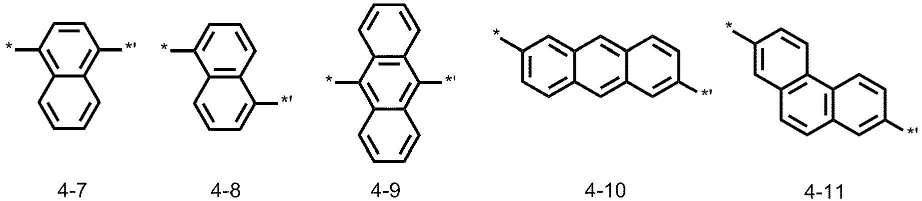

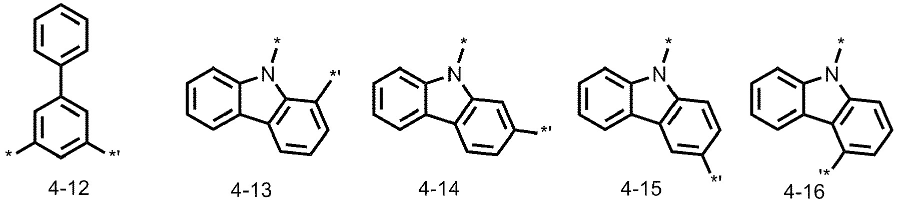

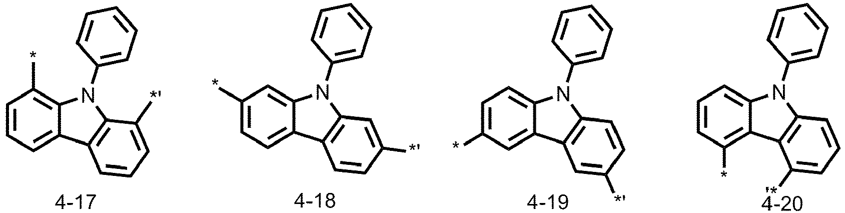

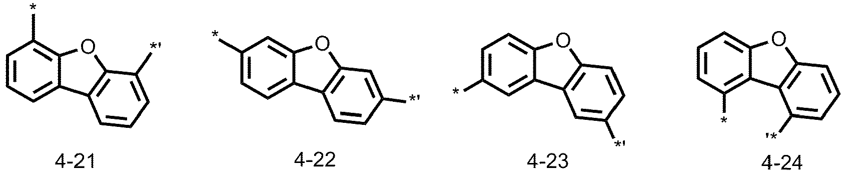

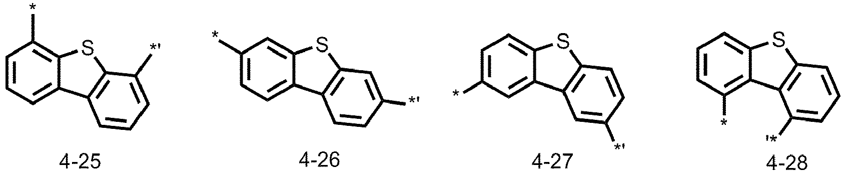

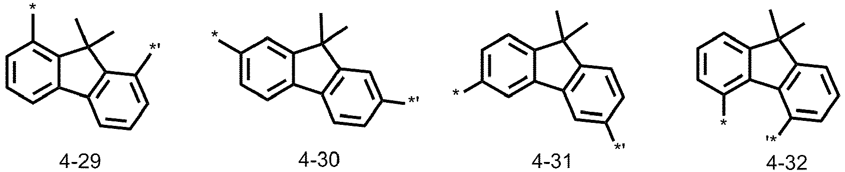

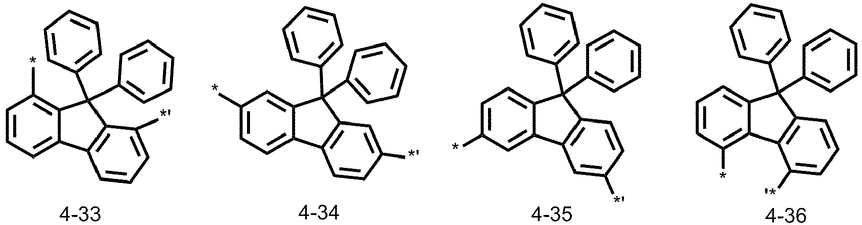

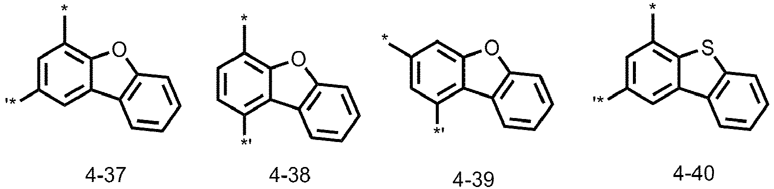

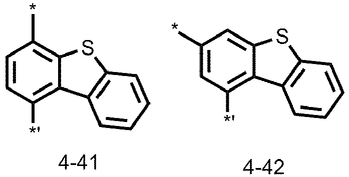

In Formulae 4-1 to 4-42,
* and *' are each independently a binding site to a neighboring atom.

In Formula 1, a11 denotes the number of L₁₁, and a11 may be selected from 0, 1, 2, and 3. When a11 is 0, (L₁₁)ₐ₁₁ is a single bond, when a11 is 2 or greater, a plurality of L₁₁ may be identical to or different from each other. For example, in Formula 1, a11 may be selected from 0 and 1.

In Formula 1, a12 denotes the number of L₁₂, and a12 may be selected from 0, 1, 2, and 3. When a12 is 0, (L₁₂)ₐ₁₂ is a single bond, when a12 is 2 or greater, a plurality of L₁₂ may be identical to or different from each other. For example, in Formula 1, a12 may be selected from 0 and 1.

In Formula 1, a13 denotes the number of L₁₃, and a13 may be selected from 0, 1, 2, and 3. When a13 is 0, (L₁₃)ₐ₁₃ is a single bond, when a13 is 2 or greater, a plurality of L₁₃ may be identical to or different from each other. For example, in Formula 1, a13 may be selected from 0 and 1.

In Formula 1, a14 denotes the number of L₁₄, and a14 may be selected from 0, 1, 2, and 3. When a14 is 0, (L₁₄)ₐ₁₄ is a single bond, when a14 is 2 or greater, a plurality of L₁₄ may be identical to or different from each other. For example, in Formula 1, a14 may be selected from 0 and 1.

In Formula 1, a15 denotes the number of L₁₅, and a15 may be selected from 0, 1, 2, and 3. When a15 is 0, (L₁₅)ₐ₁₅ is a single bond, when a15 is 2 or greater, a plurality of L₁₅ may be identical to or different from each other. For example, in Formula 1, a15 may be selected from 0 and 1.

In Formula 1, a16 denotes the number of L₁₆, and a16 may be selected from 0, 1, 2, and 3. When a16 is 0, (L₁₆)ₐ₁₆ is a single bond, when a16 is 2 or greater, a plurality of L₁₆ may be identical to or different from each other. For example, in Formula 1, a16 may be selected from 0 and 1.

In Formula 1, R₁₁ to R₁₄ are each independently selected from a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group.

For example, in Formula 1, R₁₁ to R₁₄ may be each independently selected from:
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, an imidazopyridinyl group, and an imidazopyrimidinyl group; and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, an imidazopyridinyl group, and an imidazopyrimidinyl group, each substituted with at least one selected from deuterium, -F, - CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a biphenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a benzoquinoxalinyl group, a quinazolinyl group, a benzoquinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, an imidazopyridinyl group, and an imidazopyrimidinyl group.

For example, in Formula 1, R₁₁ to R₁₄ may be each independently selected from:
a phenyl group, a naphthyl group, a fluorenyl group, a spiro-fluorenyl group, a phenalenyl group, a phenanthrenyl group, a pyrenyl group, a pyridinyl group, a pyrazinyl group, a pyrimidinyl group, a quinolinyl group, an isoquinolinyl group, a naphthyridinyl group, a quinoxalinyl group, a quinazolinyl group, a cinnolinyl group, a carbazolyl group, a triazinyl group, a dibenzofuranyl group, and a dibenzothiophenyl group; and
a phenyl group, a naphthyl group, a fluorenyl group, a spiro-fluorenyl group, a phenalenyl group, a phenanthrenyl group, a pyrenyl group, a pyridinyl group, a pyrazinyl group, a pyrimidinyl group, a quinolinyl group, an isoquinolinyl group, a naphthyridinyl group, a quinoxalinyl group, a quinazolinyl group, a cinnolinyl group, carbazolyl group, a triazinyl group, a dibenzofuranyl group, and a dibenzothiophenyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group.

For example, in Formula 1, R₁₁ to R₁₄ may be each independently selected from Formulae 5-1 to 5-18:

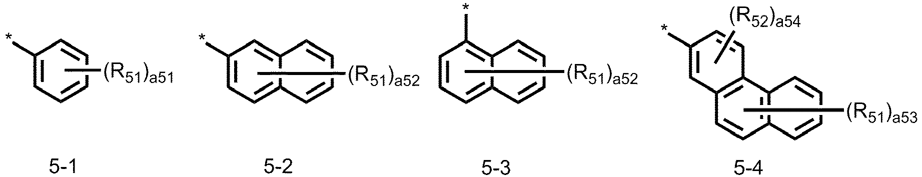

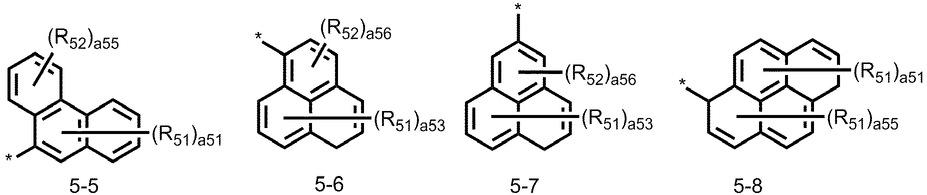

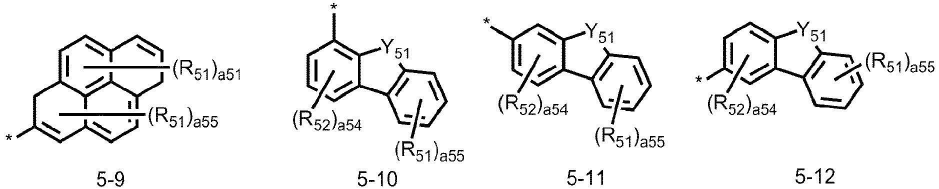

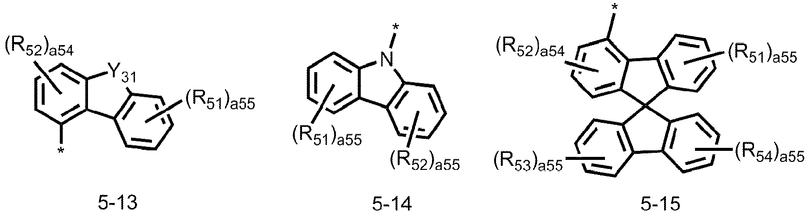

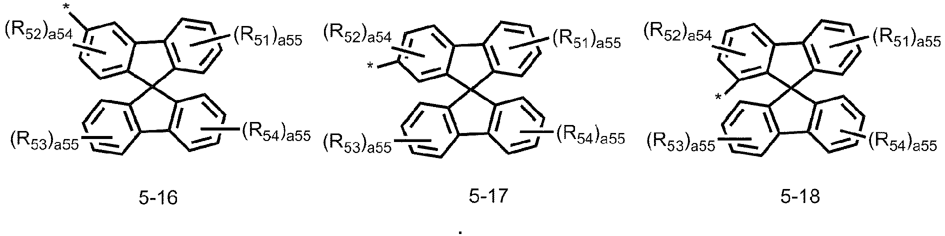

In Formulae 5-1 to 5-18,
Y₅₁ is selected from C(R₅₅)(R₅₆), N(R₅₅), O, and S;
R₅₁ to R₅₆ are each independently selected from hydrogen, deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
a51 is selected from 1, 2, 3, 4, and 5;
a52 is selected from 1, 2, 3, 4, 5, 6, and 7;
a53 is selected from 1, 2, 3, 4, 5, and 6;
a54 is selected from 1, 2, and 3;
a55 is selected from 1, 2, 3, and 4;
a56 is selected from 1 and 2; and
* is a binding site to a neighboring atom.

For example, in Formula 1, R₁₁ to R₁₄ may be each independently selected from Formulae 6-1 to 6-59:

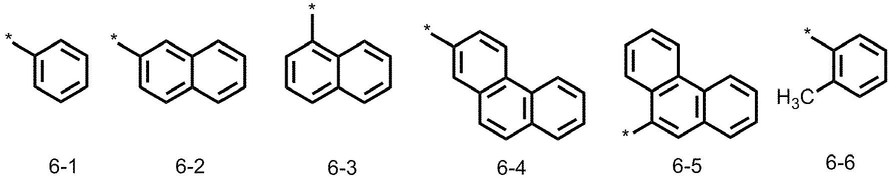

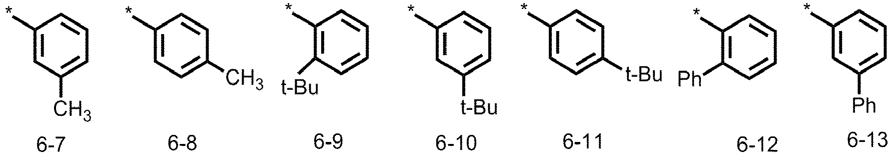

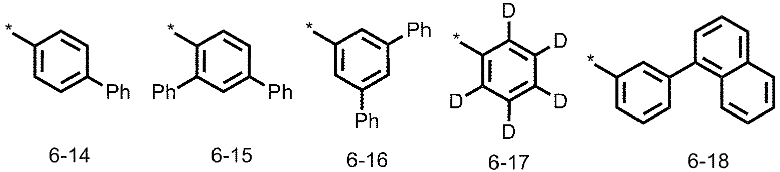

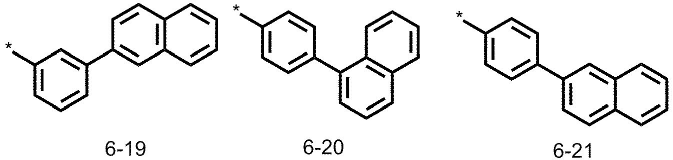

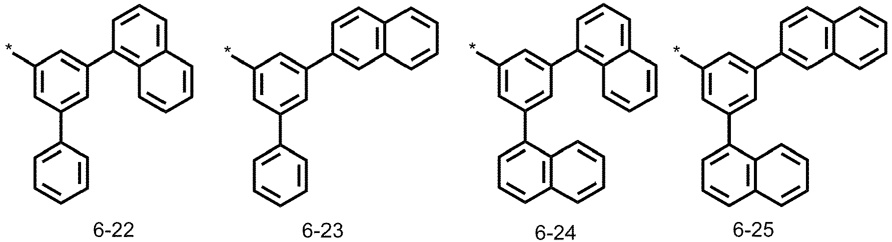

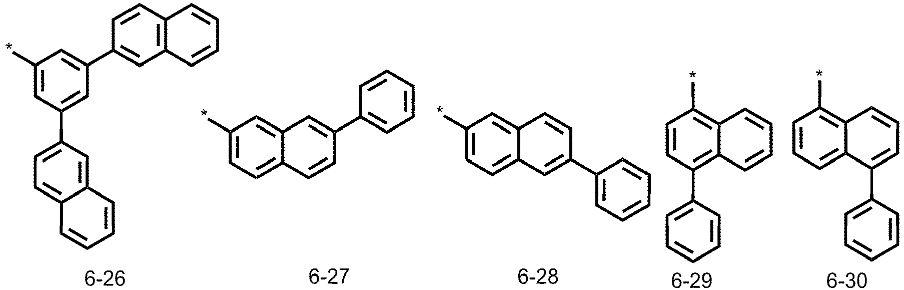

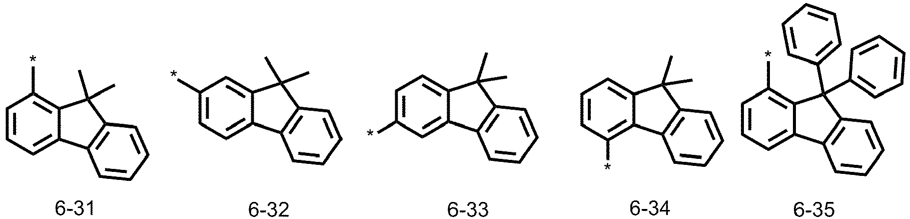

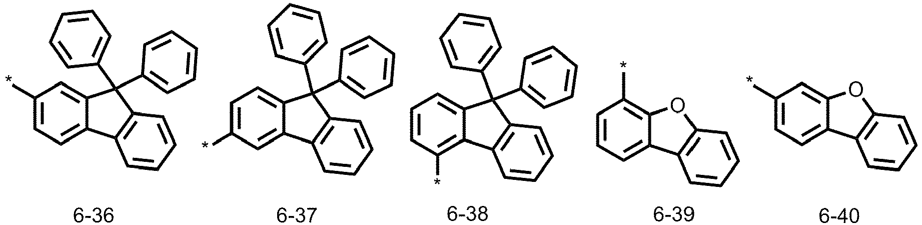

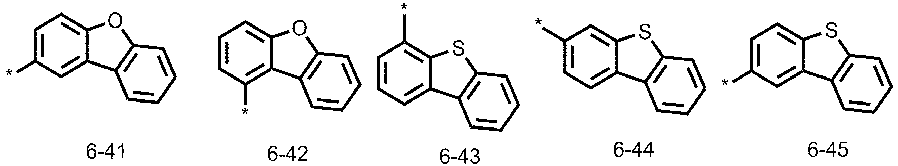

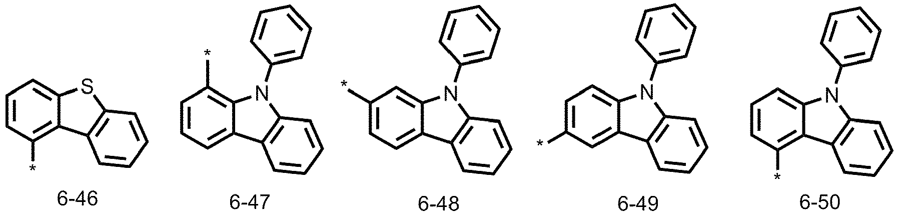

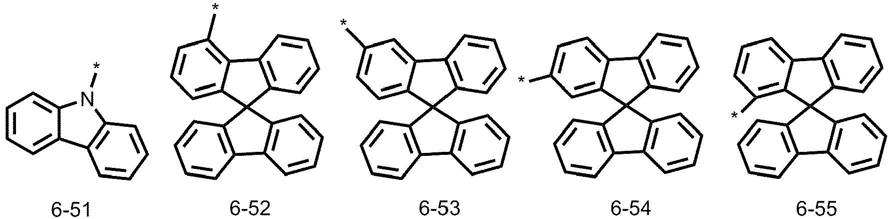

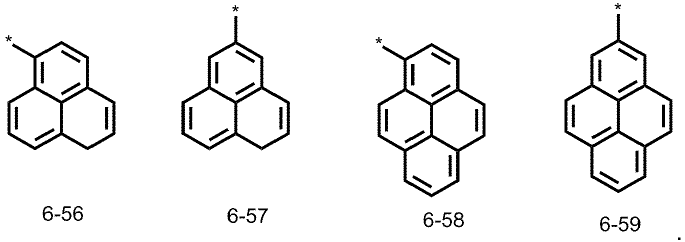

In Formulae 6-1 to 6-59,
t-Bu is a tert-butyl group;
Ph is a phenyl group; and
* is a binding site to a neighboring atom.

In Formula 1, n11 denotes the number of moieties represented by 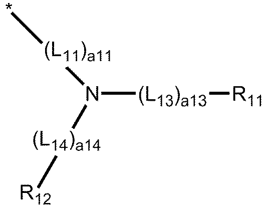 (where, * is a binding site to a neighboring atom), and n11 is selected from 0, 1, and 2. When n11 is 2, a plurality of moieties represented by 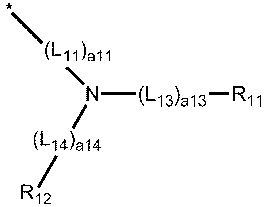 may be identical to or different from each other. For example, in Formula 1, n11 may be selected from 0 and 1.

In Formula 1, n12 denotes the number of moieties represented by 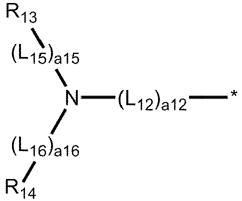 (where, * is a binding site to a neighboring atom), and n12 is selected from 0, 1, and 2.

When n12 is 2, a plurality of moieties represented by 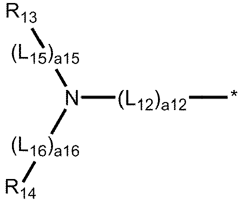 may be identical to or different from each other. For example, in Formula 1, n12 may be selected from 0 and 1.

In Formula 1, the sum of n11 and n12 is selected from 1, 2, 3, and 4. For example, in Formula 1, the sum of n11 and n12 may be selected from 1 and 2.

In Formula 1, R₁₅ to R₁₇ are each independently selected from hydrogen, deuterium, -F, - CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), and -Si(Q₃)(Q₄)(Q₅);
where Q₁ to Q₅ may be each independently selected from a C₁-C₆₀ alkyl group, a C₆-C₆₀ aryl group, C₁-C₆₀ heteroaryl group, a monovalent non-aromatic condensed polycyclic group, and a monovalent non-aromatic condensed heteropolycyclic group; and
a C₆-C₆₀ aryl group, a C₁-C₆₀ heteroaryl group, a monovalent non-aromatic condensed polycyclic group, and a monovalent non-aromatic condensed heteropolycyclic group, each substituted with a C₆-C₆₀ aryl group.

For example, in Formula 1, R₁₅ to R₁₇ may be each independently selected from hydrogen, deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, and -N(Q₁)(Q₂);
where Q₁ and Q₂ may be each independently selected from:
a C₆-C₆₀ aryl group and a monovalent non-aromatic condensed polycyclic group; and
a C₆-C₆₀ aryl group and a monovalent non-aromatic condensed polycyclic group, each substituted with a C₆-C₆₀ aryl group.

For example, in Formula 1, R₁₅ to R₁₇ may be each independently selected from:
hydrogen, a methyl group, an ethyl group, an n-propyl group, a phenyl group, a naphthyl group, a fluorenyl group, a carbazolyl group, a dibenzofuranyl group, a dibenzothiophenyl group, and -N(Q₁)(Q₂); and
a phenyl group, a naphthyl group, a fluorenyl group, a carbazolyl group, a dibenzofuranyl group, and a dibenzothiophenyl group, each substituted with -N(Q₃₁)(Q₃₂);
where Q₁, Q₂, Q₃₁, and Q₃₂ are each independently selected from a phenyl group, a naphthyl group, a fluorenyl group, a carbazolyl group, a dibenzofuranyl group, and a dibenzothiophenyl group; and
a phenyl group, a naphthyl group, a fluorenyl group, a carbazolyl group, a dibenzofuranyl group, and a dibenzothiophenyl group, each substituted with at least one selected from a phenyl group and a naphthyl group.

In Formula 1, b15 denotes the number of R₁₅, and b15 is selected from 1, 2, 3, and 4. When b15 is 2 or greater, a plurality of R₁₅ may be identical to or different from each other.

In Formula 1, b16 denotes the number of R₁₆, and b16 is selected from 1, 2, 3, and 4. When b16 is 2 or greater, a plurality of R₁₆ may be identical to or different from each other.

In Formula 1, b17 denotes the number of R₁₇, and b17 is selected from 1, 2, 3, 4, 5, 6, 7, and 8. When b17 is 2 or greater, a plurality of R₁₇ may be identical to or different from each other.

For example, the amine-based compound represented by Formula 1 may be represented by any one of Formulae 1-1 to 1-5:

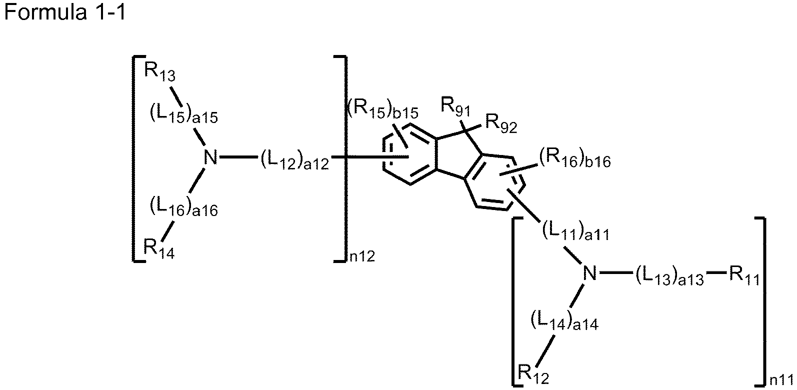

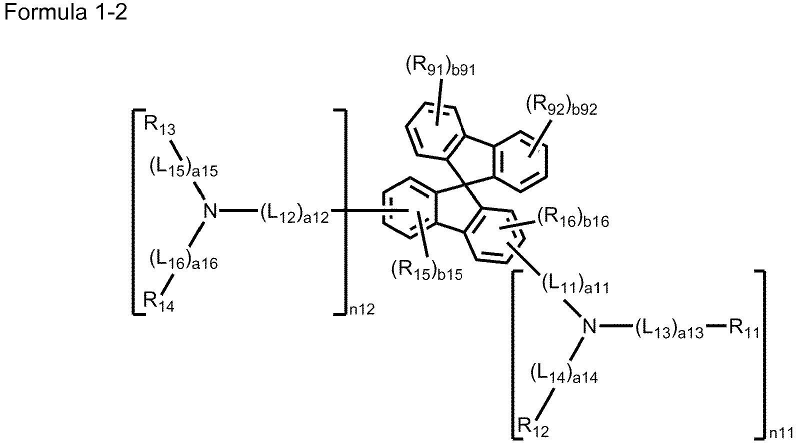

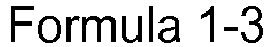

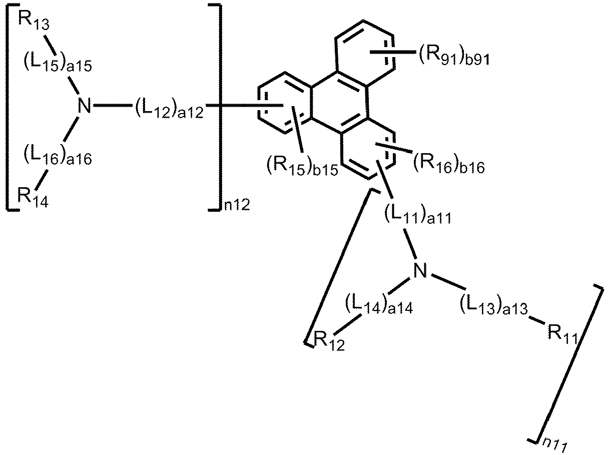

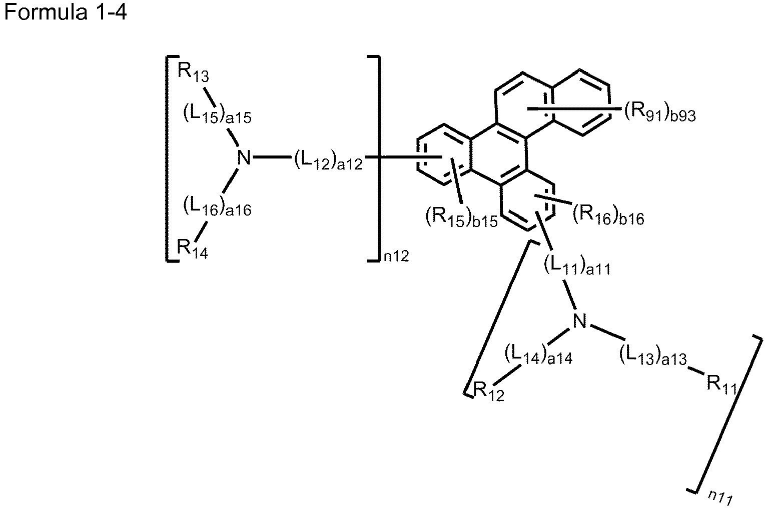

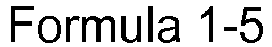

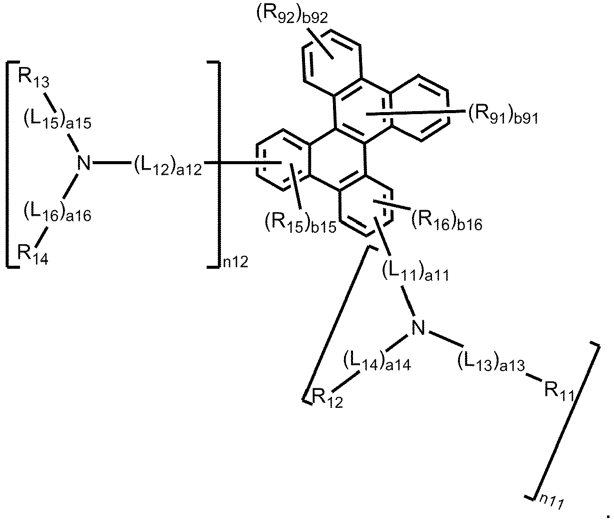

In Formulae 1-1 to 1-5,
L₁₁ to L₁₆ may be each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
a11 to a16 may be each independently selected from 0, 1, 2, and 3;
R₁₁ to R₁₄ may be each independently selected from a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group;
n11 and n12 may be each independently selected from 0, 1, and 2, and the sum of n11 and n12 may be selected from 1, 2, 3 and 4;
R₁₅, R₁₆, R₉₁ and R₉₂ may be each independently selected from hydrogen, deuterium, -F, - CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, and -N(Q₁)(Q₂);
where Q₁ and Q₂ may be each independently selected from:
a C₆-C₆₀ aryl group and a monovalent non-aromatic condensed polycyclic group; and
a C₆-C₆₀ aryl group and a monovalent non-aromatic condensed polycyclic group, each substituted with a C₆-C₆₀ aryl group;
b15 to b16 may be each independently selected from 1, 2, 3, and 4;
b91 and b92 may be each independently selected from 1, 2, 3, and 4;
b93 is selected from 1, 2, 3, 4, 5, and 6.

For example, the amine-based compound represented by Formula 1 may be one selected from Compounds 1 to 48:

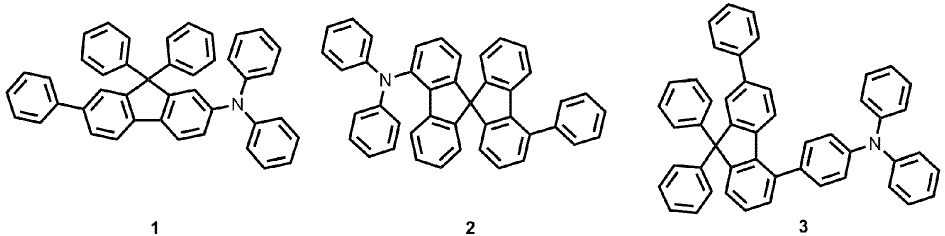

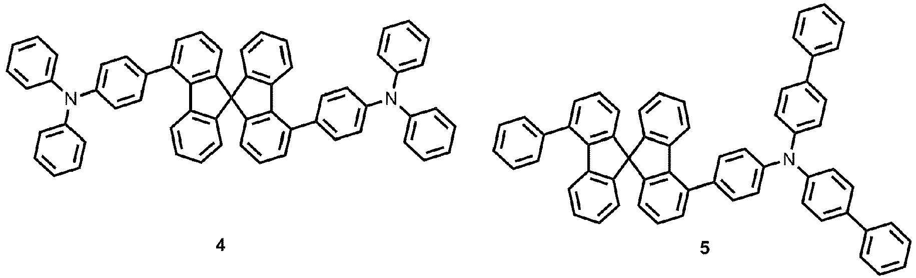

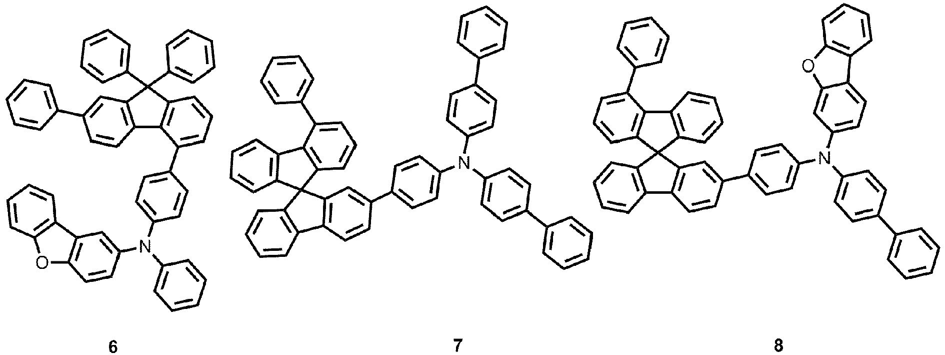

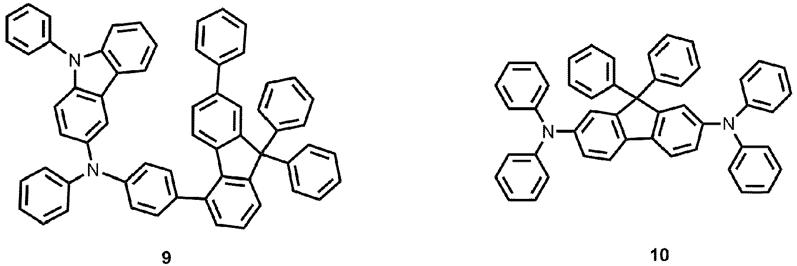

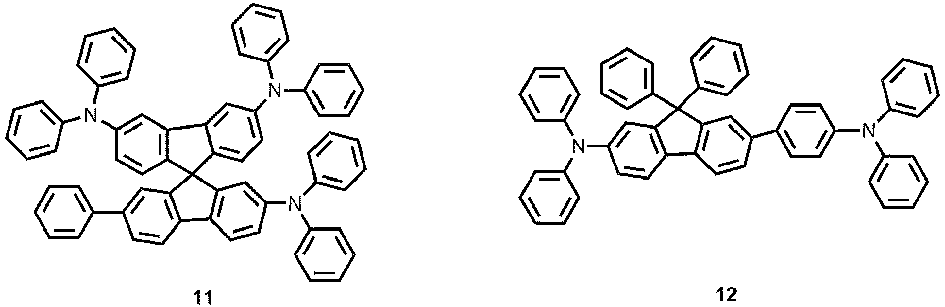

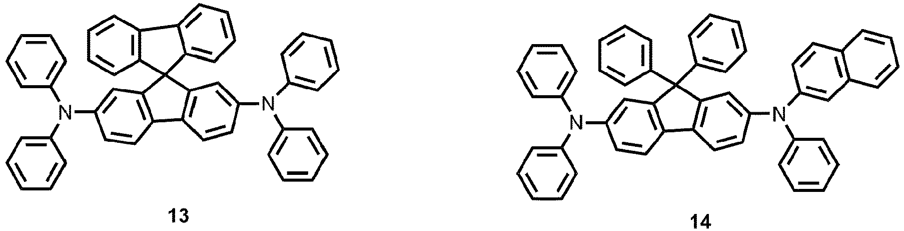

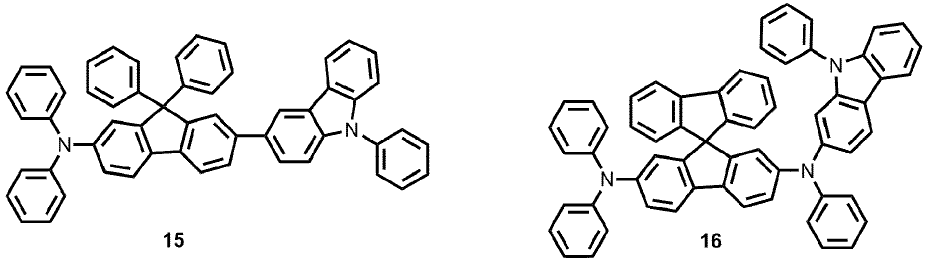

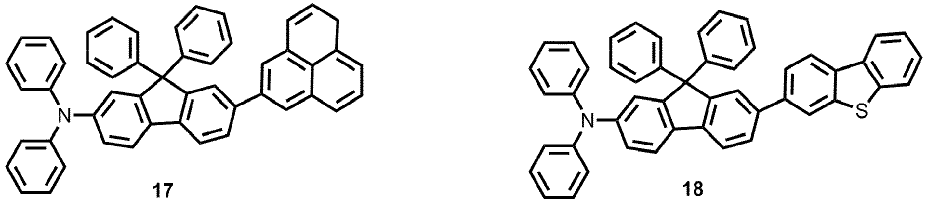

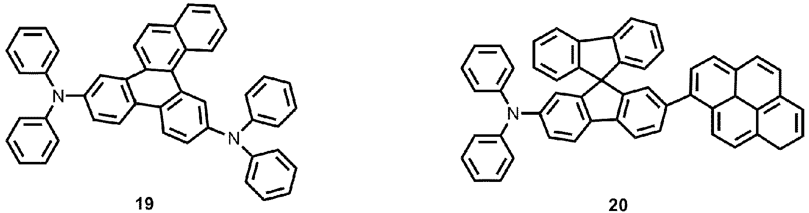

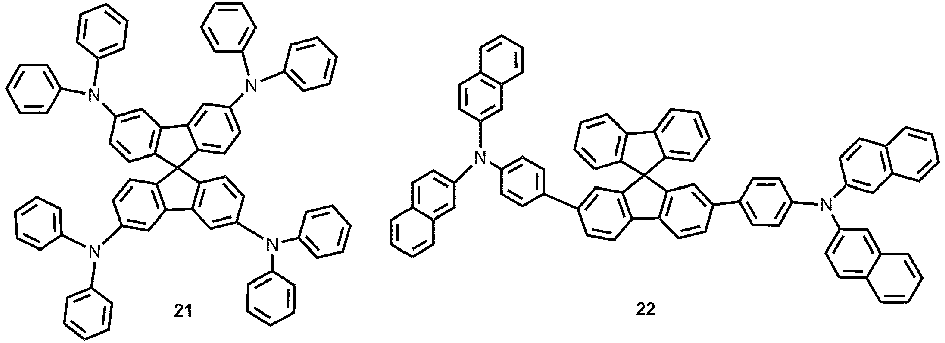

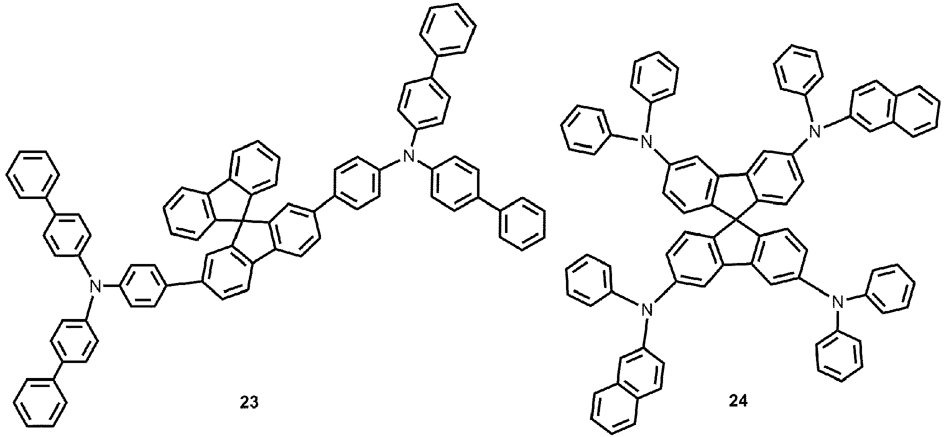

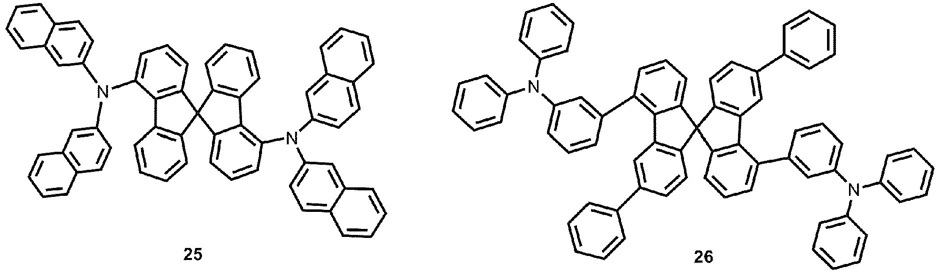

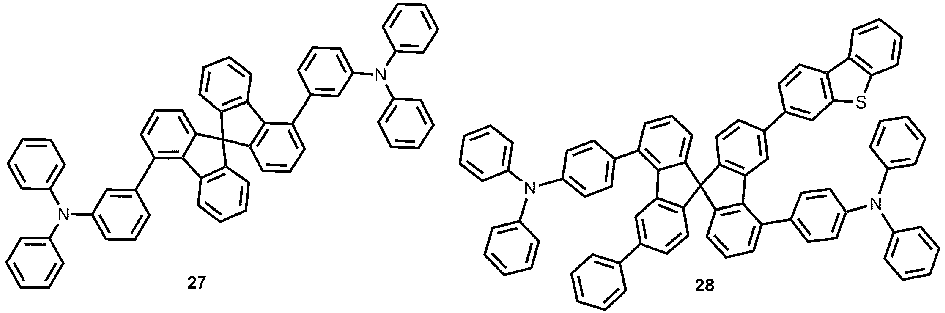

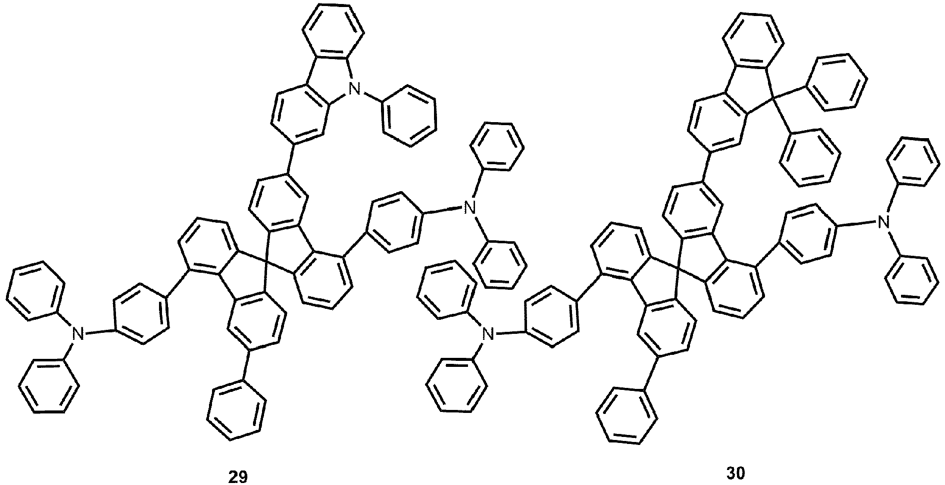

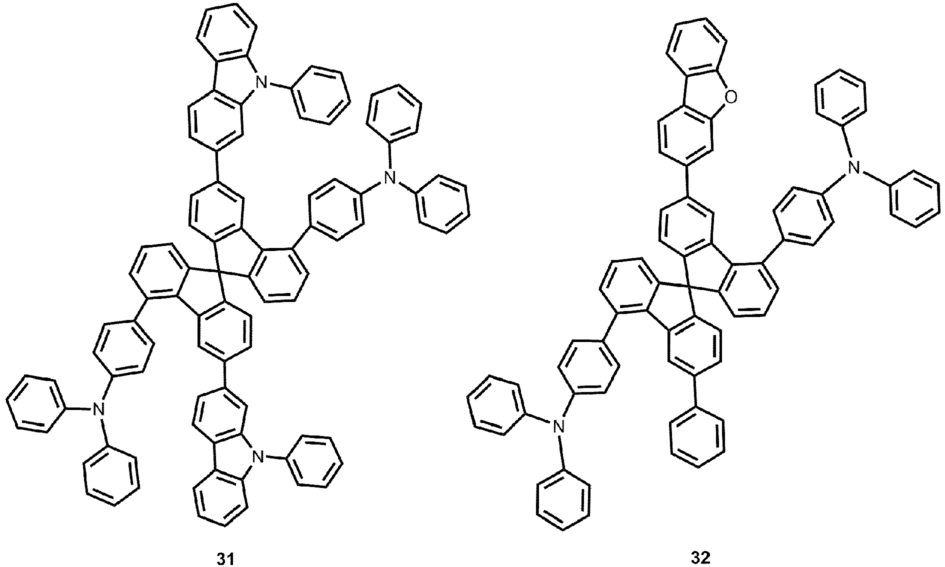

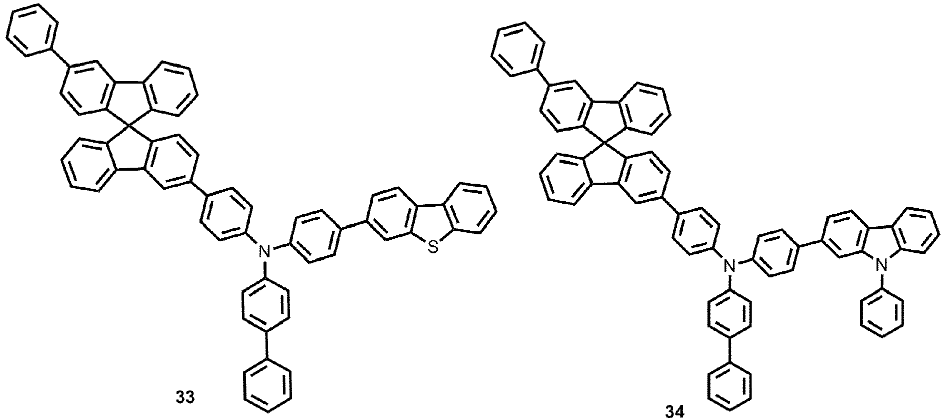

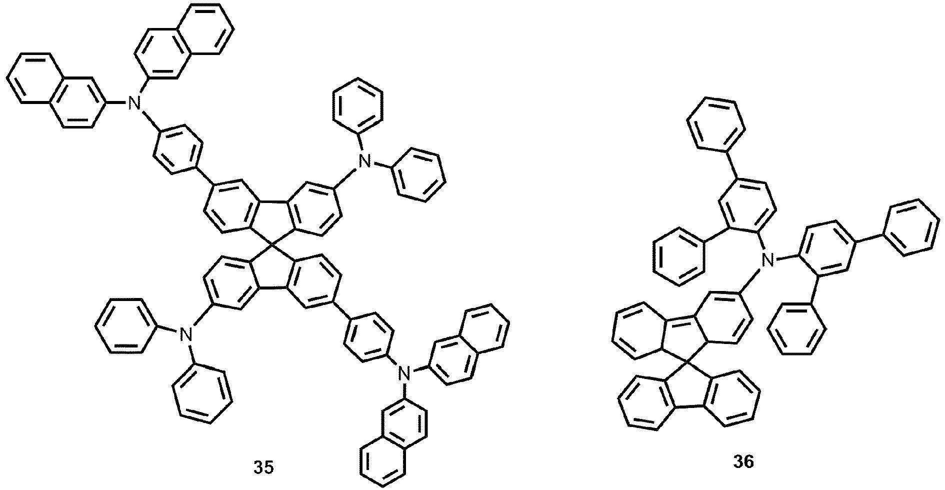

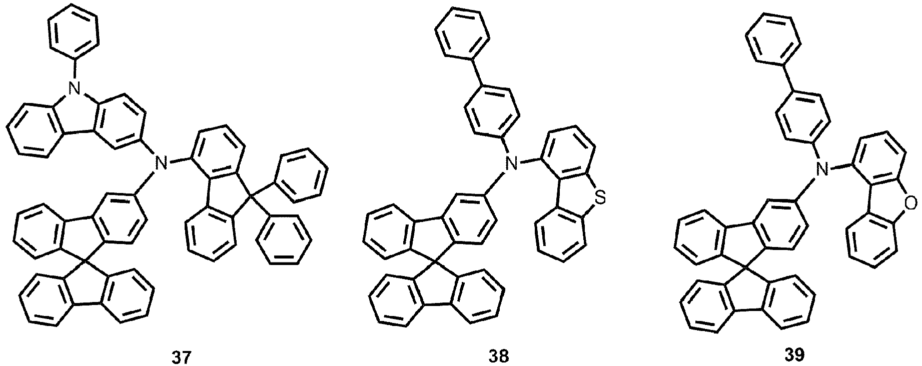

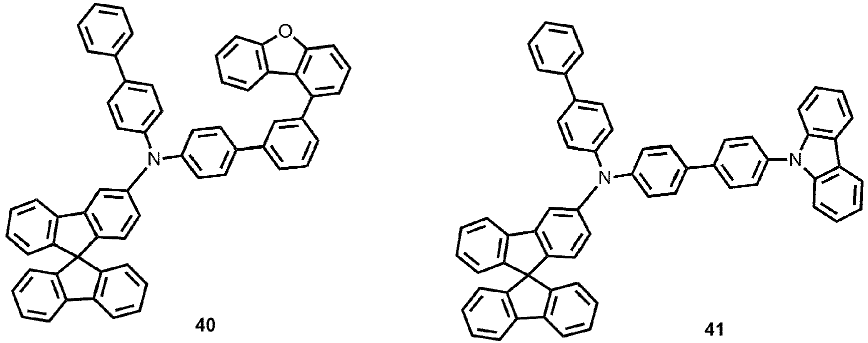

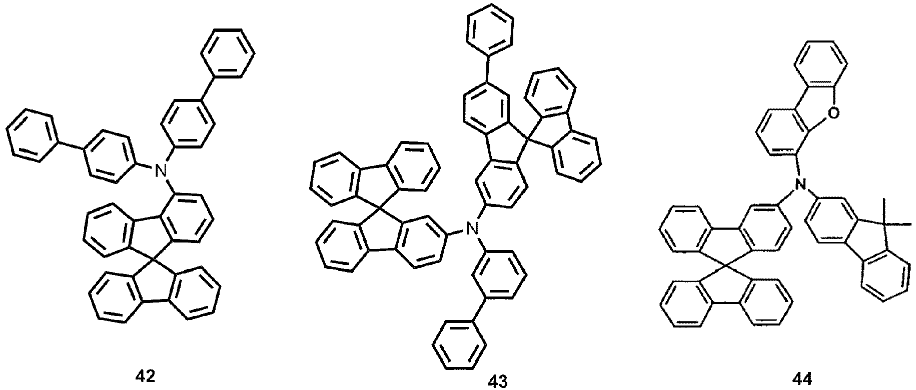

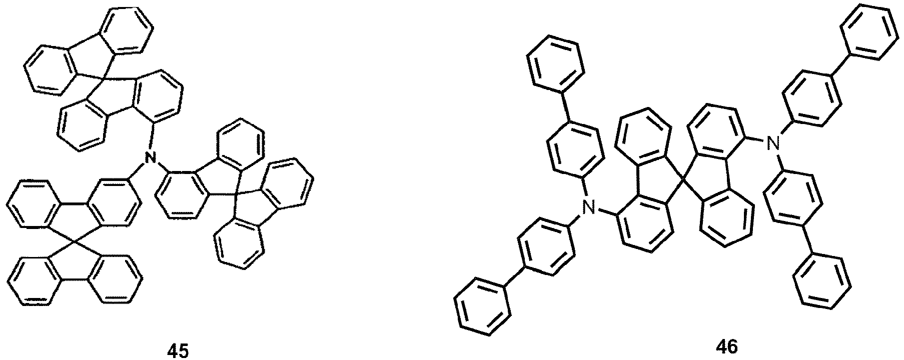

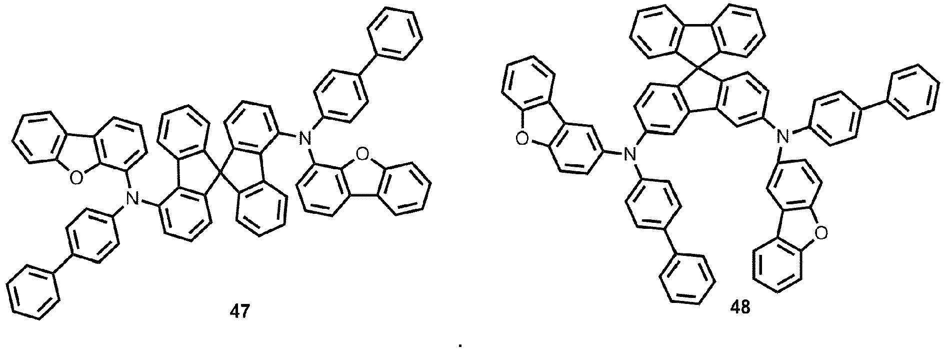

In an organic light-emitting device, when the amount of holes injected into the emission layer is smaller than the amount of the injected electrons, excitons generated in the emission layer may collide with excess electrons. In this regard, exciton-polaron quenching may occur in the emission layer, and the efficiency of the organic light-emitting device may decrease as the excitons dissociate in the emission layer.

In addition, difference between the amount of injected holes and the amount of injected electrons may increase at higher current, and a roll-off phenomenon (in which efficiency significantly decreases at higher current) may occur.

The amine-based compound represented by Formula 1 increases a charge balance in the emission layer by facilitating injection of holes into the emission layer in the hole transport region 130. Thus, when an organic light-emitting device includes the amine-based compound represented by Formula 1, the efficiency of the organic light-emitting device may increase, and a roll-off phenomenon may decrease.

Also, the amine-based compound represented by Formula 1 has high thermal stability, and thus the organic light-emitting device may have a small efficiency change with respect to a change in temperature.

A thickness of the auxiliary layer may be in a range of about 10 Å to about 800 Å, for example about 50 Å to about 500 Å. When a thickness of the auxiliary layer is within any of these ranges, satisfactory hole transporting properties of the auxiliary layer may be obtained without a substantial increase in driving voltage.

An emission layer is formed on the hole transport region 130 by using one or more suitable methods, such as vacuum deposition, spin coating, casting, a LB method, ink-jet printing, laser-printing, and/or laser-induced thermal imaging. When the emission layer is formed by vacuum deposition and/or spin coating, deposition and coating conditions for the emission layer may be similar to the deposition and coating conditions for the hole injection layer.

When the organic light-emitting device 10 is a full-color organic light-emitting device, the emission layer may be patterned into a red emission layer, a green emission layer, and a blue emission layer. Alternatively, the emission layer may emit white light by having a multi-layer structure, in which a red emission layer, a green emission layer, and a blue emission layer are stacked on each other, or a single-layer structure including a red-light emitting material, a green-light emitting material, and a blue-light emitting material to emit white light. The emission layer may be a white emission layer and may further include a color converting layer or a color filter to convert white light to light of desired color.

The emission layer may include a host and a dopant.

The host may include at least one selected from TPBi, TBADN, ADN, CBP, CDBP, and TCP:

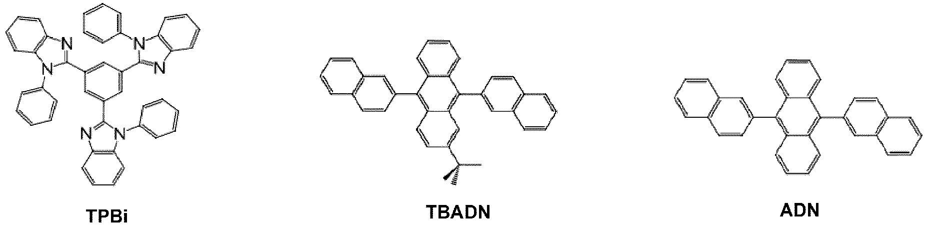

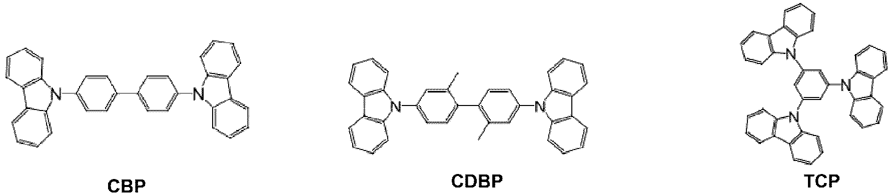

In some embodiments, the host may include a compound represented by Formula 301: Formula 301

Ar₃₀₁-(L₃₀₁)_{xb1}R₃₀₁]_{xb2}.

In Formula 301,
Ar₃₀₁ is selected from:
a naphthalene, a heptalene, a fluorene, a spiro-fluorene, a benzofluorene, a dibenzofluorene, a phenalene, a phenanthrene, an anthracene, a fluoranthene, a triphenylene, a pyrene, a chrysene, a naphthacene, a picene, a perylene, a pentaphene, and an indenoanthracene;
a naphthalene, a heptalene, a fluorene, a spiro-fluorene, a benzofluorene, a dibenzofluorene, a phenalene, a phenanthrene, an anthracene, a fluoranthene, a triphenylene, a pyrene, a chrysene, naphthacene, a picene, a perylene, a pentaphene, and an indenoanthracene, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -Si(Q₃₀₁)(Q₃₀₂)(Q₃₀₃) (where Q₃₀₁ to Q₃₀₃ may be each independently selected from hydrogen, a C₁-C₆₀ alkyl group, a C₂-C₆₀ alkenyl group, a C₆-C₆₀ aryl group, and a C₁-C₆₀ heteroaryl group);
L₃₀₁ is defined the same as the definition provided in connection with L₂₀₁;
R₃₀₁ is selected from:
a C₁-C₂₀ alkyl group and a C₁-C₂₀ alkoxy group;
a C₁-C₂₀ alkyl group and a C₁-C₂₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
xb1 is selected from 0, 1, 2, and 3;
xb2 is selected from 1, 2, 3, and 4.

For example, in Formula 301,
L₃₀₁ may be selected from:
a phenylene group, a naphthylene group, a fluorenylene group, a spiro-fluorenylene group, a benzofluorenylene group, a dibenzofluorenylene group, a phenanthrenylene group, an anthracenylene group, a pyrenylene group, and a chrysenylene group; and
a phenylene group, a naphthylene group, a fluorenylene group, a spiro-fluorenylene group, a benzofluorenylene group, a dibenzofluorenylene group, a phenanthrenylene group, an anthracenylene group, a pyrenylene group, and a chrysenylene group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, and a chrysenyl group;
R₃₀₁ may be selected from:
a C₁-C₂₀ alkyl group and a C₁-C₂₀ alkoxy group;
a C₁-C₂₀ alkyl group and a C₁-C₂₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, and a chrysenyl group;
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, and a chrysenyl group; and
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, and a chrysenyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, and a chrysenyl group.

The compound represented by Formula 301 may include at least one of Compounds H1 to H42:

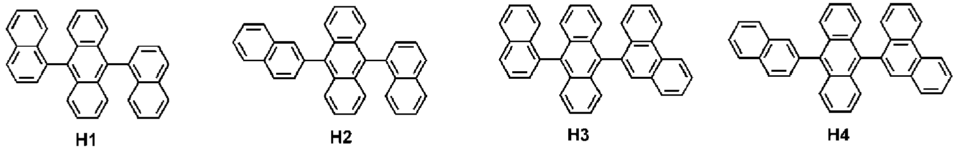

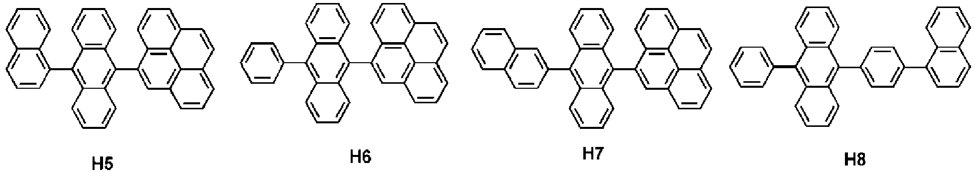

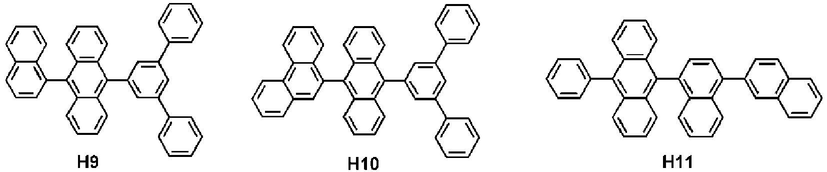

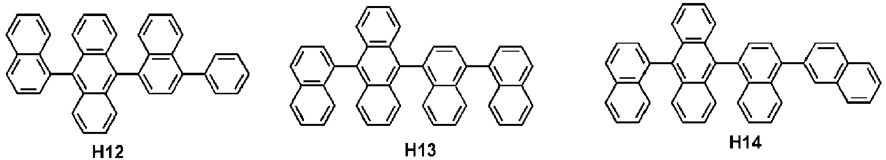

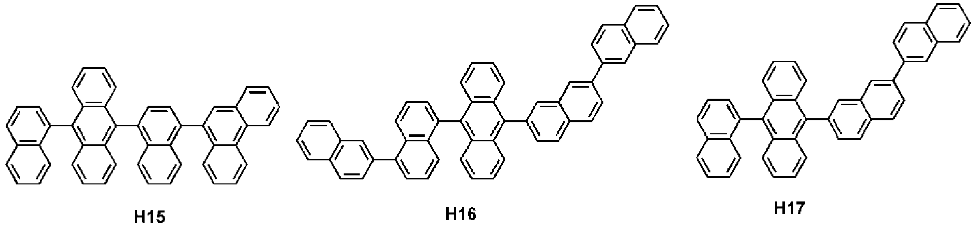

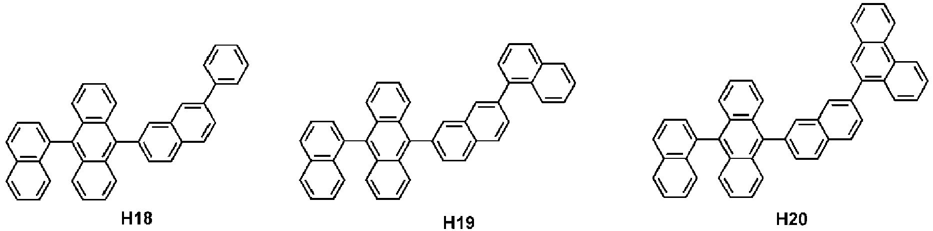

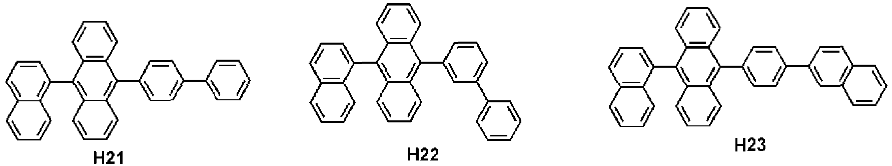

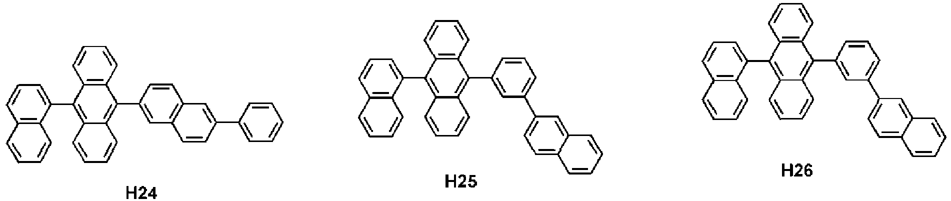

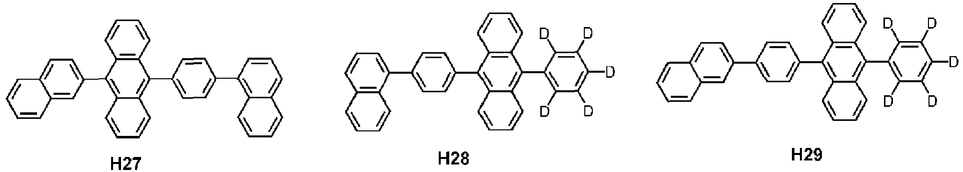

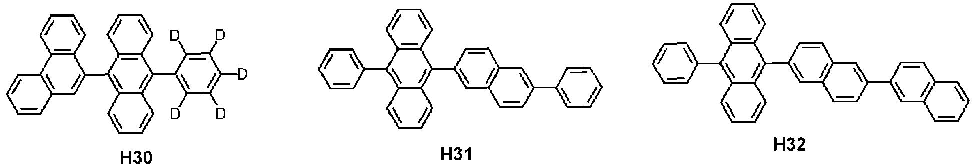

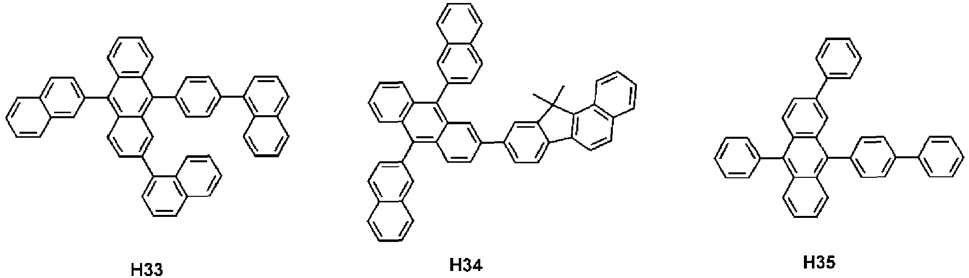

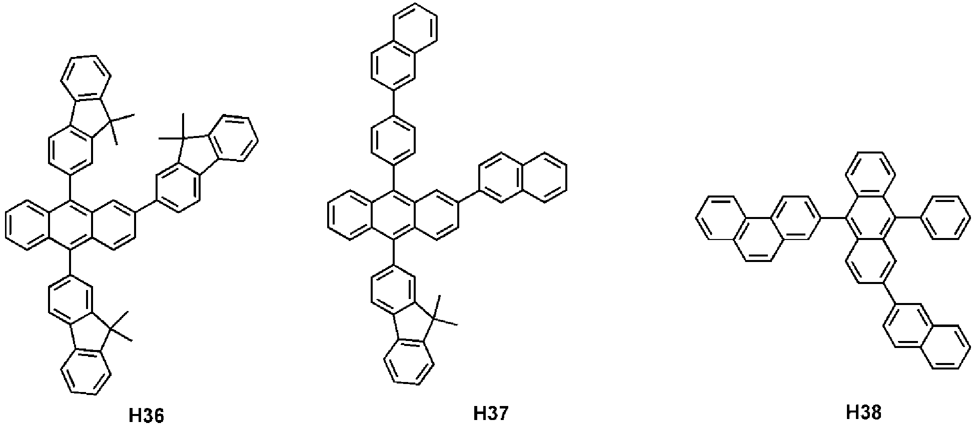

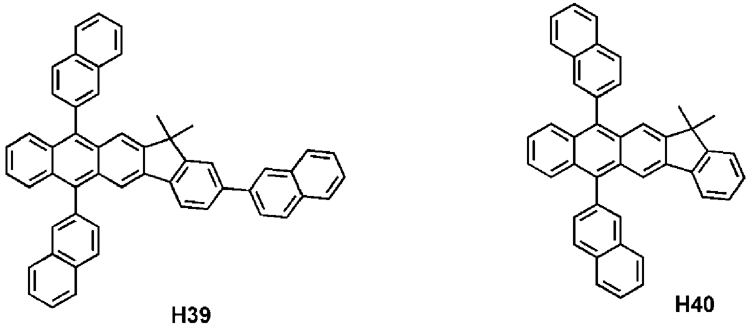

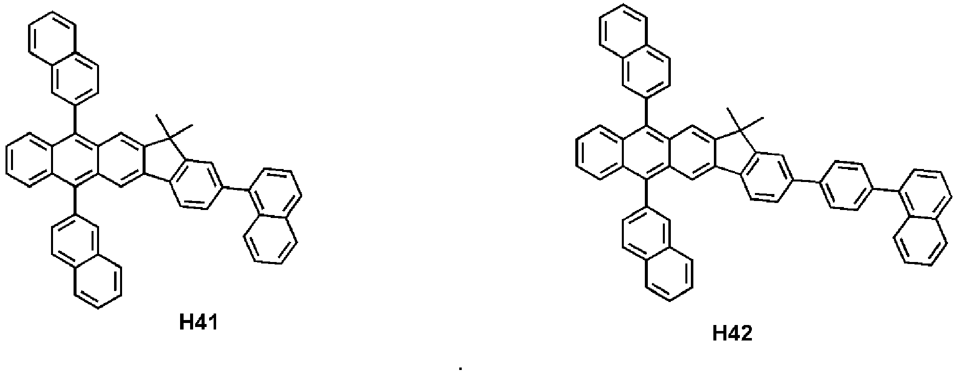

Alternatively, the host may include at least one of Compounds H43 to H49:

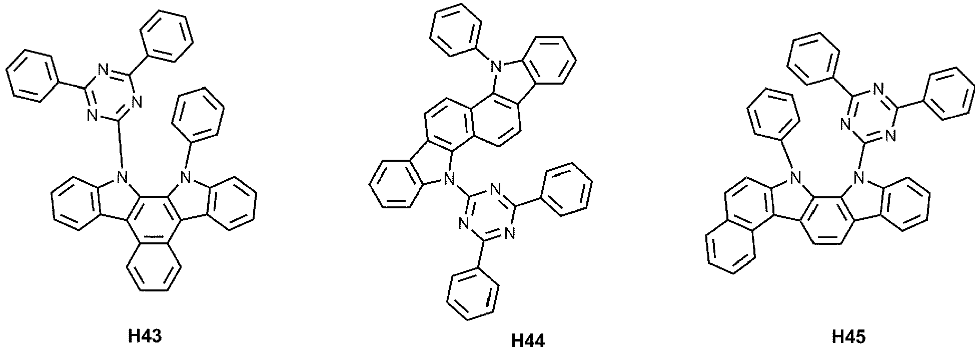

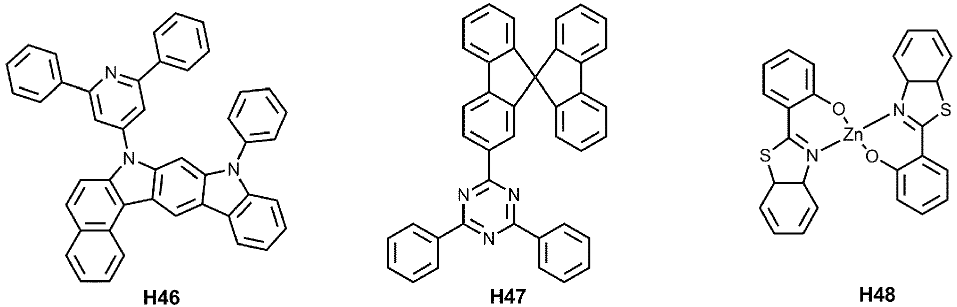

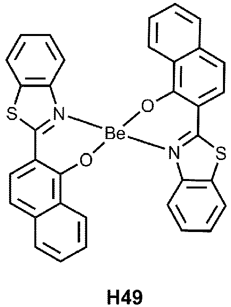

The dopant may include at least one of a fluorescent dopant and a phosphorescent dopant.

The phosphorescent dopant may include an organometallic compound represented by Formula 401:

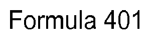

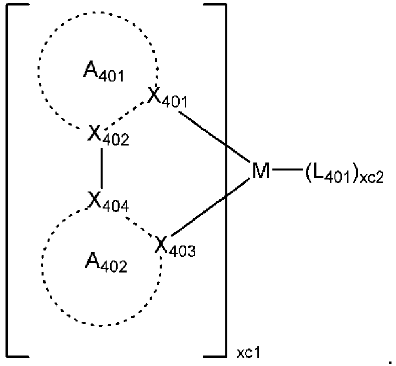

In Formula 401,
M is selected from iridium (Ir), platinum (Pt), osmium (Os), titanium (Ti), zirconium (Zr), hafnium (Hf), europium (Eu), terbium (Tb), and thulium (Tm);
X₄₀₁ to X₄₀₄ are each independently nitrogen (-N-) or carbon (-C-);
rings A₄₀₁ and A₄₀₂ are each independently selected from a substituted or unsubstituted benzene, a substituted or unsubstituted naphthalene, a substituted or unsubstituted fluorene, a substituted or unsubstituted spiro-fluorene, a substituted or unsubstituted indene, a substituted or unsubstituted pyrrole, a substituted or unsubstituted thiophene, a substituted or unsubstituted furan, a substituted or unsubstituted imidazole, a substituted or unsubstituted pyrazole, a substituted or unsubstituted thiazole, a substituted or unsubstituted isothiazole, a substituted or unsubstituted oxazole, a substituted or unsubstituted isoxazole, a substituted or unsubstituted pyridine, a substituted or unsubstituted pyrazine, a substituted or unsubstituted pyrimidine, a substituted or unsubstituted pyridazine, a substituted or unsubstituted quinoline, a substituted or unsubstituted isoquinoline, a substituted or unsubstituted benzoquinoline, a substituted or unsubstituted quinoxaline, a substituted or unsubstituted quinazoline, a substituted or unsubstituted carbazole, a substituted or unsubstituted benzimidazole, a substituted or unsubstituted benzofuran, a substituted or unsubstituted benzothiophene, a substituted or unsubstituted isobenzothiophene, a substituted or unsubstituted benzoxazole, a substituted or unsubstituted isobenzoxazole, a substituted or unsubstituted triazole, a substituted or unsubstituted oxadiazole, a substituted or unsubstituted triazine, a substituted or unsubstituted dibenzofuran and a substituted or unsubstituted dibenzothiophene;
at least one substituent of the substituted benzene, substituted naphthalene, substituted fluorene, substituted spiro-fluorene, substituted indene, substituted pyrrole, substituted thiophene, substituted furan, substituted imidazole, substituted pyrazole, substituted thiazole, substituted isothiazole, substituted oxazole, substituted isoxazole, substituted pyridine, substituted pyrazine, substituted pyrimidine, substituted pyridazine, substituted quinoline, substituted isoquinoline, substituted benzoquinoline, substituted quinoxaline, substituted quinazoline, substituted carbazole, substituted benzimidazole, substituted benzofuran, substituted benzothiophene, substituted isobenzothiophene, substituted benzoxazole, substituted isobenzoxazole, substituted triazole, substituted oxadiazole, substituted triazine, substituted dibenzofuran, and substituted dibenzothiophene is selected from:
deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₄₀₁)(Q₄₀₂), -Si(Q₄₀₃)(Q₄₀₄)(Q₄₀₅), and-B(Q₄₀₆)(Q₄₀₇);
a C₃-C₁₀ cycloalkyl group, a C₁-C₁₀ heterocycloalkyl, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀heterocycloalkyl, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -CI, -Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₄₁₁)(Q₄₁₂), -Si(Q₄₁₃)(Q₄₁₄)(Q₄₁₅), and -B(Q₄₁₆)(Q₄₁₇); and -N(Q₄₂₁)(Q₄₂₂), -Si(Q₄₂₃)(Q₄₂₄)(Q₄₂₅), and -B(Q₄₂₆)(Q₄₂₇);
L₄₀₁ is an organic ligand;
xc1 is 1, 2, or 3;
xc2 is 0, 1, 2, or 3; and
Q₄₀₁ to Q₄₀₇, Q₄₁₁ to Q₄₁₇, and Q₄₂₁ to Q₄₂₇ may be each independently selected from hydrogen, a C₁-C₆₀ alkyl group, a C₂-C₆₀ alkenyl group, a C₆-C₆₀ aryl group, and a C₁-C₆₀ heteroaryl group.

In Formula 401, L₄₀₁ may be a monovalent, divalent, or trivalent organic ligand. For example, L₄₀₁ may be selected from a halogen ligand (for example, Cl and/or F), a diketone ligand (for example, acetylacetonate, 1,3-diphenyl-1,3-propanedionate, 2,2,6,6-tetramethyl-3,5-heptanedionate, and/or hexafluoroacetonate), a carboxylic acid ligand (for example, picolinate, dimethyl-3-pyrazolecarboxylate, and/or benzoate), a carbon monoxide ligand, an isonitrile ligand, a cyano ligand, and a phosphorus ligand (for example, phosphine and/or phosphite).

In Formula 401, when A₄₀₁ has two or more substituents, the two or more substituents of A₄₀₁ may be linked to each other to form a saturated or unsaturated ring.

In Formula 401, when A₄₀₂ has two or more substituents, the two or more substituents of A₄₀₂ may be linked to each other to form a saturated or unsaturated ring.

In Formula 401, when xc1 is 2 or greater, a plurality of ligands 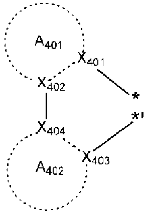 in Formula 401 may be identical to or different from each other. In Formula 401, when xc1 is 2 or greater, A₄₀₁ and/or A₄₀₂ may be respectively linked to A₄₀₁ and/or A₄₀₂ of a different neighboring ligand either directly (e.g., via a single bond) or via a linking group (e.g., a C₁-C₅ alkylene group, a C₂-C₅ alkenylene group, -N(R')- (where, R' is the C₁-C₁₀ alkyl group or the C₆-C₂₀ aryl group), or -C(=O)-) therebetween.

The phosphorescent dopant may include at least one of Compounds PD1 to PD74:

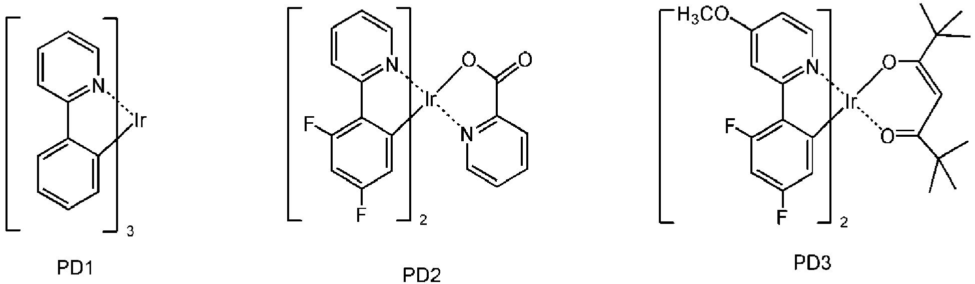

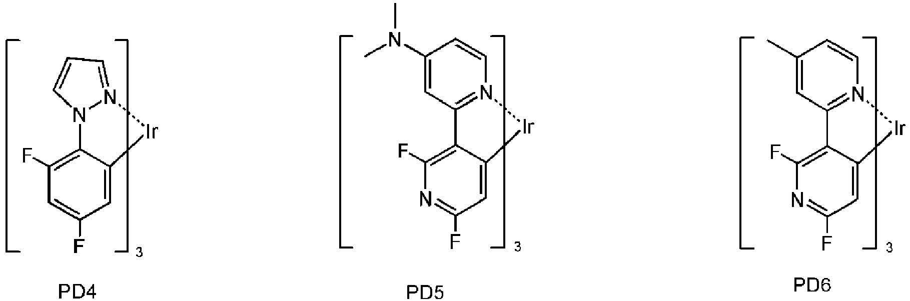

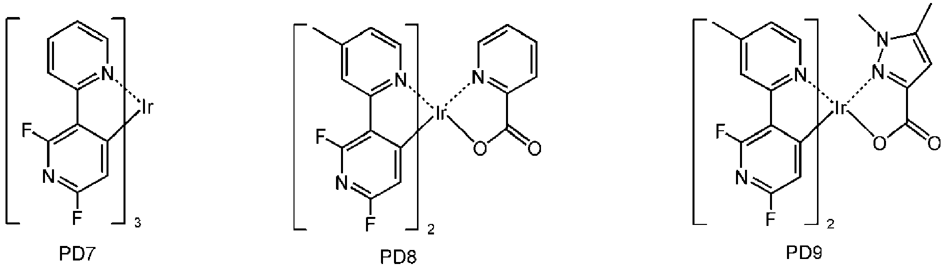

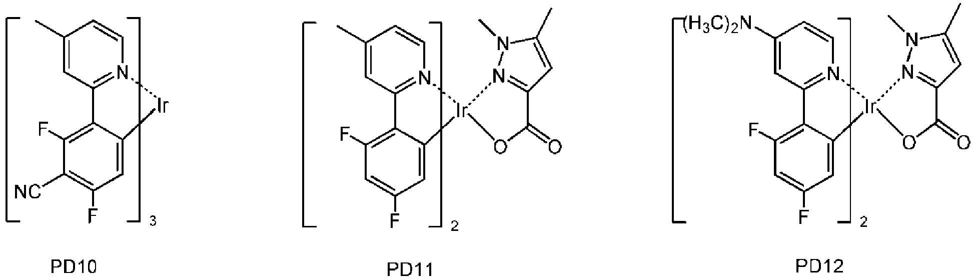

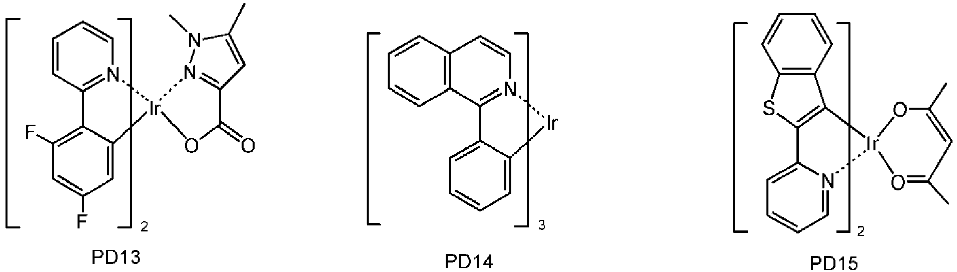

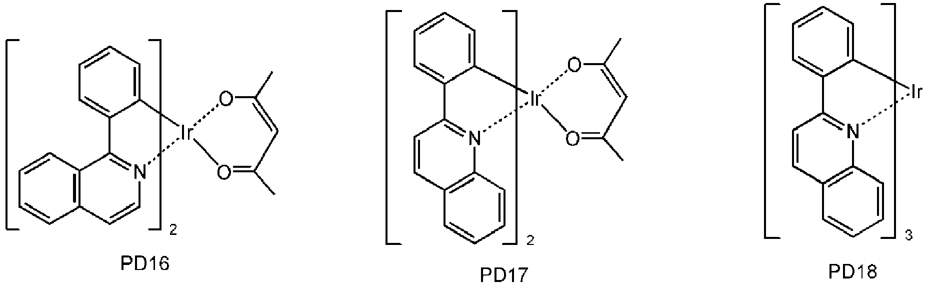

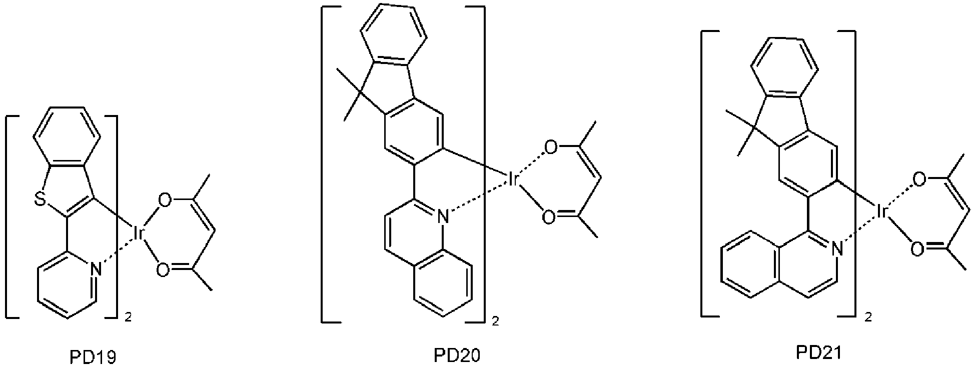

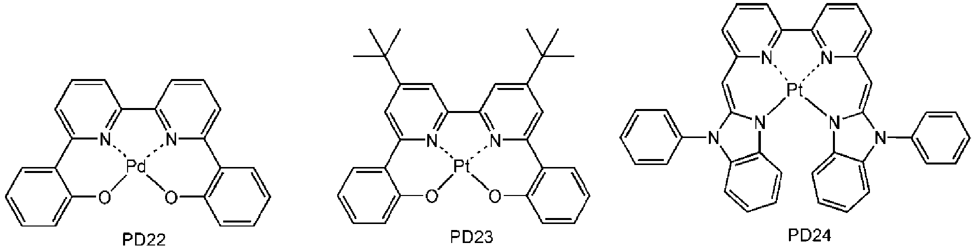

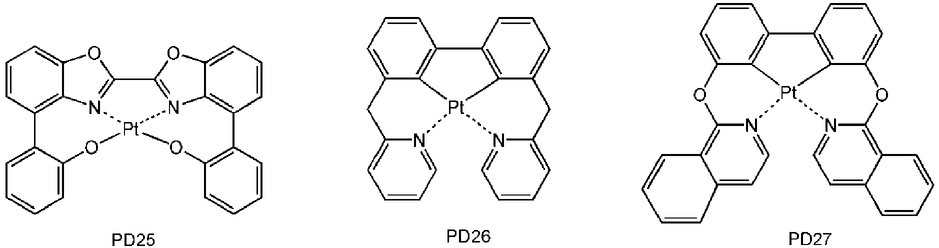

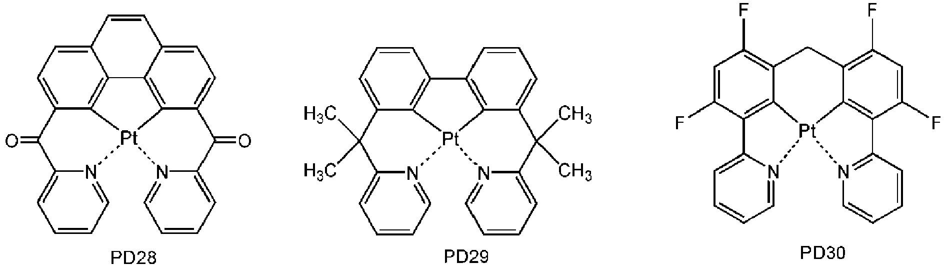

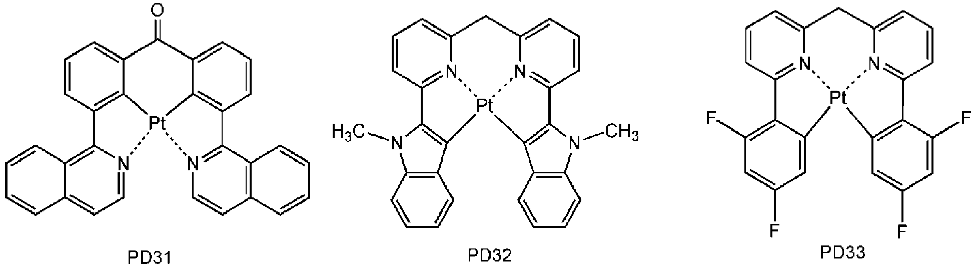

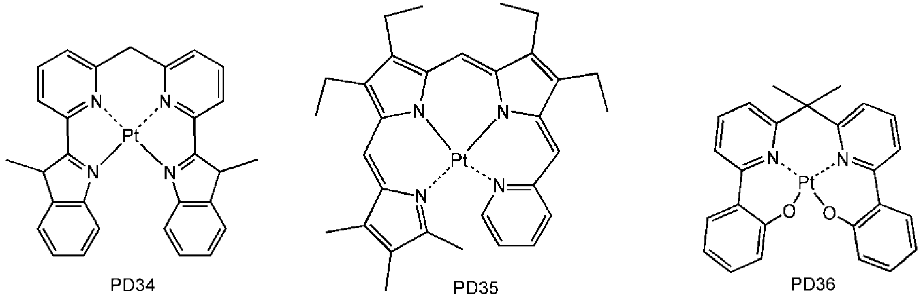

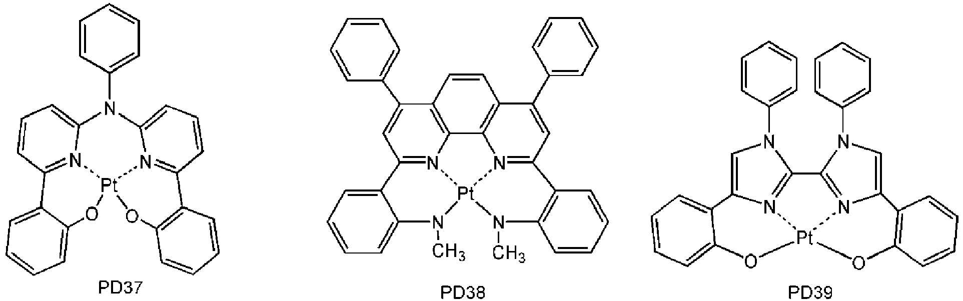

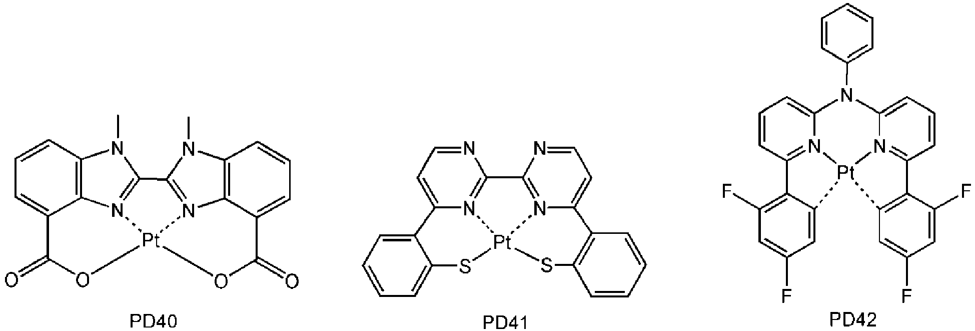

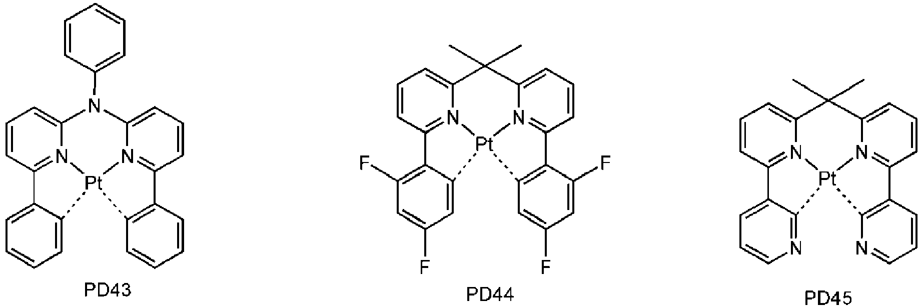

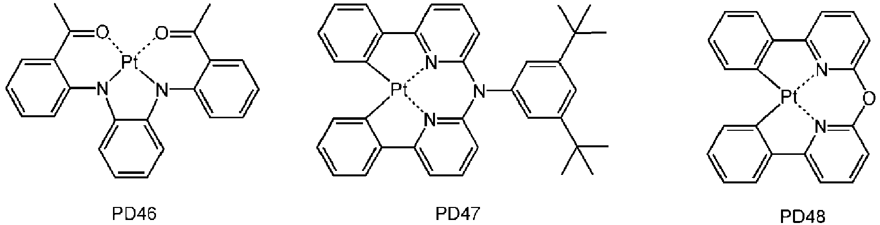

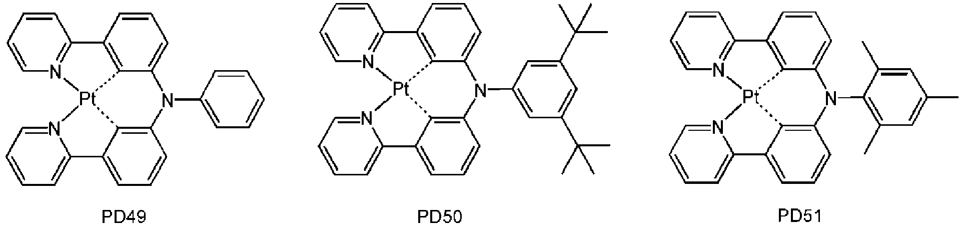

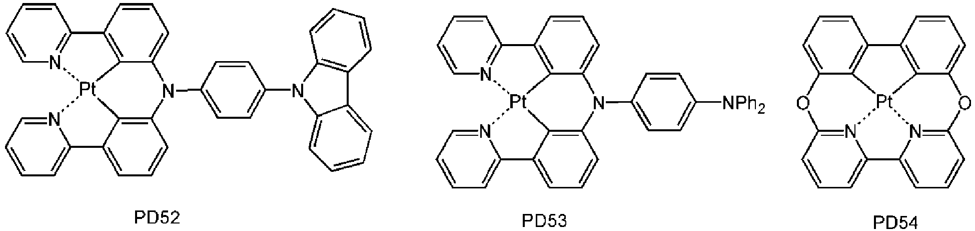

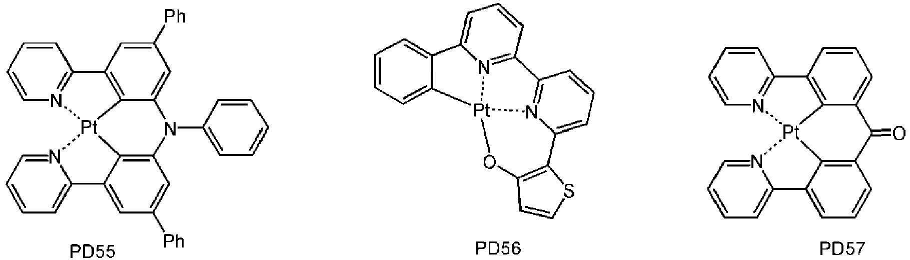

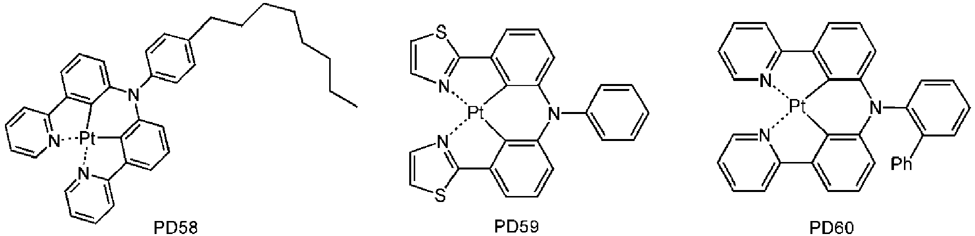

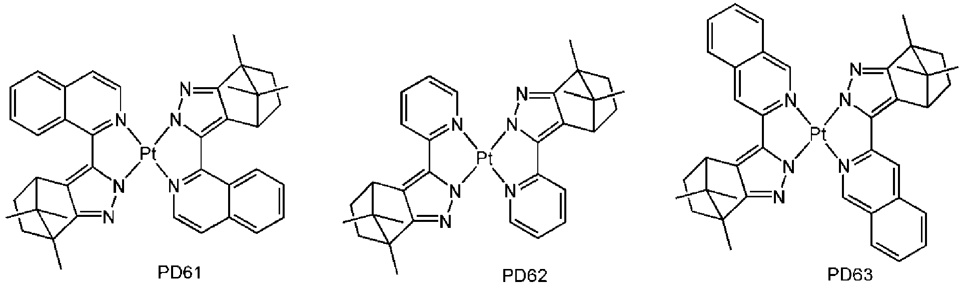

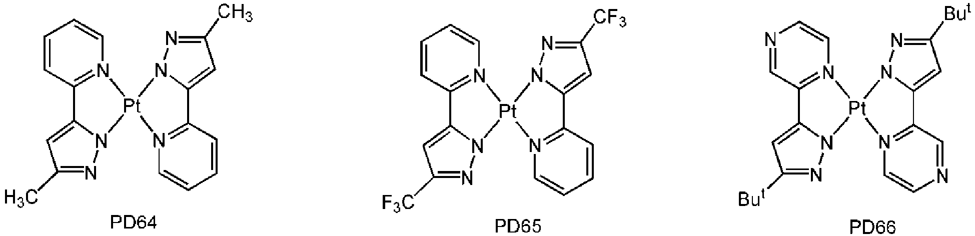

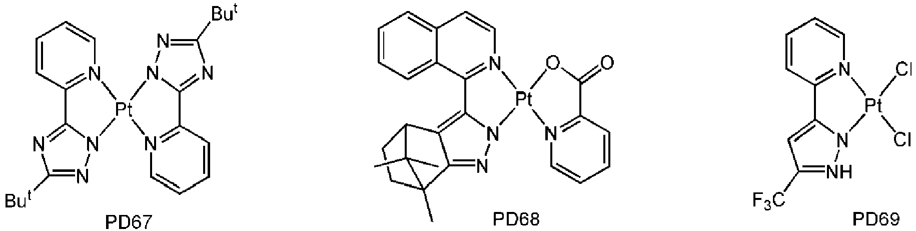

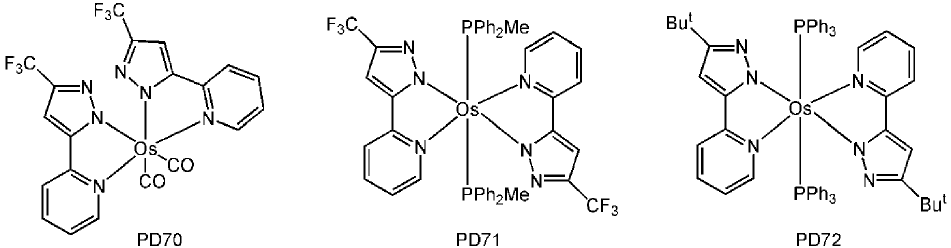

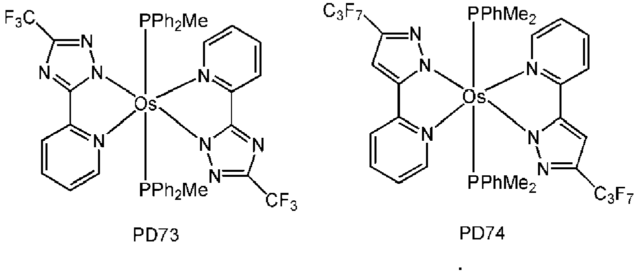

In some embodiments, the phosphorescent dopant may include PtOEP:

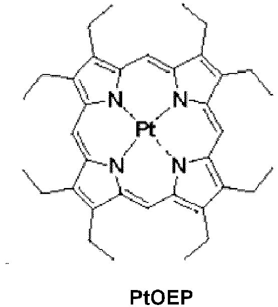

In the emission layer, the amount of the dopant may be within a range of about 0.01 to about 15 parts by weight based on 100 parts by weight of the host.

A thickness of the emission layer may be in a range of about 100 Å to about 1000 Å, for example, about 200 Å to about 600 Å. When the thickness of the emission layer is within any of these ranges, the emission layer may have excellent light-emitting properties without a substantial increase in driving voltage.

The emission layer may be a green emission layer.

The electron transport region 180 may be positioned on the emission layer.

The electron transport region 180 may include at least one selected from a hole blocking layer, an electron transport layer (ETL), and an electron injection layer.

For example, the electron transport region 180 may have a structure of electron transport layer/electron injection layer or a structure of hole blocking layer/electron transport layer/electron injection layer, wherein the layers of each structure are sequentially stacked on the emission layer in the stated order.

The electron transport region 180 may include a hole blocking layer. When a phosphorescent dopant is included in the emission layer, the hole blocking layer may be included to prevent or substantially block the diffusion of triplet excitons or holes into an electron transport layer.

When the electron transport region 180 includes a hole blocking layer, the hole blocking layer may be formed on the emission layer by using one or more suitable methods, such as vacuum deposition, spin coating, casting, a Langmuir-Blodgett (LB) method, ink-jet printing, laser-printing, and/or laser-induced thermal imaging (LITI). When the hole blocking layer is formed by vacuum deposition and/or by spin coating, the deposition conditions and the coating conditions may be similar to the deposition conditions and the coating conditions for forming the hole injection layer.

For example, the hole blocking layer may include at least one selected from BCP and Bphen.

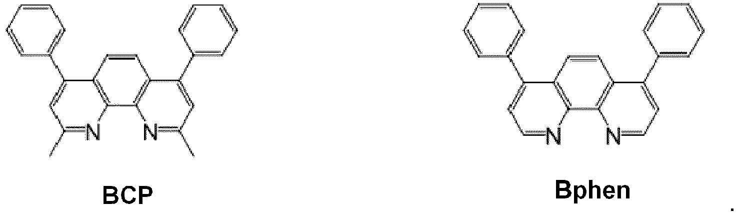

A thickness of the hole blocking layer may be in a range of about 20 Å to about 1000 Å, for example, about 30 Å to about 300 Å. When the thickness of the hole blocking layer is within any of these ranges, excellent hole blocking characteristics of the hole blocking layer may be obtained without a substantial increase in driving voltage.

The electron transport region 180 may include an electron transport layer. The electron transport layer may be formed on the emission layer or hole blocking layer by using one or more suitable methods, such as vacuum deposition, spin coating, casting, a LB method, ink-jet printing, laser-printing, and/or laser-induced thermal imaging. When the electron transport layer is formed by vacuum deposition and/or spin coating, vacuum deposition and coating conditions for the electron transport layer may be similar to the vacuum deposition and coating conditions for the hole injection layer.

The electron transport layer may include at least one selected from BCP and BPhen (illustrated above), and Alq₃, Balq, TAZ, and NTAZ (illustrated below):

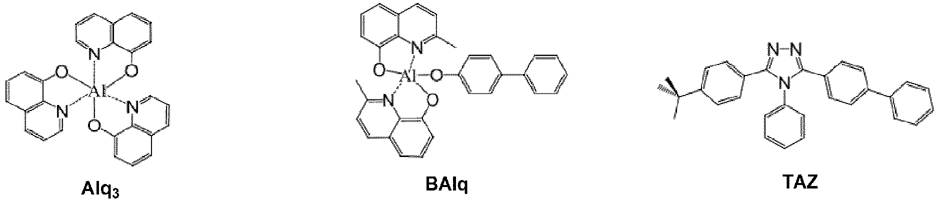

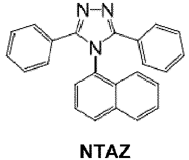

In some embodiments, the electron transport layer may include a compound represented by Formula 601:

Formula 601 Ar₆₀₁-(L₆₀₁)ₓₑ₁-E₆₀₁]ₓₑ₂.

In Formula 601,
Ar₆₀₁ is defined the same as the definition provided in connection with Ar₃₀₁;
L₆₀₁ is defined the same as the definition provided in connection with L₂₀₁; and
E₆₀₁ is selected from:
a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group; and
a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group;
xe1 is selected from 0, 1, 2, and 3; and
xe2 is selected from 1, 2, 3, and 4.

In some embodiments, the electron transport layer may include a compound represented by Formula 602:

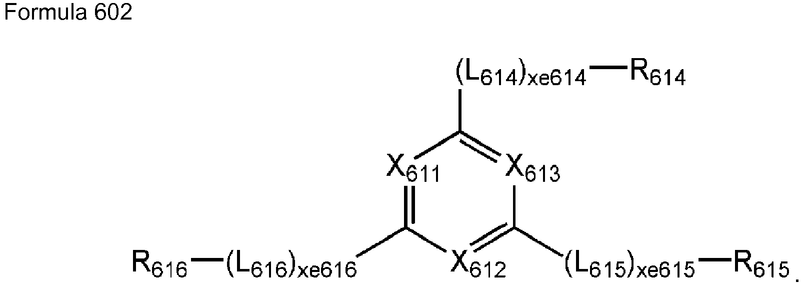

In Formula 602,
X₆₁₁ is N or C-(L₆₁₁)ₓₑ₆₁₁R₆₁₁, X₆₁₂ is N or C-(L₆₁₂)ₓₑ₆₁₂-R₆₁₂, X₆₁₃ is N or C-(L₆₁₃)ₓₑ₆₁₃-R₆₁₃, and at least one of X₆₁₁ to X₆₁₃ is N;
each of L₆₁₁ to L₆₁₆ is defined the same as the definition provided in connection with L₂₀₁;

R₆₁₁ to R₆₁₆ are each independently selected from:
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, an azulenyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
xe611 to xe616 are each independently selected from 0, 1, 2, and 3.

A compound represented by Formula 601 and a compound represented by Formula 602 may each independently include at least one of Compounds ET1 to ET15:

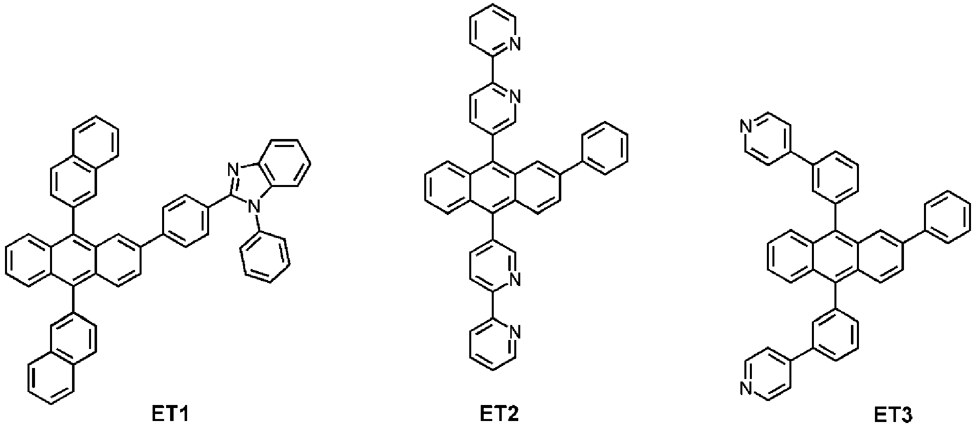

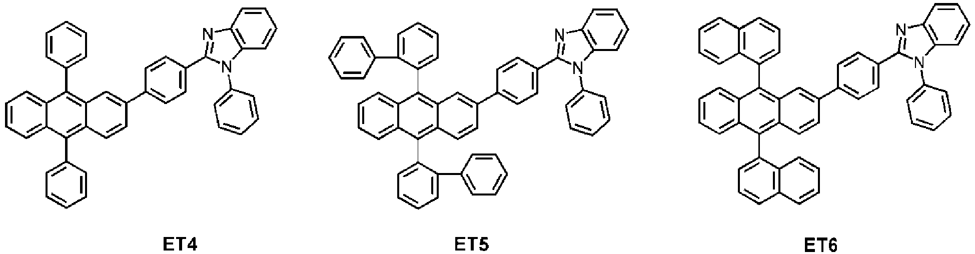

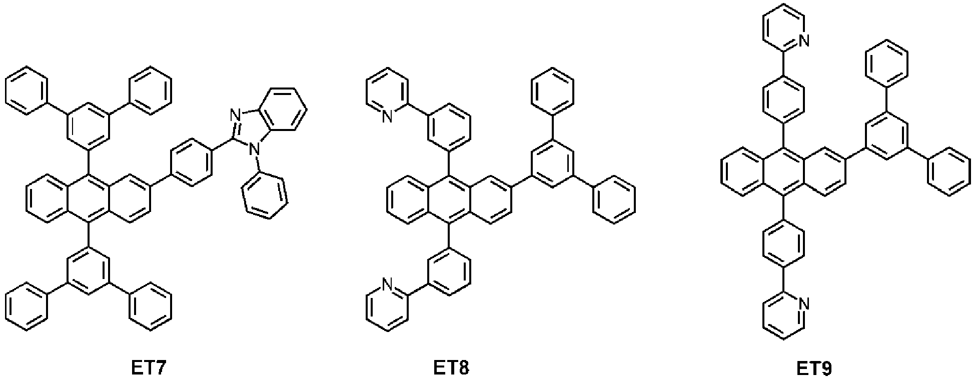

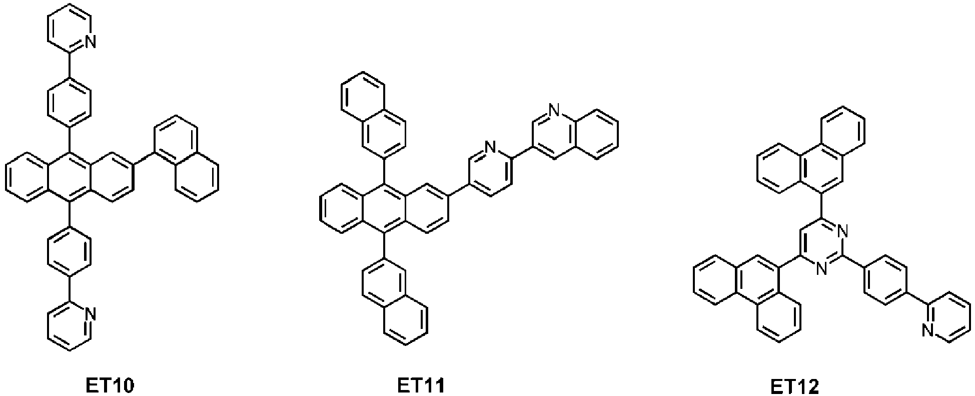

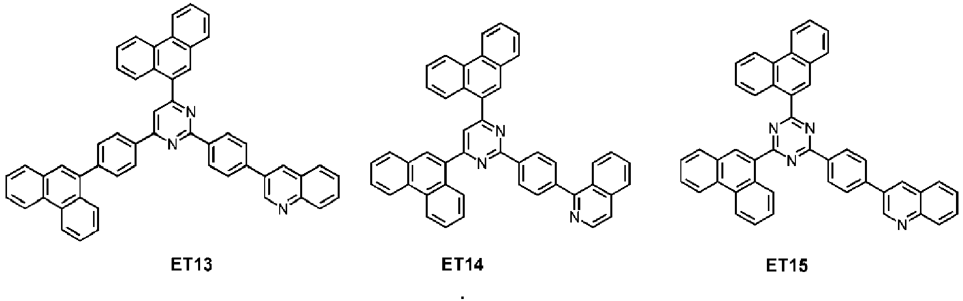

A thickness of the electron transport layer may be in a range of about 100 Å to about 1000 Å, for example, about 150 Å to about 500 Å. When the thickness of the electron transport layer is within any of these ranges, excellent electron transporting characteristics of the electron transport layer may be obtained without a substantial increase in driving voltage.

In some embodiments, the electron transport layer may further include a metal-containing material, in addition to the materials described above.

The metal-containing material may include a Li complex. The Li complex may include, for example, Compound ET-D1 (lithium quinolate, LiQ) and/or ET-D2:

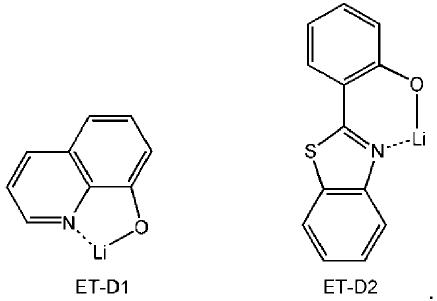

The electron transport region 180 may include an electron injection layer that facilitates electron injection from the second electrode 190.

The electron injection layer may be formed on the electron transport layer by using one or more suitable methods, such as vacuum deposition, spin coating, casting, a LB method, ink-jet printing, laser-printing, and/or LITI. When the electron injection layer is formed by vacuum deposition and/or spin coating, vacuum deposition and coating conditions for the electron injection layer may be similar to the vacuum deposition and coating conditions for the hole injection layer.

The electron injection layer may include at least one selected from LiF, NaCl, CsF, Li₂O, BaO, and LiQ.

A thickness of the electron injection layer may be in a range of about 1 Å to about 100 Å, for example, about 3 Å to about 90 Å. When the thickness of the electron injection layer is within any of these ranges, excellent electron injecting characteristics of the electron injection layer may be obtained without a substantial increase in driving voltage.

The second electrode 190 may be positioned on the organic layer 150. The second electrode 190 may be a cathode that is an electron injection electrode, and, in this regard, a material for forming the second electrode 190 may be a material having a low work function, for example, a metal, an alloy, an electrically conductive compound, or a mixture thereof. Non-limiting examples of the material for forming the second electrode 190 may include lithium (Li), magnesium (Mg), aluminum (Al), aluminum-lithium (Al-Li), calcium (Ca), magnesium-indium (Mg-In), and magnesium-silver (Mg-Ag). In some embodiments, the material for forming the second electrode 190 may be ITO and/or IZO. The second electrode 190 may be a reflective electrode, a semi-transmissive electrode, or a transmissive electrode.

The auxiliary layer 140 is located in the hole transport region 130.

The organic light-emitting device 10 according to one or more embodiments of the present invention has been described with reference to FIG. 1.

FIG. 2 schematically illustrates a cross-section of an organic light-emitting device 20 according to one or more embodiments of the present invention.

Referring to FIG. 2, an auxiliary layer 240 may be located adjacent to an emission layer 260. Materials included in the auxiliary layer 240 are different from the materials included in a hole transport layer.

Descriptions of a first electrode 210, a hole transport region 230, the auxiliary layer 240, an organic layer 250, an emission layer 260, an electron transport region 280, and a second electrode 290 may be understood by referring to the above descriptions thereof in connection with FIG. 1.

The organic light-emitting device 20 according to one or more embodiments of the present invention may be described with reference to FIG. 2.

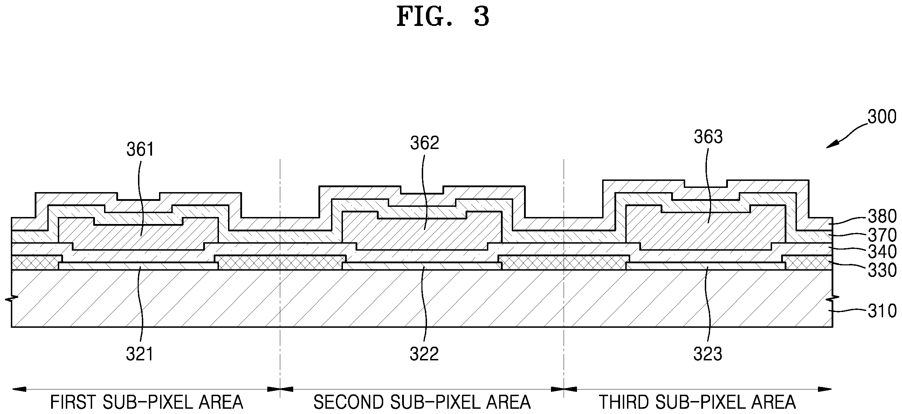

FIG. 3 schematically illustrates a cross-section of a full-color organic light-emitting device 30 according to one or more embodiments of the present invention.

The organic light-emitting device 30 of FIG. 3 includes a first sub-pixel area, a second sub-pixel area, and a third sub-pixel area.

A first sub-pixel is formed in the first sub-pixel area, a second sub-pixel is formed in the second sub-pixel area, and a third sub-pixel is formed in the third sub-pixel area.

A plurality of first electrodes 321, 322, and 323 are located in the first sub-pixel area, the second sub-pixel area, and the third sub-pixel area, respectively. That is, the first electrode 321 is located in the first sub-pixel area, the first electrode 322 is located in the second sub-pixel area, and the first electrode 323 is located in the third sub-pixel area.

A hole transport region 340 is positioned on the first electrodes 321, 322, and 323. The hole transport region 340 may be formed as a common layer on all of the first electrodes 321, 322, and 323. The hole transport region 340 may include a first hole transport region formed in the first sub-pixel area; a second hole transport region formed in the second sub-pixel area; and a third hole transport region formed in the third sub-pixel area.

An emission layer including a first emission layer 361, a second emission layer 362, and a third emission layer 363 is formed on the hole transport region 340. The first emission layer 361 is formed in the first sub-pixel area and emits first-color light, the second emission layer 362 is formed in the second sub-pixel area and emits second-color light, and the third emission layer 363 is formed in the third sub-pixel area and emits third-color light.

The first-color light may be red light, the second-color light may be green light, and the third-color light may be blue light. The first-color light, second-color light, and third-color light may be combined together to produce white light.

The hole transport region 340 includes a hole injection layer, a hole transport layer and an auxiliary layer, and the auxiliary layer is positioned between the hole transport layer and each of the emission layers 361, 362, and 363. The auxiliary layer is located adjacent to each of the emission layers 361, 362, and 363.

The auxiliary layer may include a first auxiliary layer, a second auxiliary layer, and a third auxiliary layer. The first auxiliary layer is formed in the first sub-pixel area, the second auxiliary layer is formed in the second sub-pixel area, and the third auxiliary layer is formed in the third sub-pixel area.

The auxiliary layer includes the amine-based compound represented by Formula 1.

The hole transport layer consists of a first hole transporting compound represented by one of Formulae 201A and 202A.

Materials included in the auxiliary layer are different from the materials included in the hole transport layer.

An electron transport region 370 is formed on each of the emission layers 361, 362, and 363. The electron transport region 370 may be formed as a common layer on all of the emission layers 361, 362, and 363. The electron transport region 370 may include an electron transport layer and an electron injection layer that are stacked on the emission layers 361, 362, and 363 in the stated order.

A second electrode 380 is formed as a common layer on the electron transport region 370.

As used herein, the term "common layer" refers to a layer that is not patterned according to each of the first sub-pixel area, the second sub-pixel area, and the third sub-pixel area, but instead is formed as a continuous layer throughout the first sub-pixel area, the second sub-pixel area, and the third sub-pixel area.

A pixel insulating layer 330 is formed on an edge portion of the plurality of first electrodes 321, 322, and 323. The pixel insulating layer 330 defines a pixel area and may include various suitable organic insulating materials (e.g., a silicon-based material), inorganic insulating materials, and/or organic/inorganic composite insulating materials.

Descriptions of the first electrodes 321, 322, and 323, hole transport region 340, emission layers 361,362, and 363, electron transport region 370, and second electrode 380 may be understood by referring to the descriptions thereof provided in connection with FIG. 1.

The organic light-emitting device 30 may be included in a flat display device including a thin film transistor. The thin film transistor may include a gate electrode, source and drain electrodes, a gate insulating layer, and an active layer, and one of the source and drain electrodes may be electrically connected to the first electrodes 321, 322, and 323 of the organic light-emitting device 30. The active layer may include crystalline silicon, amorphous silicon, an organic semiconductor, or an oxide semiconductor.

The full-color organic light-emitting device 30 according to one or more embodiments of the present invention has been described with reference to FIG. 3, but embodiments of the present invention are not limited thereto. For example, the third emission layer 363 may be extended to the first sub-pixel area and the second sub-pixel area and thus may be formed as a common layer. Also, the third auxiliary layer of the third sub-pixel area may be omitted. In some embodiments, only one of the first auxiliary layer and the second auxiliary layer may be included in the full-color organic light-emitting device 30.

A C₁-C₆₀ alkyl group used herein refers to a linear or branched aliphatic monovalent hydrocarbon group having 1 to 60 carbon atoms, and non-limiting examples of the C₁-C₆₀ alkyl group may include a methyl group, an ethyl group, a propyl group, an isobutyl group, a sec-butyl group, a tert-butyl group, a pentyl group, an iso-amyl group, and a hexyl group. A C₁-C₆₀ alkylene group used herein refers to a divalent group having substantially the same structure as the C₁-C₆₀ alkyl group.

A C₁-C₆₀ alkoxy group used herein refers to a monovalent group represented by -OA₁₀₁ (where A₁₀₁ is the C₁-C₆₀ alkyl group), and non-limiting examples of the C₁-C₆₀ alkoxy group may include a methoxy group, an ethoxy group, and an isopropyloxy group.

A C₂-C₆₀ alkenyl group used herein refers to a hydrocarbon group including at least one carbon-carbon double bond at one or more positions along a carbon chain of the C₂-C₆₀ alkyl group (e.g., in the middle or at either terminal end of the C₂-C₆₀ alkyl group), and non-limiting examples of the C₂-C₆₀ alkenyl group may include an ethenyl group, a propenyl group, and a butenyl group. A C₂-C₆₀ alkenylene group used herein refers to a divalent group having substantially the same structure as the C₂-C₆₀ alkenyl group.

A C₂-C₆₀ alkynyl group used herein refers to a hydrocarbon group including at least one carbon-carbon triple bond at one or more positions along a carbon chain of the C₂-C₆₀ alkyl group (e.g., in the middle or at either terminal end of the C₂-C₆₀ alkyl group), and non-limiting examples of the C₂-C₆₀ alkynyl group may include an ethynyl group and a propynyl group. A C₂-C₆₀ alkynylene group used herein refers to a divalent group having substantially the same structure as the C₂-C₆₀ alkynyl group.

A C₃-C₁₀ cycloalkyl group used herein refers to a monovalent monocyclic saturated hydrocarbon group including 3 to 10 carbon atoms as ring-forming atoms, and non-limiting examples of the C₃-C₁₀ cycloalkyl group may include a cyclopropyl group, a cyclobutyl group, a cyclopentyl group, a cyclohexyl group, and a cycloheptyl group. A C₃-C₁₀ cycloalkylene group used herein refers to a divalent group having substantially the same structure as the C₃-C₁₀ cycloalkyl group.

A C₁-C₁₀ heterocycloalkyl group used herein refers to a monovalent monocyclic group including at least one hetero atom selected from N, O, P, and S as a ring-forming atom, and 1 to 10 carbon atoms as the remaining ring-forming atoms, and non-limiting examples of the C₁-C₁₀ heterocycloalkyl group may include a tetrahydrofuranyl group and a tetrahydrothiophenyl group. A C₁-C₁₀ heterocycloalkylene group used herein refers to a divalent group having substantially the same structure as the C₁-C₁₀ heterocycloalkyl group.

A C₃-C₁₀ cycloalkenyl group used herein refers to a monovalent monocyclic group including 3 to 10 carbon atoms as ring-forming atoms and at least one double bond in the ring of the C₃-C₁₀ cycloalkenyl group, and does not have aromaticity (e.g., the ring including the at least one double bond is not aromatic). Non-limiting examples of the C₃-C₁₀ cycloalkenyl group may include a cyclopentenyl group, a cyclohexenyl group, and a cycloheptenyl group. A C₃-C₁₀ cycloalkenylene group used herein refers to a divalent group having substantially the same structure as the C₃-C₁₀ cycloalkenyl group.

A C₁-C₁₀ heterocycloalkenyl group used herein refers to a monovalent monocyclic group including at least one hetero atom selected from N, O, P, and S as a ring-forming atom, 1 to 10 carbon atoms as the remaining ring-forming atoms, and at least one double bond in its ring. Non-limiting examples of the C₁-C₁₀ heterocycloalkenyl group may include a 2,3-hydrofuranyl group and a 2,3-hydrothiophenyl group. A C₁-C₁₀ heterocycloalkenylene group used herein refers to a divalent group having substantially the same structure as the C₁-C₁₀ heterocycloalkenyl group.

A C₆-C₆₀ aryl group used herein refers to a monovalent group including a carbocyclic aromatic system having 6 to 60 carbon atoms as ring-forming atoms, and a C₆-C₆₀ arylene group used herein refers to a divalent group including a carbocyclic aromatic system having 6 to 60 carbon atoms as ring-forming atoms. Non-limiting examples of the C₆-C₆₀ aryl group may include a phenyl group, a naphthyl group, an anthracenyl group, a phenanthrenyl group, a pyrenyl group, and a chrysenyl group. When the C₆-C₆₀ aryl group and/or the C₆-C₆₀ arylene group include two or more rings, the respective rings may be fused to each other.

A C₁-C₆₀ heteroaryl group used herein refers to a monovalent group having a carbocyclic aromatic system including at least one hetero atom selected from N, O, P, and S as a ring-forming atom and 1 to 60 carbon atoms as the remaining ring-forming atoms. A C₁-C₆₀ heteroarylene group used herein refers to a divalent group having a carbocyclic aromatic system including at least one hetero atom selected from N, O, P, and S as a ring-forming atom and 1 to 60 carbon atoms as the remaining ring-forming atoms. Non-limiting examples of the C₁-C₆₀ heteroaryl group may include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and/or the C₁-C₆₀ heteroarylene group include two or more rings, the respective rings may be fused to each other.

A C₆-C₆₀ aryloxy group used herein refers to a monovalent group represented by -OA₁₀₂ (where A₁₀₂ is the C₆-C₆₀ aryl group), and a C₆-C₆₀ arylthio group used herein refers to a monovalent group represented by -SA₁₀₃ (where A₁₀₃ is the C₆-C₆₀ aryl group).

A monovalent non-aromatic condensed polycyclic group used herein refers to a monovalent group that has two or more rings condensed to each other (e.g., combined together), includes only carbon atoms as ring-forming atoms (for example, having 8 to 60 carbon atoms), and does not have overall aromaticity. Non-limiting example of the monovalent non-aromatic condensed polycyclic group may be a fluorenyl group. A divalent non-aromatic condensed polycyclic group used herein refers to a divalent group having substantially the same structure as the monovalent non-aromatic condensed polycyclic group.

A monovalent non-aromatic condensed heteropolycyclic group used herein refers to a monovalent group that has two or more rings condensed to each other (e.g., combined together), has at least one hetero atom selected from N, O, P, and S as a ring forming atom, and carbon atoms as the remaining ring-forming atoms (for example, having 2 to 60 carbon atoms), and does not have overall aromaticity. Non-limiting example of the monovalent non-aromatic condensed heteropolycyclic group may be a carbazolyl group.

A divalent non-aromatic condensed heteropolycyclic group used herein refers to a divalent group having substantially the same structure as the monovalent non-aromatic condensed heteropolycyclic group.

As used herein, at least one substituent of the substituted C₃-C₁₀ cycloalkylene group, substituted C₁-C₁₀ heterocycloalkylene group, substituted C₃-C₁₀ cycloalkenylene group, substituted C₁-C₁₀ heterocycloalkenylene group, substituted C₆-C₆₀ arylene group, substituted C₁-C₆₀ heteroarylene group, substituted divalent non-aromatic condensed polycyclic group, substituted divalent non-aromatic condensed heteropolycyclic group, substituted C₁-C₆₀ alkyl group, substituted C₁-C₆₀ alkoxy group, substituted C₆-C₆₀ aryl group, substituted C₆-C₆₀ aryloxy group, substituted C₆-C₆₀ arylthio group, substituted C₁-C₆₀ heteroaryl group, substituted monovalent non-aromatic condensed polycyclic group, and substituted monovalent non-aromatic condensed heteropolycyclic group is selected from:
deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), and -Si(Q₁₃)(Q₁₄)(Q₁₅);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), and -Si(Q₂₃)(Q₂₄)(Q₂₅); and
-N(Q₃₁)(Q₃₂) and -Si(Q₃₃)(Q₃₄)(Q₃₅);
where Q₁₁ to Q₁₅, Q₂₁ to Q₂₅, and Q₃₁ to Q₃₅ are each independently selected from a C₁-C₆₀ alkyl group, a C₆-C₆₀ aryl group, a C₁-C₆₀ heteroaryl group, a monovalent non-aromatic condensed polycyclic group, and a monovalent non-aromatic condensed heteropolycyclic group; and
a C₆-C₆₀ aryl group, a C₁-C₆₀ heteroaryl group, a monovalent non-aromatic condensed polycyclic group, and a monovalent non-aromatic condensed heteropolycyclic group, each substituted with a C₆-C₆₀ aryl group.

For example, at least one substituent of the substituted C₃-C₁₀ cycloalkylene group, substituted C₁-C₁₀ heterocycloalkylene group, substituted C₃-C₁₀ cycloalkenylene group, substituted C₁-C₁₀ heterocycloalkenylene group, substituted C₆-C₆₀ arylene group, substituted C₁-C₆₀ heteroarylene group, substituted divalent non-aromatic condensed polycyclic group, substituted divalent non-aromatic condensed heteropolycyclic group, substituted C₁-C₆₀ alkyl group, substituted C₁-C₆₀ alkoxy group, substituted C₆-C₆₀ aryl group, substituted C₆-C₆₀ aryloxy group, substituted C₆-C₆₀ arylthio group, substituted C₁-C₆₀ heteroaryl group, substituted monovalent non-aromatic condensed polycyclic group, and substituted monovalent non-aromatic condensed heteropolycyclic group is selected from:
deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₃₀ alkyl group, a C₂-C₃₀ alkenyl group, a C₂-C₃₀ alkynyl group, and a C₁-C₃₀ alkoxy group;
a C₁-C₃₀ alkyl group, a C₂-C₃₀ alkenyl group, a C₂-C₃₀ alkynyl group, and a C₁-C₃₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryl group, a monovalent non-aromatic condensed polycyclic group, a monovalent non-aromatic condensed heteropolycyclic group, -N(Q₁₁)(Q₁₂), and -Si(Q₁₃)(Q₁₄)(Q₁₅);
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthenylene group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, and an imidazopyridinyl group, each substituted with at least one selected from a cyclopentyl group, a cyclohexyl group, a cycloheptyl group, a cyclooctyl group, a cyclopentenyl group, a cyclohexenyl group, a cycloheptenyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, and a quinazolinyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthenylene group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, and an imidazopyridinyl group, each substituted with at least one selected from a cyclopentyl group, a cyclohexyl group, a cycloheptyl group, a cyclooctyl group, a cyclopentenyl group, a cyclohexenyl group, a cycloheptenyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, and a quinazolinyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₃₀ alkyl group, a C₂-C₃₀ alkenyl group, a C₂-C₃₀ alkynyl group, a C₁-C₃₀ alkoxy group, a C₃-C_{1O} cycloalkyl group, C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryl group, a monovalent non-aromatic condensed polycyclic group, a monovalent non-aromatic condensed heteropolycyclic group, -N(Q₂₁)(Q₂₂), and - Si(Q₂₃)(Q₂₄)(Q₂₅); and
-N(Q₃₁)(Q₃₂) and -Si(Q₃₃)(Q₃₄)(Q₃₅);
where Q₁₁ to Q₁₅ , Q₂₁ to Q₂₅, and Q₃₁ to Q₃₅ are each independently selected from a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthenylene group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, and an imidazopyridinyl group, each substituted with at least one selected from hydrogen, a C₁-C₃₀ alkyl group, a C₂-C₃₀ alkenyl group, a C₂-C₃₀ alkynyl group, a C₁-C₃₀ alkoxy group, a cyclopentyl group, a cyclohexyl group, a cycloheptyl group, a cyclooctyl group, a cyclopentenyl group, a cyclohexenyl group, a cycloheptenyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, and a quinazolinyl group, but embodiments of the present invention are not limited thereto.

"Ph" used herein refers to a phenyl group, "Me" refers to a methyl group, "Et" refers to an ethyl group, and "ter-Bu" or "But" refers to a tert-butyl group.

Hereinafter, an organic light-emitting device according to one or more embodiments of the present invention will be described in more detail with reference to Synthesis Examples and Examples. The term "B was used instead of A" used in describing Synthesis Examples indicates that a molar equivalent of A was identical to a molar equivalent of B.

### Example 1

As an anode, a 15 Ω/cm² (1200Å) ITO glass substrate (available from Corning Co.) was cut to a size of 50 mm x 50 mm x 0.7 mm, washed with ultrasonic waves in isopropyl alcohol and pure water for 5 minutes each, and then cleaned with UV and ozone for 30 minutes. The obtained ITO glass substrate was then mounted on a vacuum depositor.

2-TNATA was vacuum deposited on the ITO anode of the ITO glass substrate to form a hole injection layer having a thickness of 500 Å, and then Compound HT1, as a hole transporting compound, was vacuum deposited on the hole injection layer to form a hole transport layer having a thickness of 1000 Å. Next, Compound 2 was deposited on the hole transport layer to form an auxiliary layer having a thickness of 300 Å.

CBP as a host and PD1 as a phosphorescent dopant were co-deposited at a weight ratio of 90:10 to form an emission layer having a thickness of 400 Å. Then, Compound 201 was deposited at a thickness of 300 Å on the emission layer to form an electron transport layer, LiF, which is a halogenized alkali metal, was deposited at a thickness of 10 Å on the electron transport layer to form an electron injection layer, and Al was vacuum deposited at a thickness of 3000 Å (as a cathode) on the electron injection layer to form a LiF/AI electrode, thereby completing the manufacture of an organic light-emitting device.

### Example 2

An organic light-emitting device was manufactured in the same (or substantially the same) manner as in Example 1, except that Compound 4 was used instead of Compound 2 in the formation of the auxiliary layer.

### Example 3

An organic light-emitting device was manufactured in the same (or substantially the same) manner as in Example 1, except that Compound 6 was used instead of Compound 2 in the formation of the auxiliary layer.

### Example 4

An organic light-emitting device was manufactured in the same (or substantially the same) manner as in Example 1, except that Compound 7 was used instead of Compound 2 in the formation of the auxiliary layer.

### Example 5

An organic light-emitting device was manufactured in the same (or substantially the same) manner as in Example 1, except that Compound 15 was used instead of Compound 2 in the formation of the auxiliary layer.

### Example 6

An organic light-emitting device was manufactured in the same (or substantially the same) manner as in Example 1, except that Compound 17 was used instead of Compound 2 in the formation of the auxiliary layer.

### Example 7

An organic light-emitting device was manufactured in the same (or substantially the same) manner as in Example 1, except that Compound 25 was used instead of Compound 2 in the formation of the auxiliary layer.

### Comparative Example 1

An organic light-emitting device was manufactured in the same (or substantially the same) manner as in Example 1, except that NPB was used instead of Compound 2 in the formation of the auxiliary layer.

### Comparative Example 2

An organic light-emitting device was manufactured in the same (or substantially the same) manner as in Example 1, except that Compound A was used instead of Compound 2 in the formation of the auxiliary layer.

### Compound A

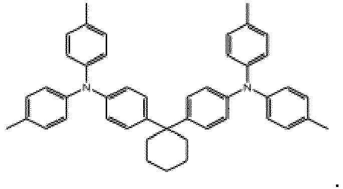

### Comparative Example 3

An organic light-emitting device was manufactured in the same (or substantially the same) manner as in Example 1, except that NPB was used instead of HT1 in the formation of the hole transport layer.

### Comparative Example 4

An organic light-emitting device was manufactured in the same (or substantially the same) manner as in Example 1, except that NPB was used instead of HT1 in the formation of the hole transport layer, and NPB was used instead of Compound 2 in the formation of the auxiliary layer.

### Evaluation Example

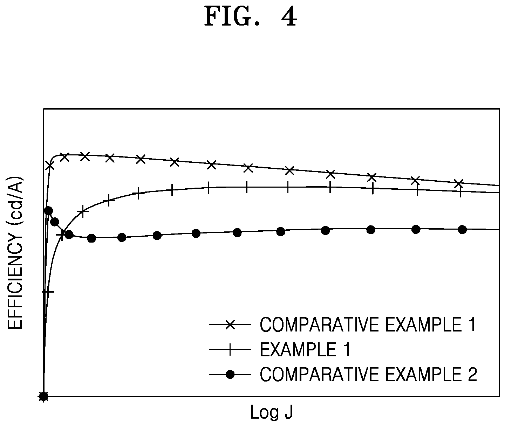

Driving voltages, efficiencies, and lifespans (at a current density of 9000 nit) of the organic light-emitting devices prepared in Examples 1 to 7 and Comparative Examples 1 to 4 were evaluated by using PR650 Spectroscan Source Measurement Unit (available from Photo Research, Inc.). The results are shown in Table 1 and FIG. 4. The efficiencies and lifespans in Table 1 are all relative values with respect to efficiencies (unit Cd/A) and lifespans (unit: hour) of the organic light-emitting device prepared in Comparative Example 1.

**Table 1**

| | Driving voltage (V) | Efficiency | Lifespan |
|---|---|---|---|
| Example 1 | 5.1 | 1.25 | 1.4 |
| Example 2 | 5.2 | 1.2 | 1.2 |
| Example 3 | 5.0 | 1.29 | 1.25 |
| Example 4 | 5.1 | 1.16 | 1.3 |
| Example 5 | 5.0 | 1.3 | 1.3 |
| Example 6 | 5.1 | 1.1 | 1.2 |
| Example 7 | 5.1 | 1.1 | 1.2 |
| Comparative Example 1 | 5.0 | 1.0 | 1 |
| Comparative Example 2 | 5.1 | 0.9 | 1.1 |
| Comparative Example 3 | 5.0 | 1.2 | 0.75 |
| Comparative Example 4 | 5.0 | 0.75 | 0.7 |

Referring to Table 1, the organic light-emitting devices of Examples 1 to 7 mostly had improved efficiencies and lifespans as compared to those of the organic light-emitting devices of Comparative Examples 1 to 4. Referring to FIG. 4, the organic light-emitting device of Example 1 may have less roll-off (e.g., have less of an efficiency roll-off) occurrence than the organic light-emitting devices of Comparative Examples 1 and 2, at a required luminance.

As described above, an organic light-emitting device according to one or more embodiments of the present invention exhibits high efficiency. In particular, the efficiency of the organic light-emitting device is improved within a relatively small dynamic range.

## Claims

1. An organic light-emitting device comprising:
a first electrode (110);
a second electrode (190);
an emission layer (160) between the first electrode (110) and the second electrode (190); and
a hole transport region (130) between the first electrode (110) and the emission layer (160), wherein the hole transport region (130) comprises a hole injection layer, a hole transport layer and an auxiliary layer (140), wherein the layers are sequentially stacked from the first electrode (110) in this stated order and wherein the emission layer (160) and the auxiliary layer (140) are adjacent to each other, and
wherein the auxiliary layer comprises (140) an amine-based compound represented by Formula 1: 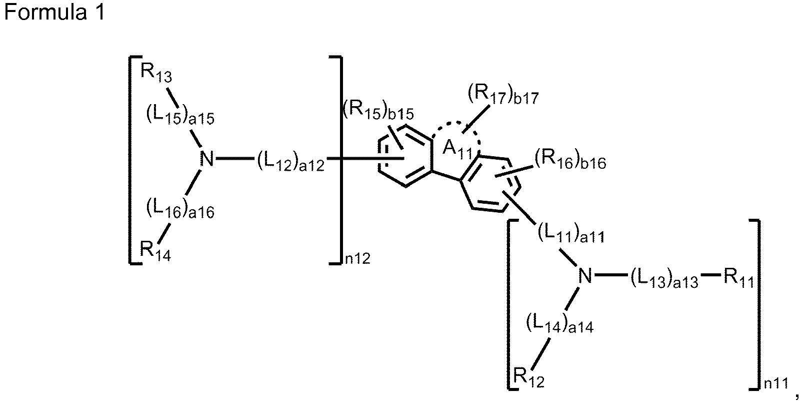
wherein, in Formula 1,
A₁₁ is selected from a C₃-C₁₀ cycloalkane, a C₁-C₁₀ heterocycloalkane, a C₃-C₁₀ cycloalkene, a C₁-C₁₀ heterocycloalkene, a C₆-C₆₀ arene, a C₁-C₆₀ heteroarene, a non-aromatic condensed polycyclic hydrocarbon group, and a non-aromatic condensed heteropolycyclic hydrocarbon group;
L₁₁ to L₁₆ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
a11 to a16 are each independently selected from 0, 1, 2, and 3;
R₁₁ to R₁₄ are each independently selected from a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group;
n11 and n12 are each independently selected from 0, 1, and 2, wherein the sum of n11 and n12 is selected from 1, 2, 3, and 4;
R₁₅ to R₁₇ are each independently selected from hydrogen, deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), and -Si(Q₃)(Q₄)(Q₅);
b15 to b17 are each independently selected from 1, 2, 3, and 4;
at least one substituent of the substituted C₃-C₁₀ cycloalkylene group, substituted C₁-C₁₀ heterocycloalkylene group, substituted C₃-C₁₀ cycloalkenylene group, substituted C₁-C₁₀ heterocycloalkenylene group, substituted C₆-C₆₀ arylene group, substituted C₁-C₆₀ heteroarylene group, substituted divalent non-aromatic condensed polycyclic group, substituted divalent non-aromatic condensed heteropolycyclic group, substituted C₁-C₆₀ alkyl group, substituted C₁-C₆₀ alkoxy group, substituted C₆-C₆₀ aryl group, substituted C₆-C₆₀ aryloxy group, substituted C₆-C₆₀ arylthio group, substituted C₁-C₆₀ heteroaryl group, substituted monovalent non-aromatic condensed polycyclic group, and substituted monovalent non-aromatic condensed heteropolycyclic group is selected from:
deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), and -Si(Q₁₃)(Q₁₄)(Q₁₅);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), and -Si(Q₂₃)(Q₂₄)(Q₂₅); and
-N(Q₃₁)(Q₃₂) and -Si(Q₃₃)(Q₃₄)(Q₃₅);
wherein Q₁ to Q₅, Q₁₁ to Q₁₅, Q₂₁ to Q₂₅, and Q₃₁ to Q₃₅ are each independently selected from:
a C₁-C₆₀ alkyl group, a C₆-C₆₀ aryl group, a C₁-C₆₀ heteroaryl group, a monovalent non-aromatic condensed polycyclic group, and a monovalent non-aromatic condensed heteropolycyclic group; and
a C₆-C₆₀ aryl group, a C₁-C₆₀ heteroaryl group, a monovalent non-aromatic condensed polycyclic group, and a monovalent non-aromatic condensed heteropolycyclic group, each substituted with a C₆-C₆₀ aryl group; and
wherein the hole transport layer consists of a first hole transporting compound represented by any one of Formulae 201A and 202A: 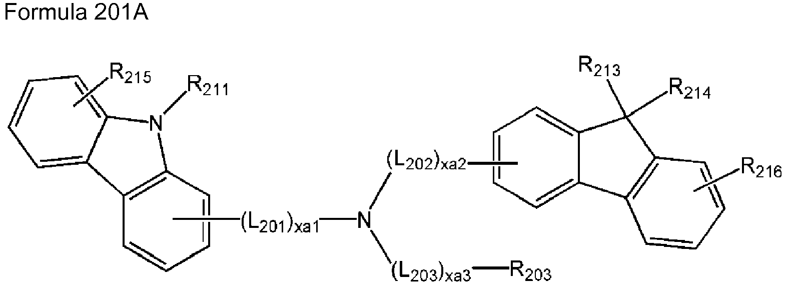 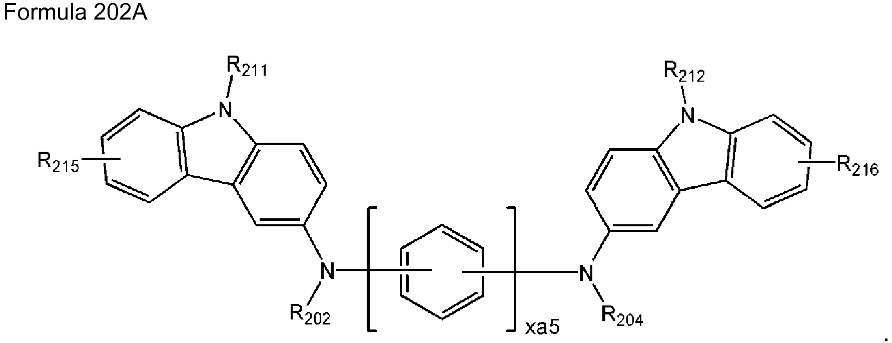 wherein
L₂₀₁ to L₂₀₃ are the same as defined in connection with L₁₁; xa1 to xa3 are independently selected from 0, 1, 2, and 3; xa5 is selected from 1, 2, 3, 4, and 5; R₂₀₂ to R₂₀₄ are the same as the definition provided in connection with R₁₁; R₂₁₁ and R₂₁₂ are each independently defined the same as the definition provided in connection with R₂₀₃ above, and
R₂₁₃ to R₂₁₆ are each independently selected from hydrogen, deuterium, -F, -CI, - Br, -I, a hydroxyl group, a cyano group, a nitro group, an amino group, an amidino group, a hydrazine group, a hydrazone group, a carboxyl group or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a C₃-C_{1O} cycloalkyl group, a C₁-C₁₀ heterocycloalkyl group, a C₃-C_{1O} cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

2. The organic light-emitting device of claim 1, wherein A₁₁ is represented by any one of Formulae 9-1 to 9-5: 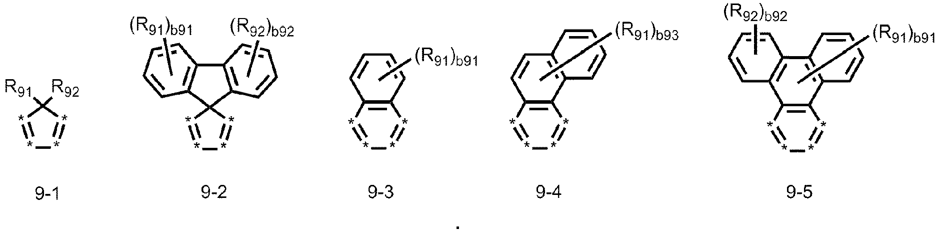
wherein, in Formulae 9-1 to 9-5,
* is a carbon atom in Formula 1;
R₉₁ and R₉₂ are each independently selected from hydrogen, deuterium, -F, -CI, - Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, and -N(O₁)(O₂),
wherein O₁ and Q₂ are each independently selected from:
a C₆-C₆₀ aryl group and a monovalent non-aromatic condensed polycyclic group; and
a C₆-C₆₀ aryl group and a monovalent non-aromatic condensed polycyclic group, each substituted with a C₆-C₆₀ aryl group;
b91 and b92 are each independently selected from 1, 2, 3, and 4; and
b93 is selected from 1, 2, 3, 4, 5, and 6.

3. The organic light-emitting device of claim 1, wherein L₁₁ to L₁₆ are each independently selected from:
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substituted with at least one selected from deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl group.

4. The organic light-emitting device of claim 1, wherein R₁₁ to R₁₄ are each independently selected from:
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, an imidazopyridinyl group, and an imidazopyrimidinyl group; and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, an imidazopyridinyl group, and an imidazopyrimidinyl group, each substituted with at least one selected from deuterium, -F, - CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a biphenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a benzoquinoxalinyl group, a quinazolinyl group, a benzoquinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, an imidazopyridinyl group, and an imidazopyrimidinyl group.

5. The organic light-emitting device of claim 1, wherein R₁₅ to R₁₇ are each independently selected from hydrogen, deuterium, -F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, and - N(Q₁)(Q₂);
wherein Q₁ and Q₂ are each independently selected from a C₆-C₆₀ aryl group and a monovalent non-aromatic condensed polycyclic group; and
a C₆-C₆₀ aryl group and a monovalent non-aromatic condensed polycyclic group, each substituted with a C₆-C₆₀ aryl group.

6. The organic light-emitting device of claim 1, wherein the amine-based compound is represented by any one of Formulae 1-1 to 1-5: 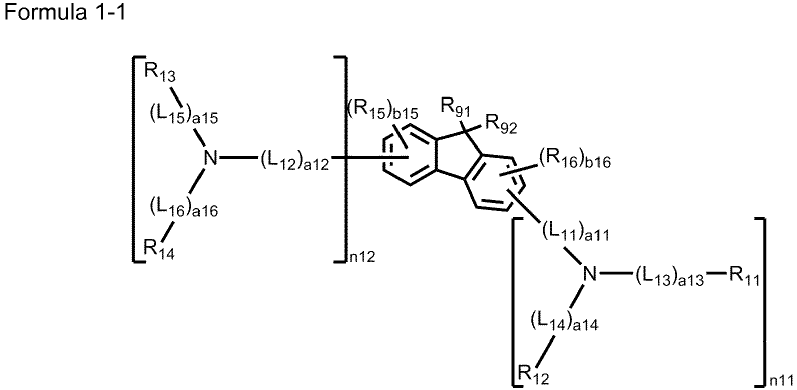 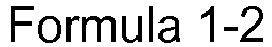 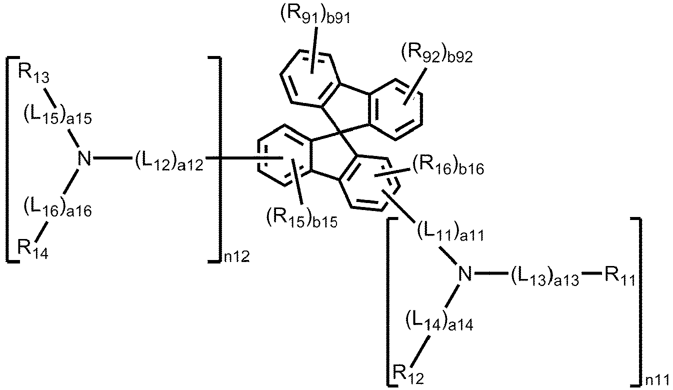 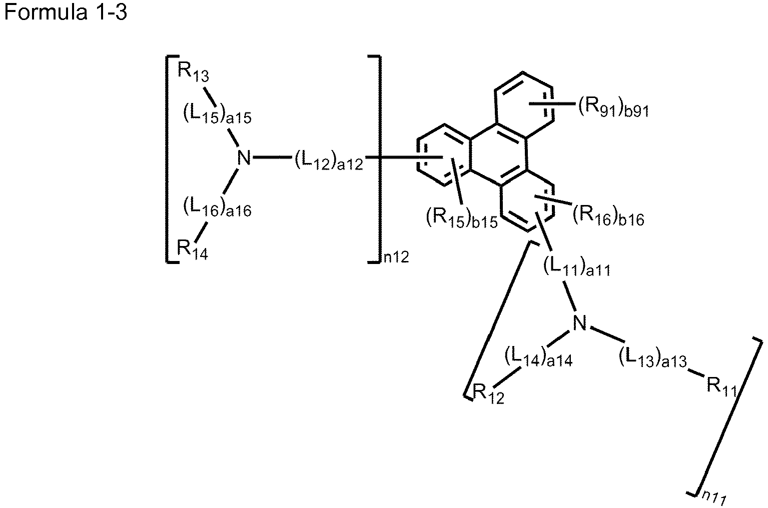 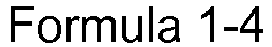 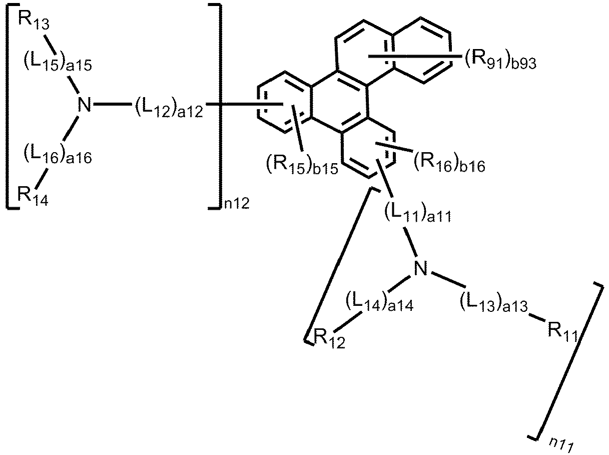 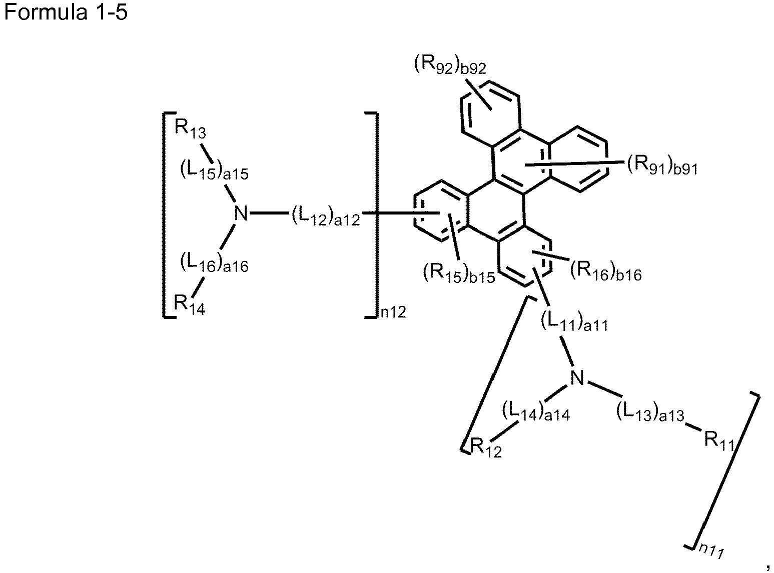
wherein, in Formulae 1-1 to 1-5,
L₁₁ to L₁₆ are each independently selected from a substituted or unsubstituted C₃-C₁₀ cycloalkylene group, a substituted or unsubstituted C₁-C₁₀ heterocycloalkylene group, a substituted or unsubstituted C₃-C_{1O} cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
a11 to a16 are each independently selected from 0, 1, 2, and 3;
R₁₁ to R₁₄ are each independently selected from a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group;
n11 and n12 are each independently selected from 0, 1, and 2, wherein the sum of n11 and n12 is selected from 1, 2, 3, and 4;
R₁₅, R₁₆, R₉₁ and R₉₂ are each independently selected from hydrogen, deuterium, - F, -CI, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted C₁-C₆₀ alkyl group, a substituted or unsubstituted C₁-C₆₀ alkoxy group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, and -N(Q₁)(Q₂);
wherein Q₁ and Q₂ are each independently selected from:
a C₆-C₆₀ aryl group and a monovalent non-aromatic condensed polycyclic group; and
a C₆-C₆₀ aryl group and a monovalent non-aromatic condensed polycyclic group, each substituted with a C₆-C₆₀ aryl group;
b15 to b16 are each independently selected from 1, 2, 3, and 4;
b91 and b92 are each independently selected from 1, 2, 3, and 4; and
b93 is selected from 1, 2, 3, 4, 5, and 6.

7. The organic light-emitting device of claim 1, wherein the amine-based compound is selected from Compounds 1 to 48:

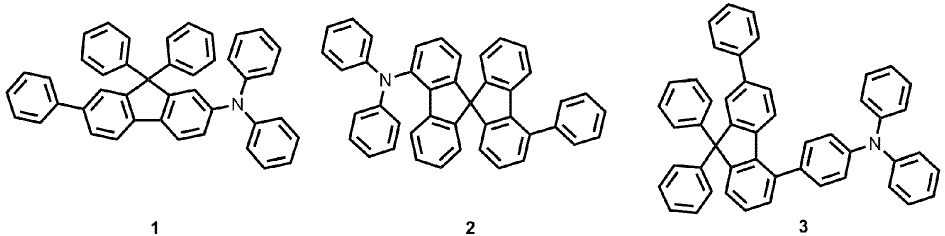

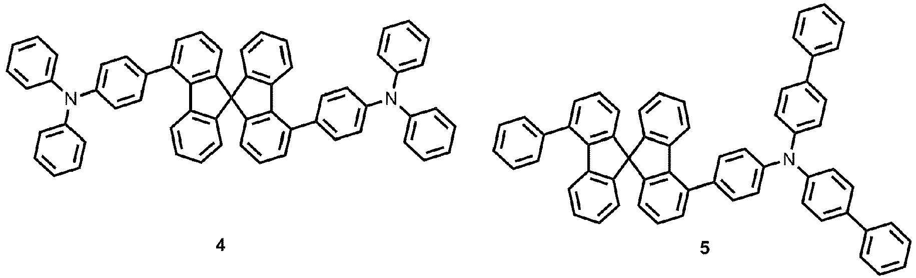

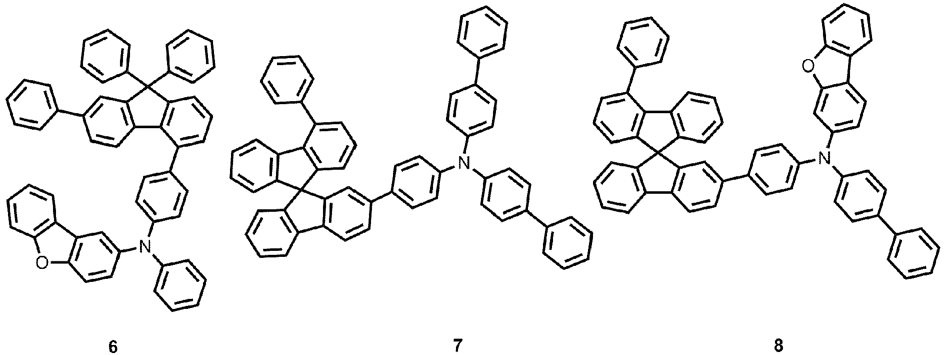

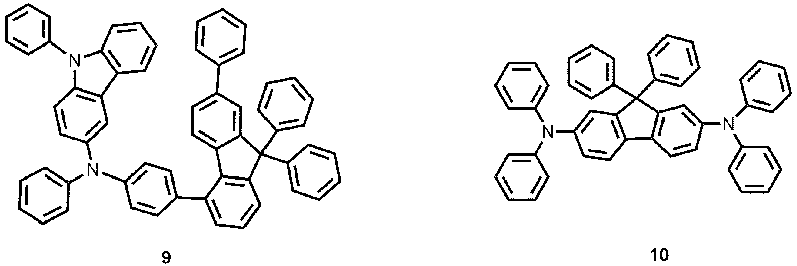

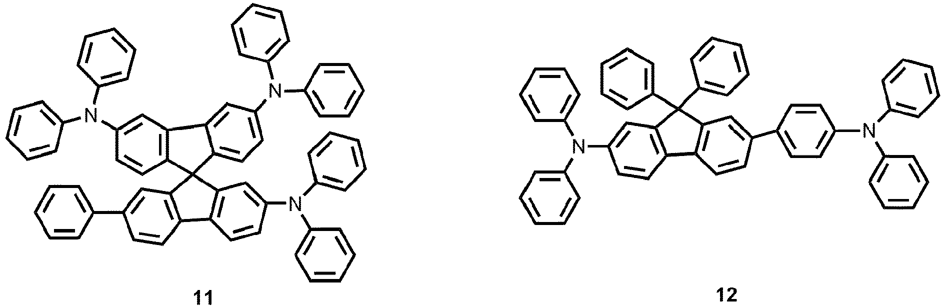

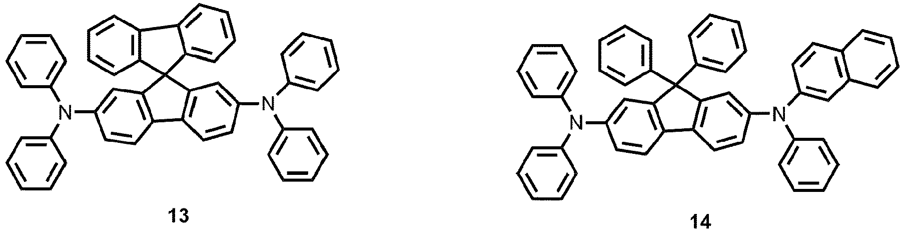

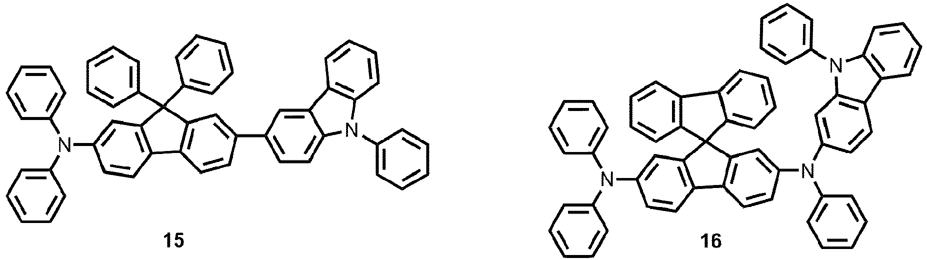

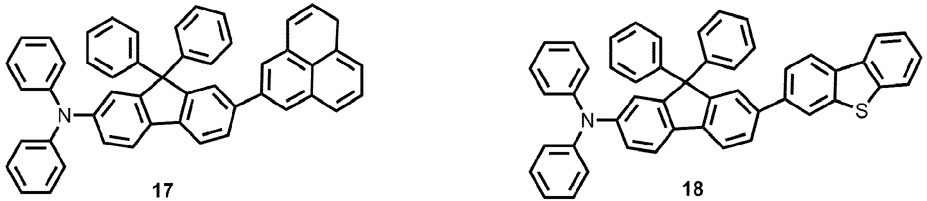

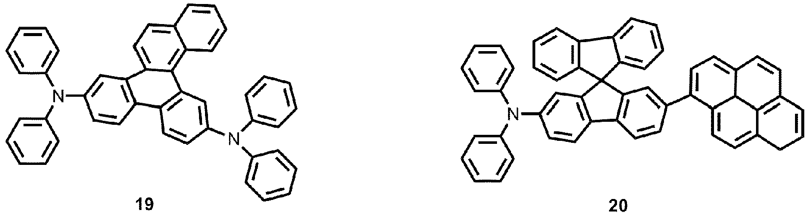

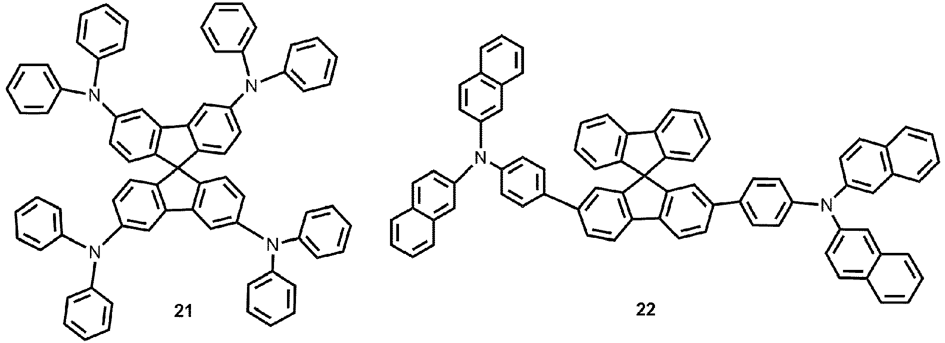

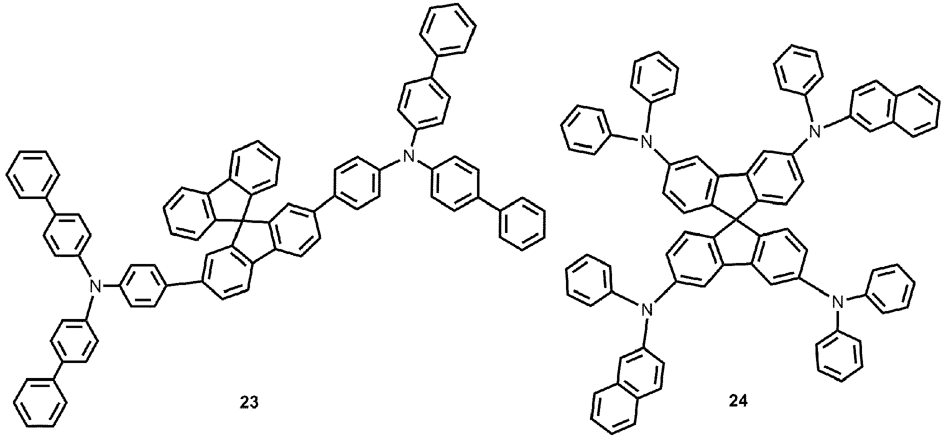

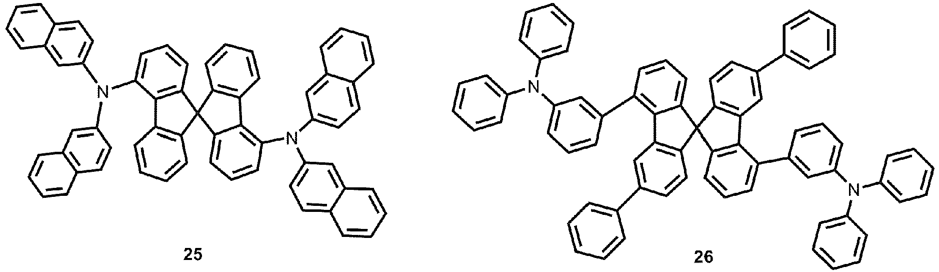

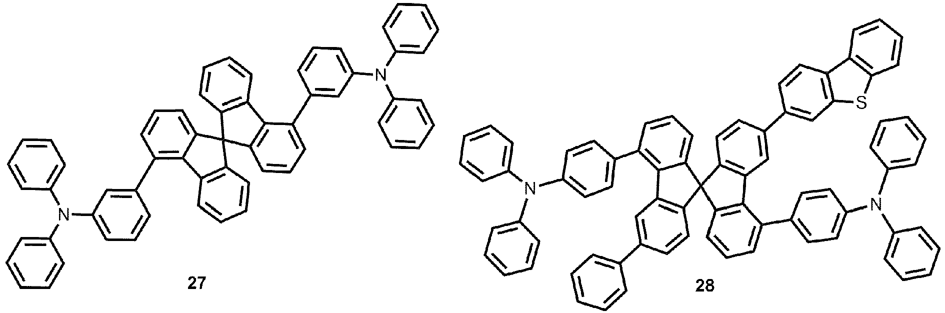

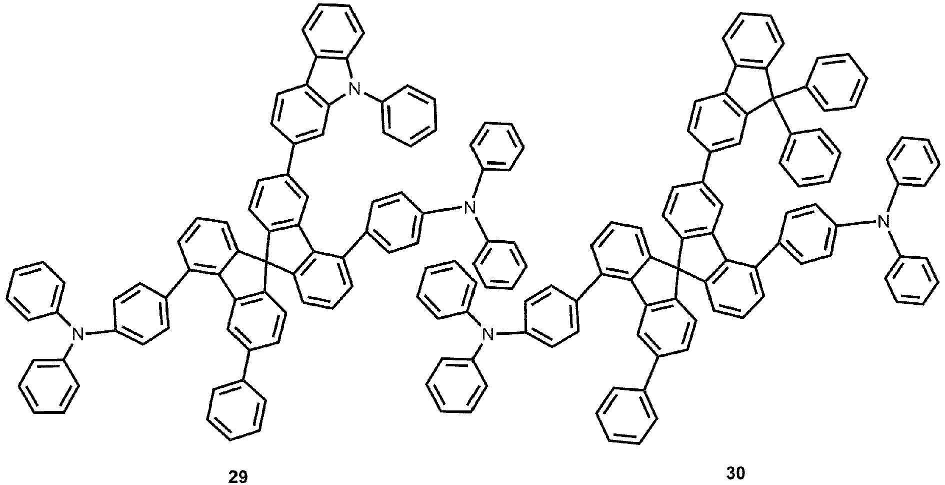

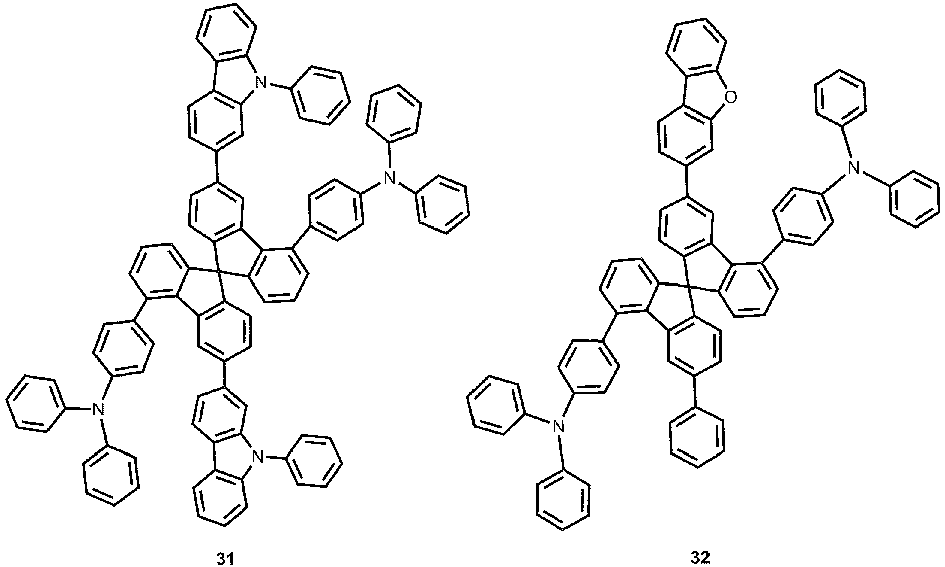

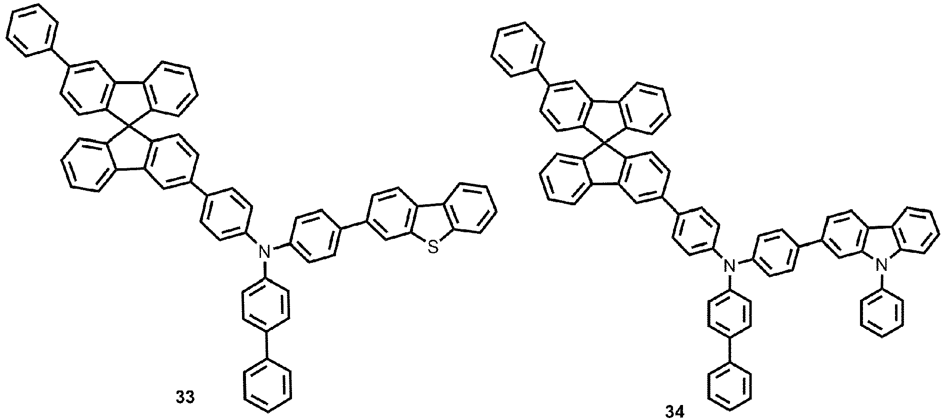

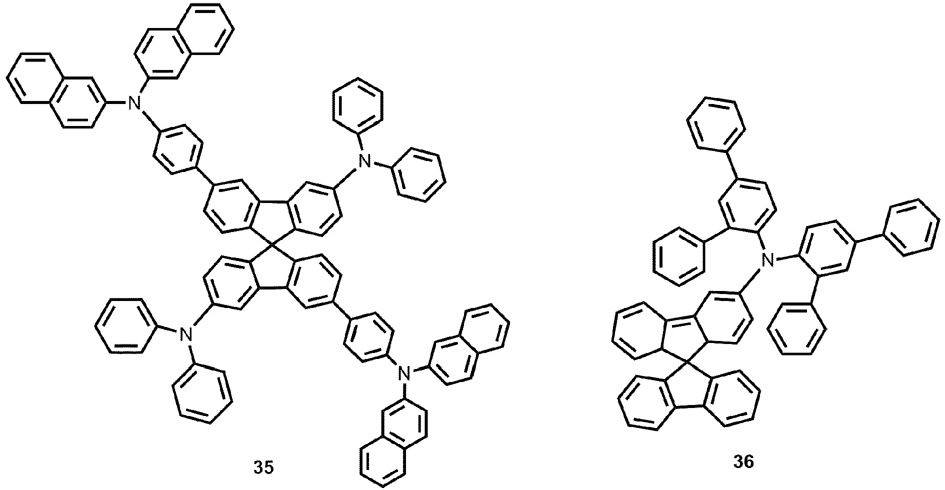

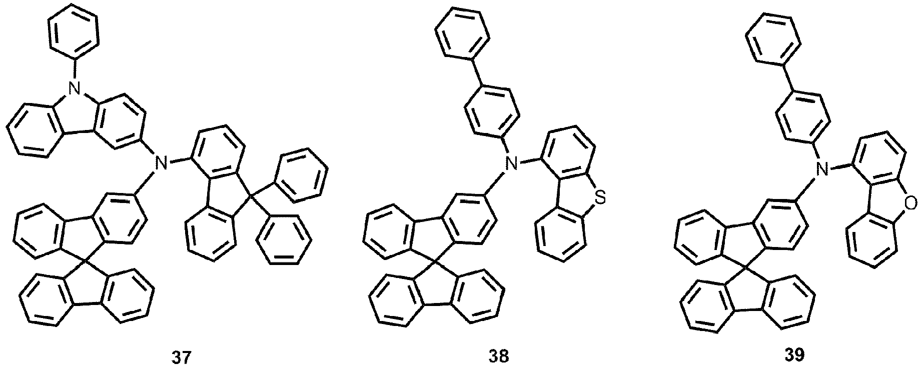

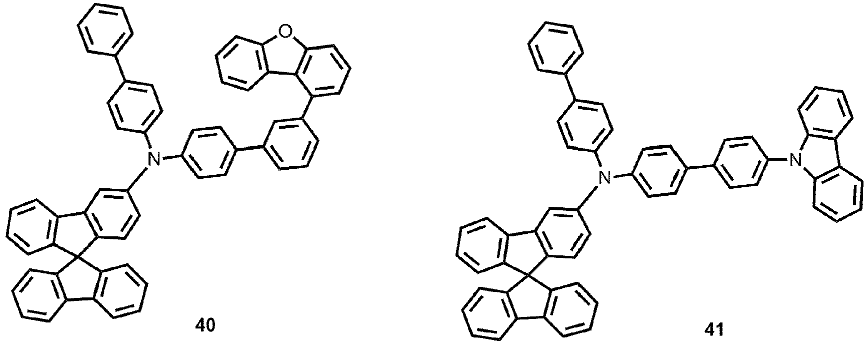

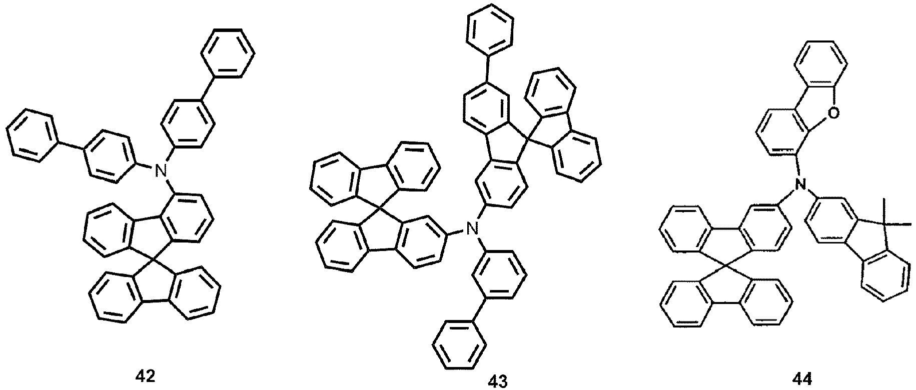

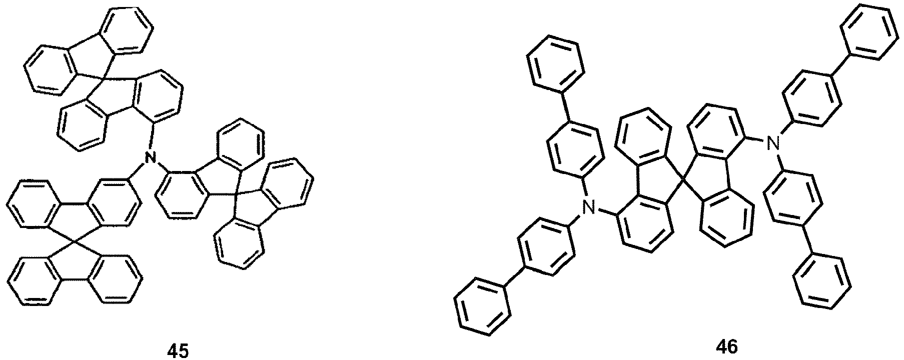

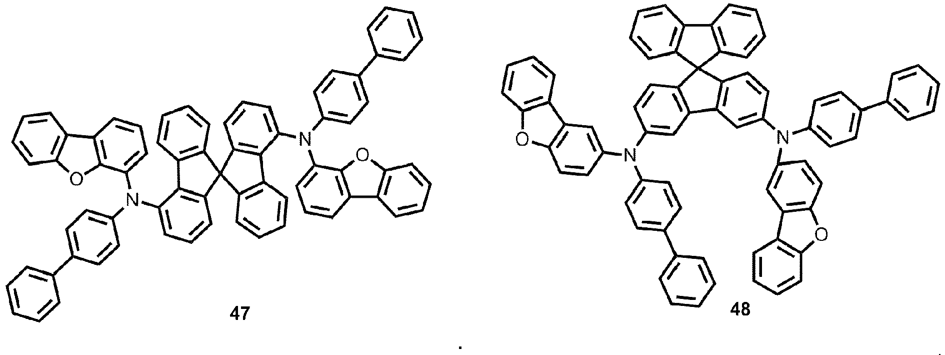

8. The organic light-emitting device of claim 1, wherein first hole transporting compound is represented by one of Compounds HT1 to HT20:

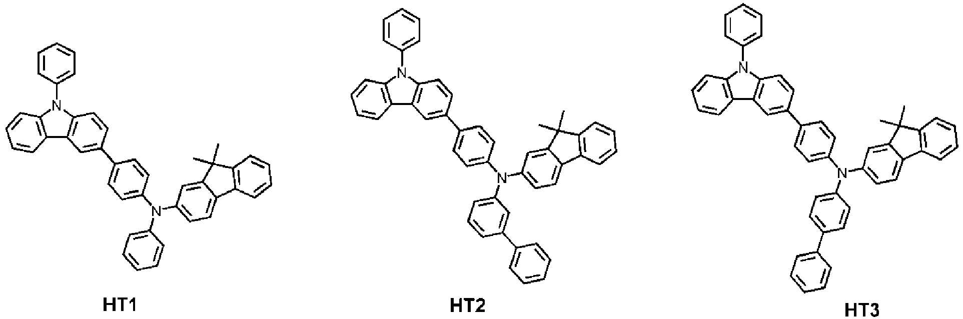

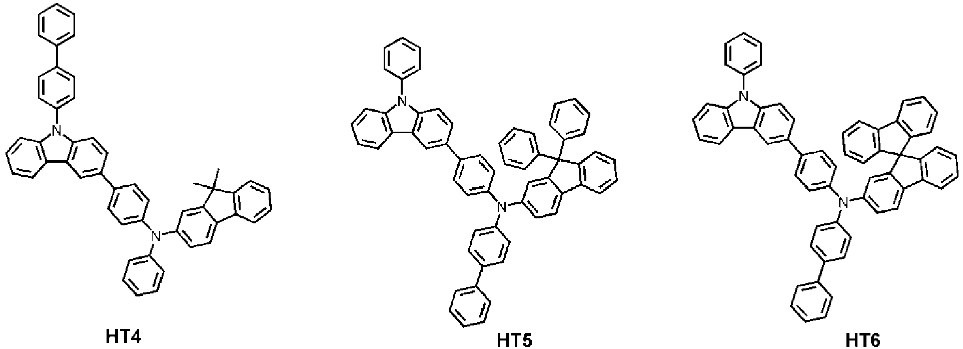

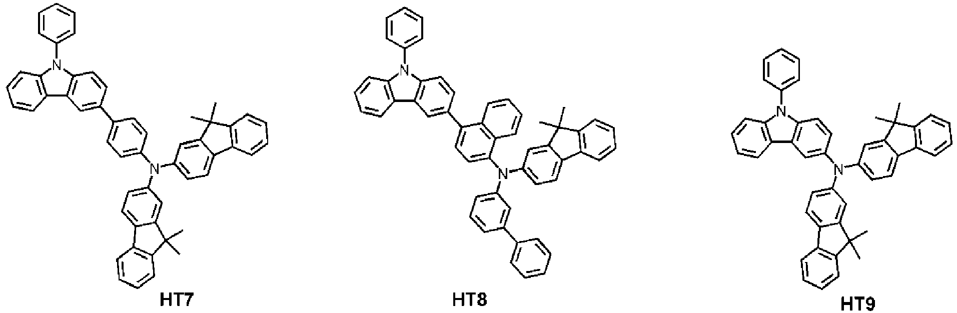

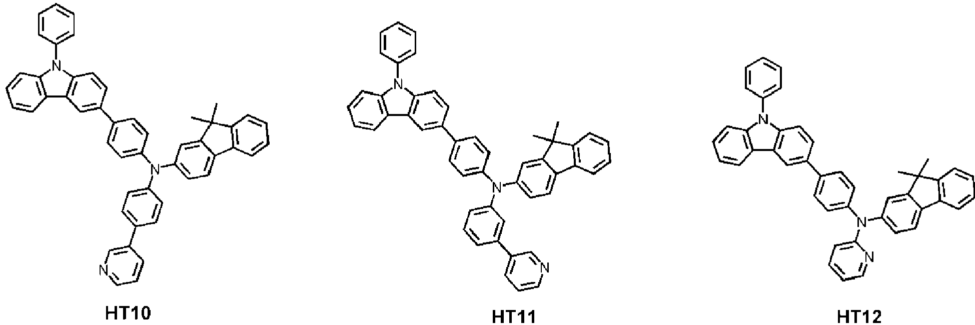

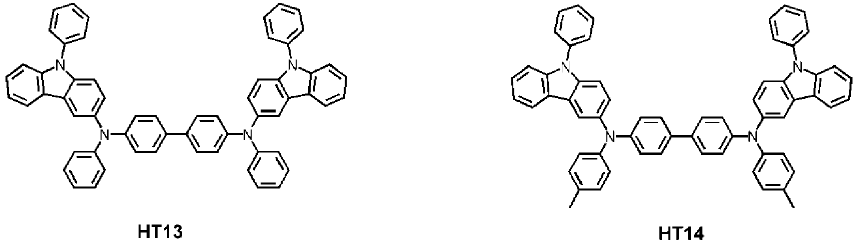

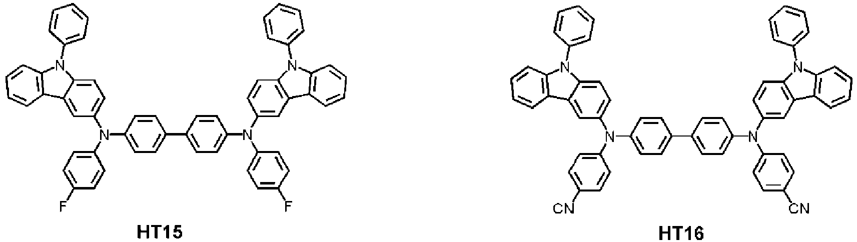

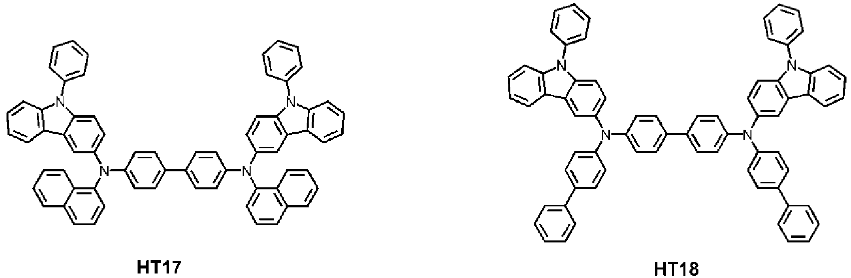

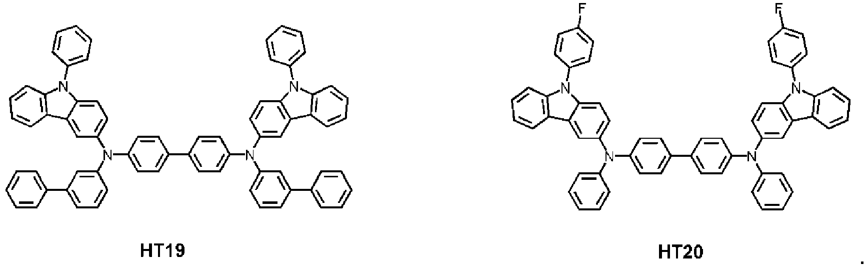

## Patentansprüche

1. Eine organische lichtemittierende Vorrichtung, aufweisend:
eine erste Elektrode (110);
eine zweite Elektrode (190);
eine Emissionsschicht (160) zwischen der ersten Elektrode (110) und der zweiten Elektrode (190); und
einen Lochtransportbereich (130) zwischen der ersten Elektrode (110) und der Emissionsschicht (160), wobei der Lochtransportbereich (130) eine Lochinjektionsschicht, eine Lochtransportschicht und eine Hilfsschicht (140) aufweist, wobei die Schichten von der ersten Elektrode (110) an in dieser genannten Reihenfolge nacheinander gestapelt sind und wobei die Emissionsschicht (160) und die Hilfsschicht (140) zueinander benachbart sind, und
wobei die Hilfsschicht (140) eine Verbindung auf Aminbasis, die durch Formel 1 dargestellt ist, aufweist: 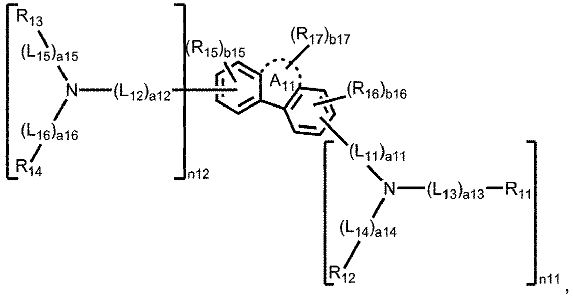
wobei in Formel 1
A₁₁ aus einem C₃-C₁₀-Cycloalkan, einem C₁-C₁₀-Heterocycloalkan, einem C₃-C₁₀-Cycloalken, einem C₁-C₁₀-Heterocycloalken, einem C₆-C₆₀-Aren, einem C₁-C₆₀-Heteroaren, einer nicht-aromatischen kondensierten polycyclischen Kohlenwasserstoffgruppe und einer nicht-aromatischen kondensierten heteropolycyclischen Kohlenwasserstoffgruppe ausgewählt ist;
L₁₁ bis L₁₆ jeweils unabhängig voneinander aus einer substituierten oder unsubstituierten C₃-C₁₀-Cycloalkylengruppe, einer substituierten oder unsubstituierten C₁-C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten C₆-C₆₀-Arylengruppe, einer substituierten oder unsubstituierten C₁-C₆₀-Heteroarylengruppe, einer substituierten oder unsubstituierten divalenten nicht-aromatischen kondensierten polycyclischen Gruppe und einer substituierten oder unsubstituierten divalenten nicht-aromatischen kondensierten heteropolycyclischen Gruppe ausgewählt sind,
a11 bis a16 jeweils unabhängig voneinander aus 0, 1, 2 und 3 ausgewählt sind;
R₁₁ bis R₁₄ jeweils unabhängig voneinander aus einer substituierten oder unsubstituierten C₆-C₆₀-Arylgruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Gruppe ausgewählt sind;
n11 und n12 jeweils unabhängig voneinander aus 0, 1 und 2 ausgewählt sind, wobei die Summe von n11 und n12 aus 1, 2, 3 und 4 ausgewählt ist;
R₁₅ bis R₁₇ jeweils unabhängig voneinander aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer substituierten oder unsubstituierten C₁-C₆₀-Alkylgruppe, einer substituierten oder unsubstituierten C₁-C₆₀-Alkoxygruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe, einer substituierten oder unsubstituierten monovalenten nicht-aromatischen kondensierten heteropolycyclischen Gruppe, -N(Q₁)(Q₂) und -Si(Q₃)(Q₄)(Q₅) ausgewählt sind;
b15 bis b17 jeweils unabhängig voneinander aus 1, 2, 3 und 4 ausgewählt sind;
zumindest ein Substituent der substituierten C₃-C₁₀-Cycloalkylengruppe, der substituierten C₁-C₁₀-Heterocycloalkylengruppe, der substituierten C₃-C₁₀-Cycloalkenylengruppe, der substituierten C₁-C₁₀-Heterocycloalkenylengruppe, der substituierten C₆-C₆₀-Arylengruppe, der substituierten C₁-C₆₀-Heteroarylengruppe, der substituierten divalenten nicht-aromatischen kondensierten polycyclischen Gruppe, der substituierten divalenten nicht-aromatischen kondensierten heteropolycyclischen Gruppe, der substituierten C₁-C₆₀-Alkylgruppe, der substituierten C₁-C₆₀-Alkoxygruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten monovalenten nicht-aromatischen kondensierten polycyclischen Gruppe und der substituierten monovalenten nicht-aromatischen kondensierten heteropolycyclischen Gruppe ausgewählt ist aus:
Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer C₁-C₆₀-Alkylgruppe, einer C₂-C₆₀-Alkenylgruppe, einer C₂-C₆₀-Alkynylgruppe und einer C₁-C₆₀-Alkoxygruppe;
einer C₁-C₆₀-Alkylgruppe, einer C₂-C₆₀-Alkenylgruppe, einer C₂-C₆₀-Alkynylgruppe und einer C₁-C₆₀-Alkoxygruppe, jeweils substituiert mit zumindest einem ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe, einer monovalenten nicht-aromatischen kondensierten heteropolycyclischen Gruppe, -N(Q₁₁)(Q₁₂) und -Si(Q₁₃)(Q₁₄)(Q₁₅);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe;
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe, jeweils substituiert mit zumindest einem ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe, einer monovalenten nicht-aromatischen kondensierten heteropolycyclischen Gruppe, -N(Q₂₁)(Q₂₂) und -Si(Q₂₃)(Q₂₄)(Q₂₅); und
-N(Q₃₁)(Q₃₂) und -Si(Q₃₃)(Q₃₄)(Q₃₅);
wobei Q₁ bis Q₅, Q₁₁ bis Q₁₅, Q₂₁ bis Q₂₅ und Q₃₁ bis Q₃₅ jeweils unabhängig voneinander ausgewählt sind aus:
einer C₁-C₆₀-Alkylgruppe, einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe; und
einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe, jeweils substituiert mit einer C₆₋C₆₀-Arylgruppe; und
wobei die Lochtransportschicht aus einer ersten lochtransportierenden Verbindung besteht, die durch eine der Formeln 201A und 202A dargestellt ist: 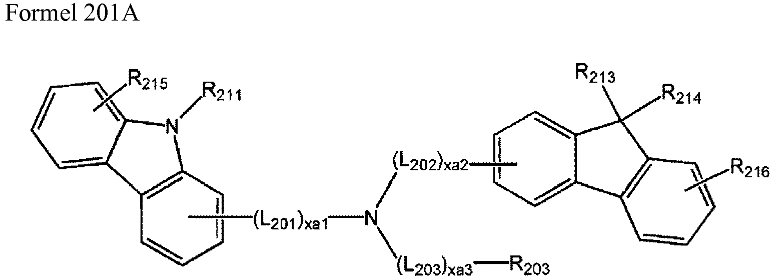 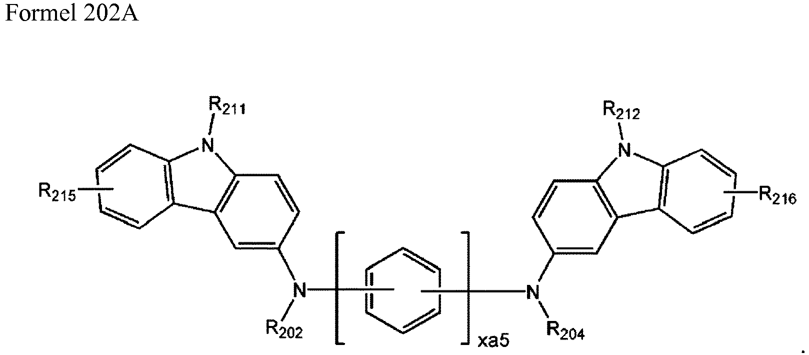
wobei L₂₀₁ bis L₂₀₃ wie in Verbindung mit L₁₁ definiert sind; xa1 bis xa3 jeweils unabhängig voneinander aus 0, 1, 2 und 3 ausgewählt sind; xa5 aus 1, 2, 3, 4 und 5 ausgewählt ist; R₂₀₂ bis R₂₀₄ gleich der in Verbindung mit R₁₁ festgelegten Definition sind; R₂₁₁ und R₂₁₂ jeweils unabhängig voneinander gleich der oben in Verbindung mit R₂₀₃ festgelegten Definition definiert sind, und
R₂₁₃ bis R₂₁₆ jeweils unabhängig voneinander aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carboxylgruppe oder einem Salz davon, einer Sulfonsäure oder einem Salz davon, einer Phosphorsäure oder einem Salz davon, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe ausgewählt sind.

2. Die organische lichtemittierende Vorrichtung nach Anspruch 1, wobei A₁₁ durch eine der Formeln 9-1 bis 9-5 dargestellt ist: 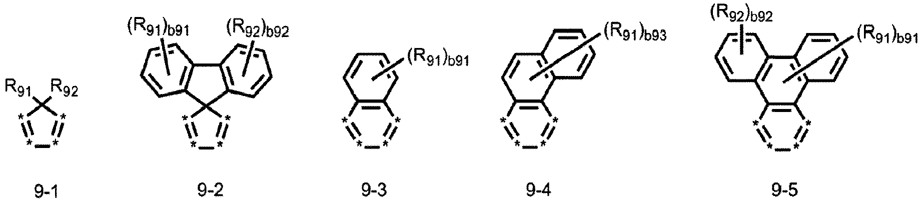
wobei in den Formeln 9-1 bis 9-5
* ein Kohlenstoffatom in Formel 1 ist;
R₉₁ und R₉₂ jeweils unabhängig voneinander aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer substituierten oder unsubstituierten C₁-C₆₀-Alkylgruppe, einer substituierten oder unsubstituierten C₁-C₆₀-Alkoxygruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe, einer substituierten oder unsubstituierten monovalenten nicht-aromatischen kondensierten heteropolycyclischen Gruppe und -N(Q₁)(Q₂) ausgewählt sind,
wobei Q₁ und Q₂ jeweils unabhängig voneinander ausgewählt sind aus:
einer C₆-C₆₀-Arylgruppe und einer monovalenten nicht-aromatischen kondensierten polycyclischen Gruppe; und
einer C₆-C₆₀-Arylgruppe und einer monovalenten nicht-aromatischen kondensierten polycyclischen Gruppe, jeweils substituiert mit einer C₆-C₆₀-Arylgruppe;
b91 und b92 jeweils unabhängig voneinander aus 1, 2, 3 und 4 ausgewählt sind; und
b93 aus 1, 2, 3, 4, 5 und 6 ausgewählt ist.

3. Die organische lichtemittierende Vorrichtung nach Anspruch 1, wobei L₁₁ bis L₁₆ jeweils unabhängig voneinander ausgewählt sind aus:
einer Phenylengruppe, einer Pentalenylengruppe, einer Indenylengruppe, einer Naphthylengruppe, einer Azulenylengruppe, einer Heptalenylengruppe, einer Indacenylengruppe, einer Acenaphthylengruppe, einer Fluorenylengruppe, einer Spiro-Fluorenylengruppe, einer Benzofluorenylengruppe, einer Dibenzofluorenylengruppe, einer Phenalenylengruppe, einer Phenanthrenylengruppe, einer Anthracenylengruppe, einer Fluoranthenylengruppe, einer Triphenylenylengruppe, einer Pyrenylengruppe, einer Chrysenylengruppe, einer Naphthacenylengruppe, einer Picenylengruppe, einer Perylenylengruppe, einer Pentaphenylengruppe, einer Hexacenylengruppe, einer Pentacenylengruppe, einer Rubicenylengruppe, einer Coronenylengruppe, einer Ovalenylengruppe, einer Pyrrolylengruppe, einer Thiophenylengruppe, einer Furanylengruppe, einer Imidazolylengruppe, einer Pyrazolylengruppe, einer Thiazolylengruppe, einer Isothiazolylengruppe, einer Oxazolylengruppe, einer Isoxazolylengruppe, einer Pyridinylengruppe, einer Pyrazinylengruppe, einer Pyrimidinylengruppe, einer Pyridazinylengruppe, einer Isoindolylengruppe, einer Indolylengruppe, einer Indazolylengruppe, einer Purinylengruppe, einer Chinolinylengruppe, einer Isochinolinylengruppe, einer Benzochinolinylengruppe, einer Phthalazinylengruppe, einer Napththyridinylengruppe, einer Chinoxalinylengruppe, einer Chinazolinylengruppe, einer Cinnolinylengruppe, einer Carbazolylengruppe, einer Phenanthridinylengruppe, einer Acridinylengruppe, einer Phenanthrolinylengruppe, einer Phenazinylengruppe, einer Benzimidazolylengruppe, einer Benzofuranylengruppe, einer Benzothiophenylengruppe, einer Isobenzothiazolylengruppe, einer Benzoxazolylengruppe, einer Isobenzoxazolylengruppe, einer Triazolylengruppe, einer Tetrazolylengruppe, einer Oxadiazolylengruppe, einer Triazinylengruppe, einer Dibenzofuranylengruppe, einer Dibenzothiophenylengruppe, einer Benzocarbazolylengruppe und einer Dibenzocarbazolylengruppe; und
einer Phenylengruppe, einer Pentalenylengruppe, einer Indenylengruppe, einer Naphthylengruppe, einer Azulenylengruppe, einer Heptalenylengruppe, einer Indacenylengruppe, einer Acenaphthylengruppe, einer Fluorenylengruppe, einer Spiro-Fluorenylengruppe, einer Benzofluorenylengruppe, einer Dibenzofluorenylengruppe, einer Phenalenylengruppe, einer Phenanthrenylengruppe, einer Anthracenylengruppe, einer Fluoranthenylengruppe, einer Triphenylenylengruppe, einer Pyrenylengruppe, einer Chrysenylengruppe, einer Naphthacenylengruppe, einer Picenylengruppe, einer Perylenylengruppe, einer Pentaphenylengruppe, einer Hexacenylengruppe, einer Pentacenylengruppe, einer Rubicenylengruppe, einer Coronenylengruppe, einer Ovalenylengruppe, einer Pyrrolylengruppe, einer Thiophenylengruppe, einer Furanylengruppe, einer Imidazolylengruppe, einer Pyrazolylengruppe, einer Thiazolylengruppe, einer Isothiazolylengruppe, einer Oxazolylengruppe, einer Isoxazolylengruppe, einer Pyridinylengruppe, einer Pyrazinylengruppe, einer Pyrimidinylengruppe, einer Pyridazinylengruppe, einer Isoindolylengruppe, einer Indolylengruppe, einer Indazolylengruppe, einer Purinylengruppe, einer Chinolinylengruppe, einer Isochinolinylengruppe, einer Benzochinolinylengruppe, einer Phthalazinylengruppe, einer Naphthyridinylengruppe, einer Chinoxalinylengruppe, einer Chinazolinylengruppe, einer Cinnolinylengruppe, einer Carbazolylengruppe, einer Phenanthridinylengruppe, einer Acridinylengruppe, einer Phenanthrolinylengruppe, einer Phenazinylengruppe, einer Benzimidazolylengruppe, einer Benzofuranylengruppe, einer Benztothiophenylengruppe, einer Isobenzothiazolylengruppe, einer Benzoxazolylengruppe, einer Isobenzoxazolylengruppe, einer Triazolylengruppe, einer Tetrazolylengruppe, einer Oxadiazolylengruppe, einer Triazinylengruppe, einer Dibenzofuranylengruppe, einer Dibenzothiophenylengruppe, einer Benzocarbazolylengruppe und einer Dibenzocarbazolylengruppe, jeweils substituiert mit zumindest einem ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer C₁-C₂₀-Alkylgruppe, einer C₁-C₂₀-Alkoxygruppe, einer Cyclopentylgruppe, einer Cyclohexylgruppe, einer Cycloheptylgruppe, einer Cyclopentenylgruppe, einer Cyclohexenylgruppe, einer Phenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe einer Pentacenylgruppe, einer Rubicenylgruppe, einer Coronenylgruppe, einer Ovalenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Phthalazinylgruppe, einer Naphthyridinylgruppe, einer Chinoxalinylgruppe, einer Chinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Thiadiazolylgruppe und einer Imidazopyridinylgruppe.

4. Die organische lichtemittierende Vorrichtung nach Anspruch 1, wobei R₁₁ bis R₁₄ jeweils unabhängig voneinander ausgewählt sind aus:
einer Phenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe einer Pentacenylgruppe, einer Rubicenylgruppe, einer Coronenylgruppe, einer Ovalenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Carbazolylgruppe, einer Benzochinolinylgruppe, einer Phthalazinylgruppe, einer Naphthyridinylgruppe, einer Chinoxalinylgruppe, einer Benzochinoxalinylgruppe, einer Chinazolinylgruppe, einer Benzochinazolinylgruppe, einer Cinnolinylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Thiadiazolylgruppe, einer Imidazopyridinylgruppe und einer Imidazopyrimidinylgruppe; und
einer Phenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe einer Pentacenylgruppe, einer Rubicenylgruppe, einer Coronenylgruppe, einer Ovalenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Carbazolylgruppe, einer Benzochinolinylgruppe, einer Phthalazinylgruppe, einer Naphthyridinylgruppe, einer Chinoxalinylgruppe, einer Benzochinoxalinylgruppe, einer Chinazolinylgruppe, einer Benzochinazolinylgruppe, einer Cinnolinylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einr Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Thiadiazolylgruppe, einer Imidazopyridinylgruppe und einer Imidazopyrimidinylgruppe, jeweils substituiert mit zumindest einem ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer C₁-C₂₀-Alkylgruppe, einer C₁-C₂₀-Alkoxygruppe, einer Phenylgruppe, einer Biphenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe einer Pentacenylgruppe, einer Rubicenylgruppe, einer Coronenylgruppe, einer Ovalenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Carbazolylgruppe, einer Benzochinolinylgruppe, einer Phthalazinylgruppe, einer Naphthyridinylgruppe, einer Chinoxalinylgruppe, einer Benzochinoxalinylgruppe, einer Chinazolinylgruppe, einer Benzochinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarabzolylgruppe, einer Thiadiazolylgruppe, einer Imidazopyridinylgruppe und einer Imidazopyrimidinylgruppe.

5. Die organische lichtemittierende Vorrichtung nach Anspruch 1, wobei R₁₅ bis R₁₇ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer substituierten oder unsubstituierten C₁-C₆₀-Alkylgruppe, einer substituierten oder unsubstituierten C₁-C₆₀-Alkoxygruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe, einer substituierten oder unsubstituierten monovalenten nicht-aromatischen kondensierten heteropolycyclischen Gruppe und -N(Q₁)(Q₂);
wobei Q₁ und Q₂ jeweils unabhängig voneinander aus einer C₆-C₆₀-Arylgruppe und einer monovalenten nicht-aromatischen kondensierten polycyclischen Gruppe ausgewählt sind; und
einer C₆-C₆₀-Arylgruppe und einer monovalenten nicht-aromatischen kondensierten polycyclischen Gruppe, jeweils substituiert mit einer C₆-C₆₀-Arylgruppe.

6. Die organische lichtemittierende Vorrichtung nach Anspruch 1, wobei die Verbindung auf Aminbasis durch eine der Formeln 1-1 bis 1-5 dargestellt ist: 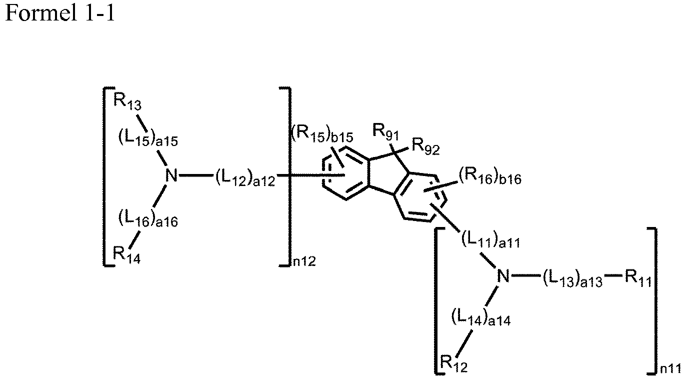 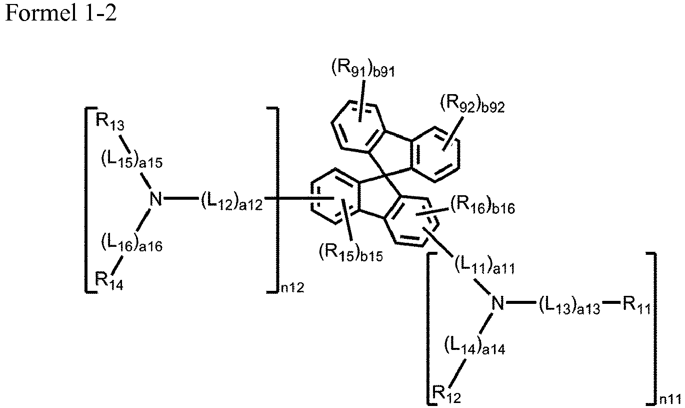 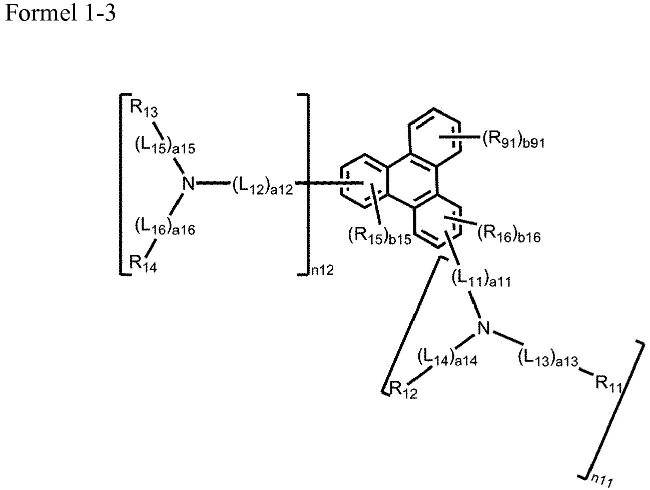 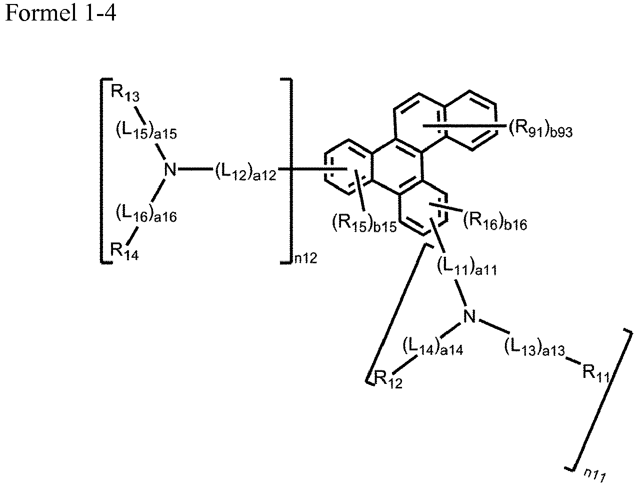 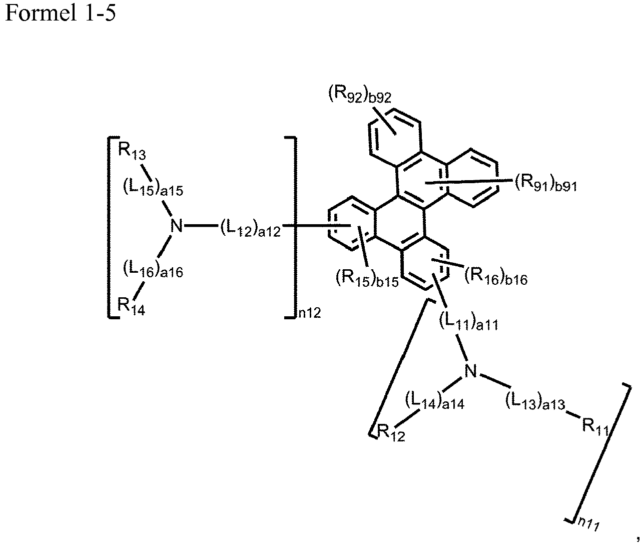
wobei in den Formeln 1-1 bis 1-5
L₁₁ bis L₁₆ jeweils unabhängig voneinander aus einer substituierten oder unsubstituierten C₃-C₁₀-Cycloalkylengruppe, einer substituierten oder unsubstituierten C₁-C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten C₆-C₆₀-Arylengruppe, einer substituierten oder unsubstituierten C₁-C₆₀-Heteroarylengruppe, einer substituierten oder unsubstituierten divalenten nicht-aromatischen kondensierten polycyclischen Gruppe und einer substituierten oder unsubstituierten divalenten nicht-aromatischen kondensierten heteropolycyclischen Gruppe ausgewählt sind;
a11 bis a16 jeweils unabhängig voneinander aus 0, 1, 2 und 3 ausgewählt sind;
R₁₁ bis R₁₄ jeweils unabhängig voneinander aus einer substituierten oder unsubstituierten C₆-C₆₀-Arylgruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Gruppe ausgewählt sind;
n11 und n12 jeweils unabhängig voneinander aus 0, 1 und 2 ausgewählt sind, wobei die Summe von n11 und n12 aus 1, 2, 3 und 4 ausgewählt ist;
R₁₅, R₁₆, R₉₁ und R₉₂ jeweils unabhängig voneinander aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer substituierten oder unsubstituierten C₁-C₆₀-Alkylgruppe, einer substituierten oder unsubstituierten C₁-C₆₀-Alkoxygruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe, einer substituierten oder unsubstituierten monovalenten nicht-aromatischen kondensierten heteropolycyclischen Gruppe und -N(Q₁)(Q₂) ausgewählt sind;
wobei Q₁ und Q₂ jeweils unabhängig voneinander ausgewählt sind aus:
einer C₆-C₆₀-Arylgruppe und einer monovalenten nicht-aromatischen kondensierten polycyclischen Gruppe; und
einer C₆-C₆₀-Arylgruppe und einer monovalenten nicht-aromatischen kondensierten polycyclischen Gruppe, jeweils substituiert mit einer C₆-C₆₀-Arylgruppe;
b15 und b16 jeweils unabhängig voneinander aus 1, 2, 3 und 4 ausgewählt sind;
b91 und b92 jeweils unabhängig voneinander aus 1, 2, 3 und 4 ausgewählt sind; und
b93 aus 1, 2, 3, 4, 5 und 6 ausgewählt ist.

7. Die organische lichtemittierende Vorrichtung nach Anspruch 1, wobei die Verbindung auf Aminbasis aus den Verbindungen 1 bis 48 ausgewählt ist:

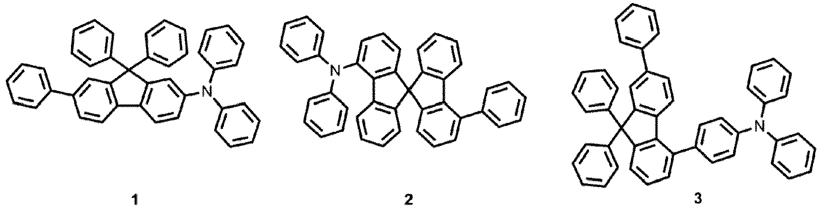

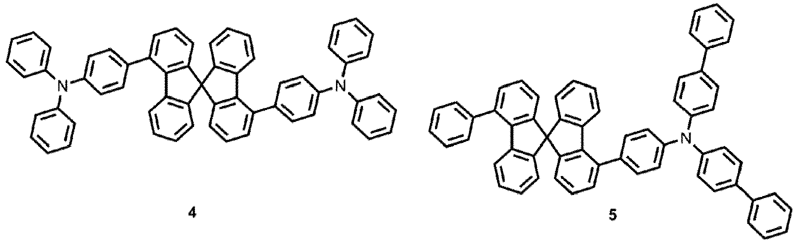

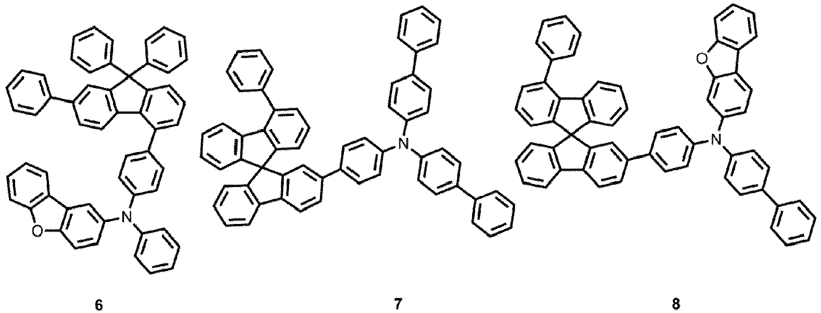

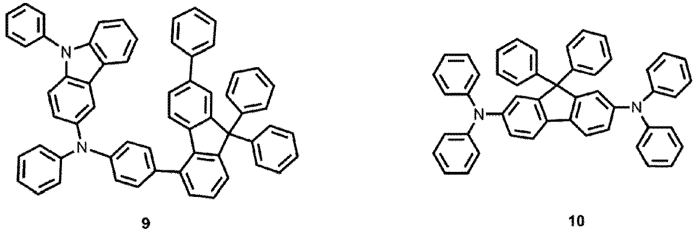

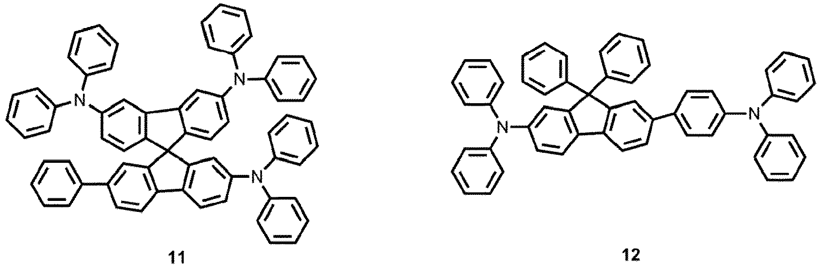

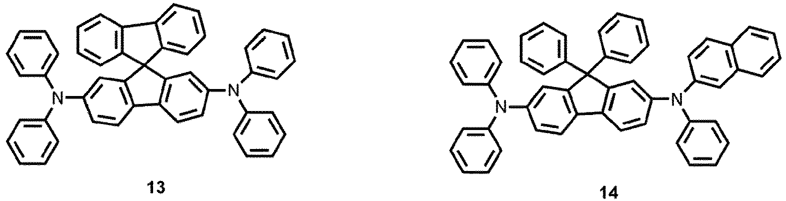

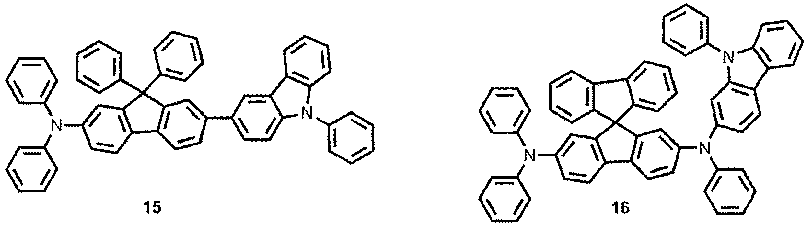

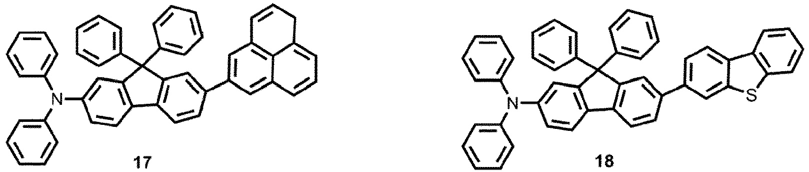

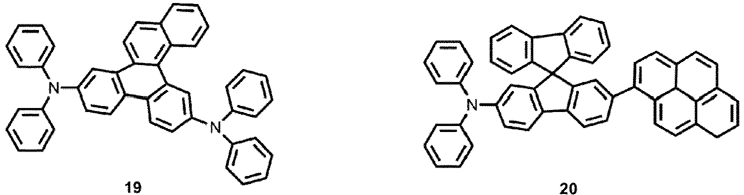

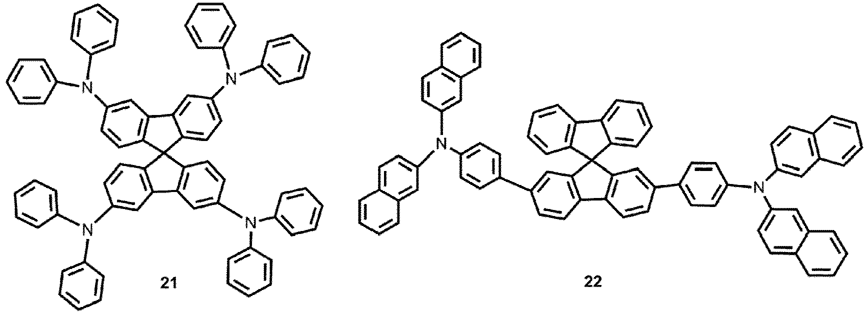

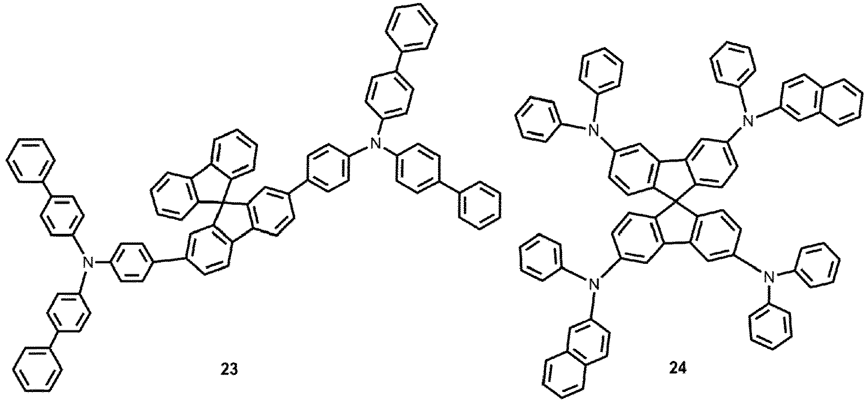

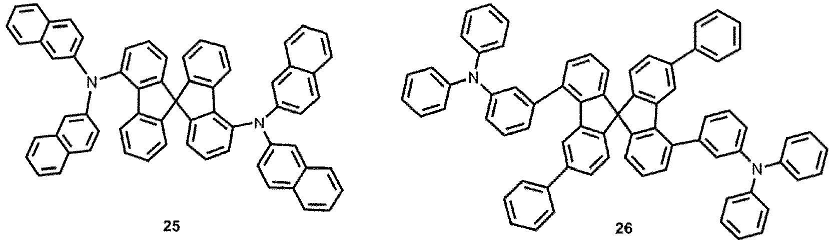

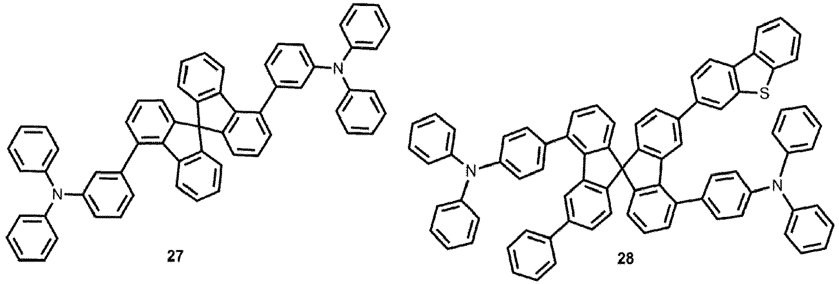

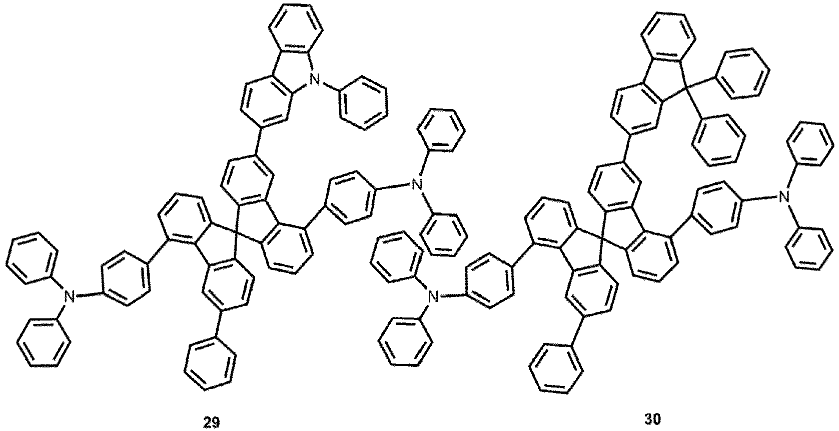

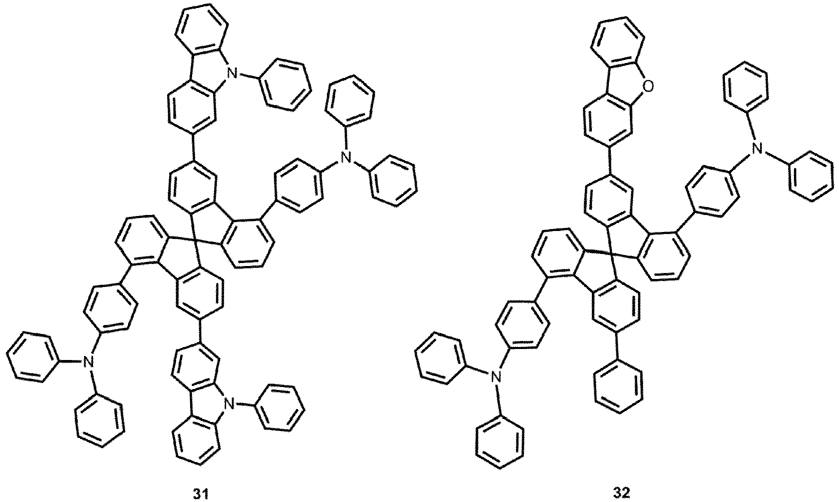

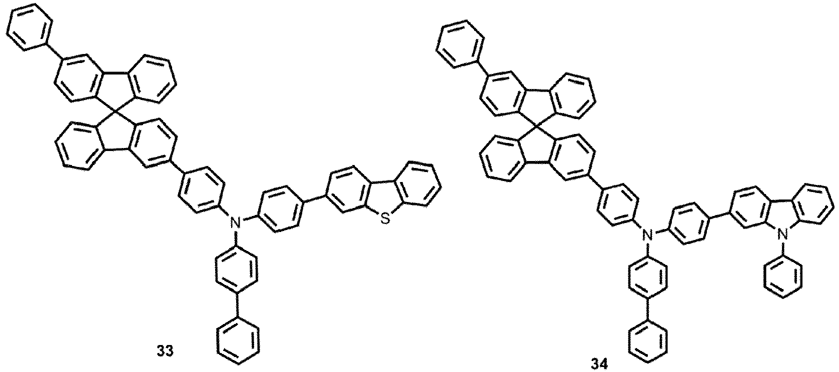

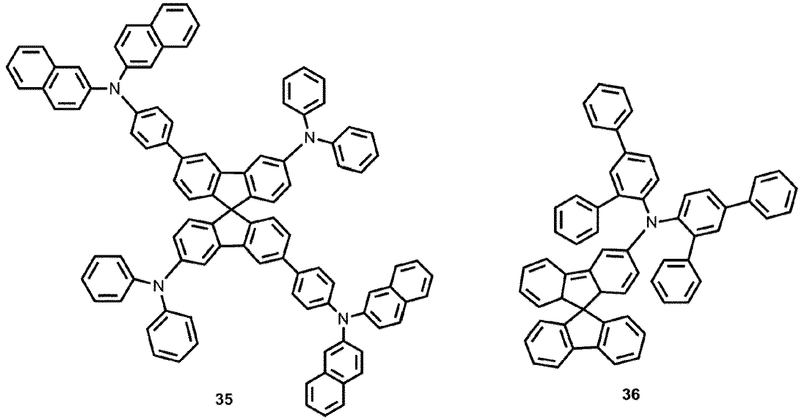

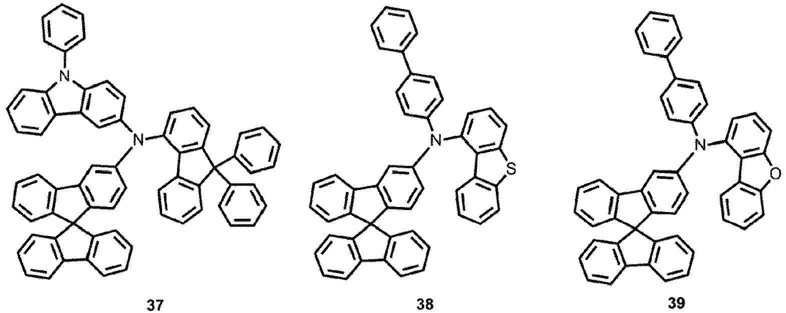

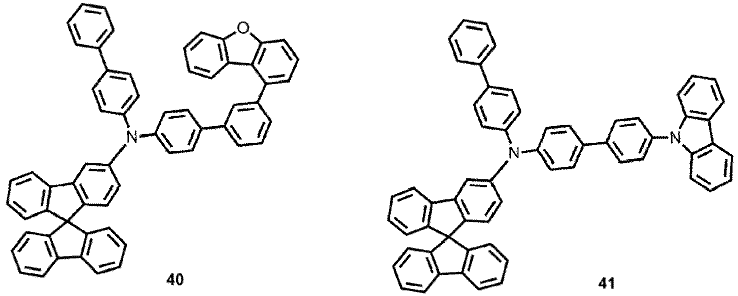

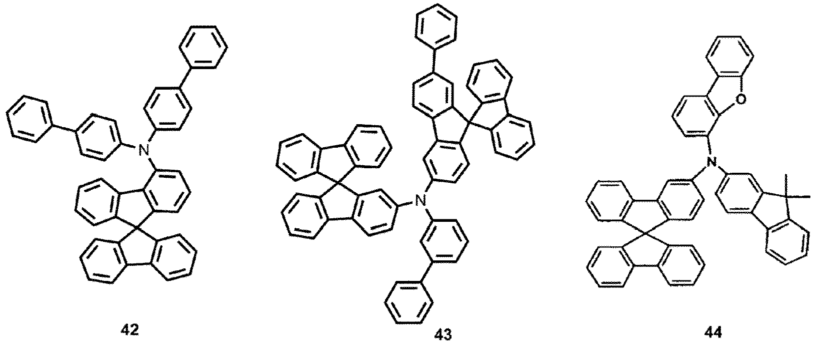

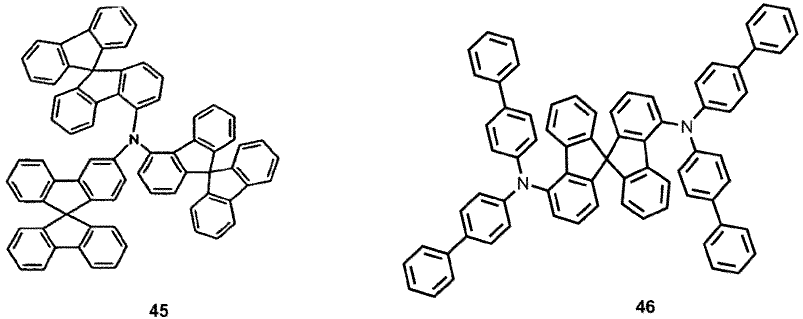

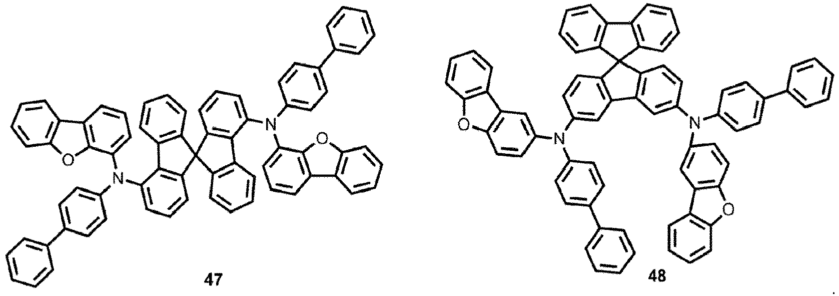

8. Die organische lichtemittierende Vorrichtung nach Anspruch 1, wobei die erste lochtransportierende Verbindung durch eine der Verbindungen HT1 bis HT20 dargestellt ist:

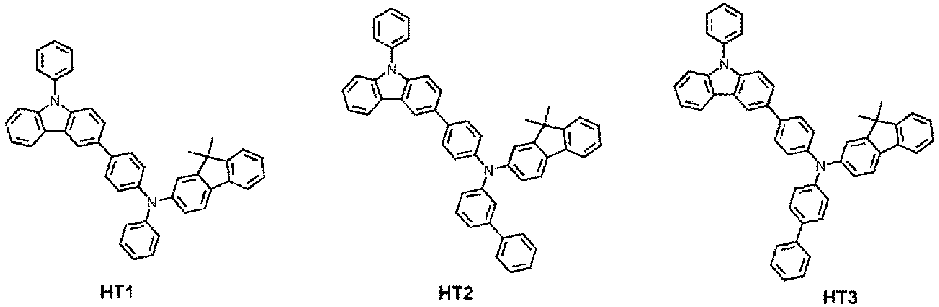

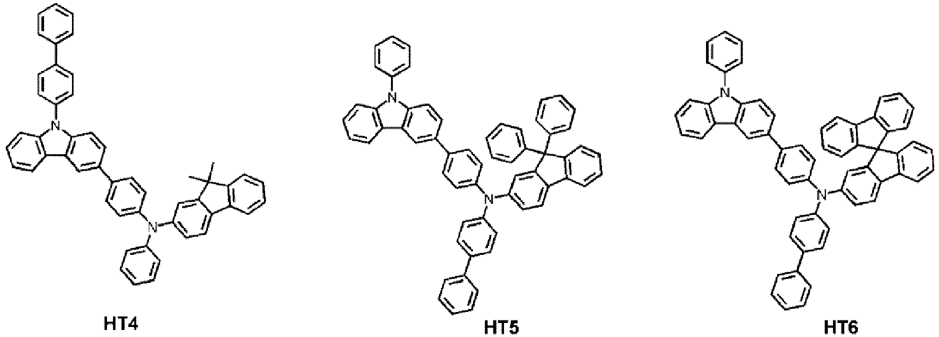

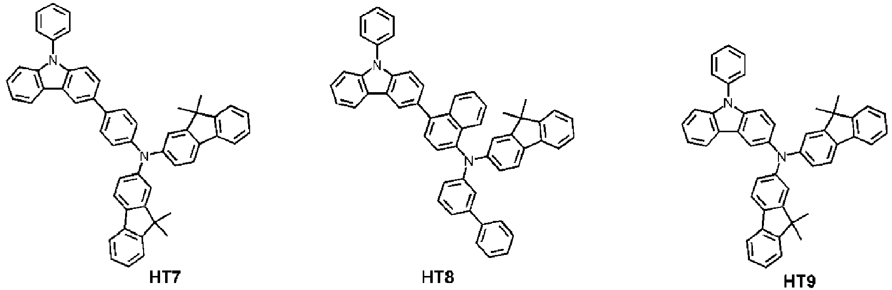

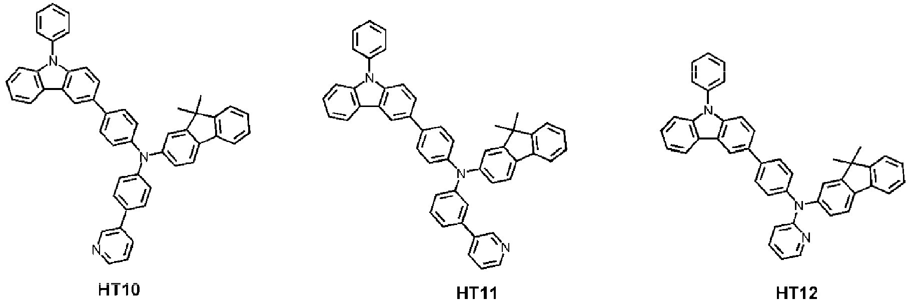

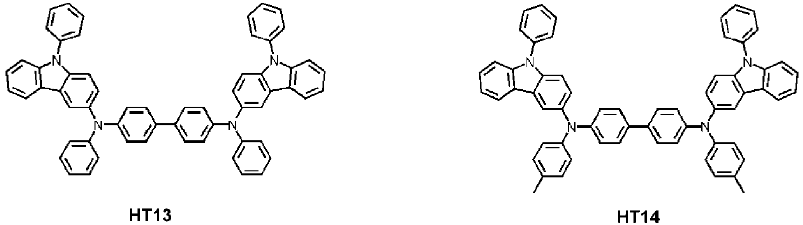

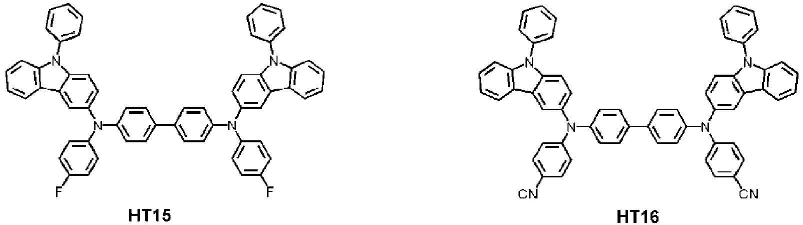

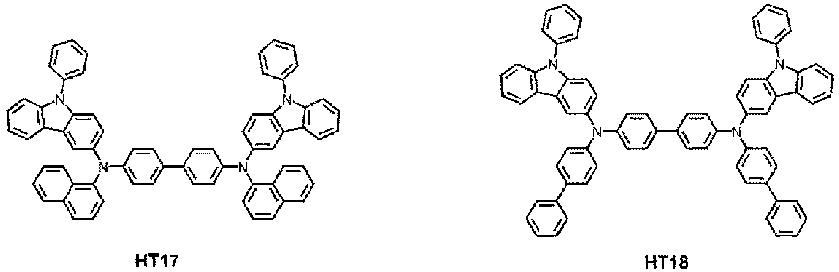

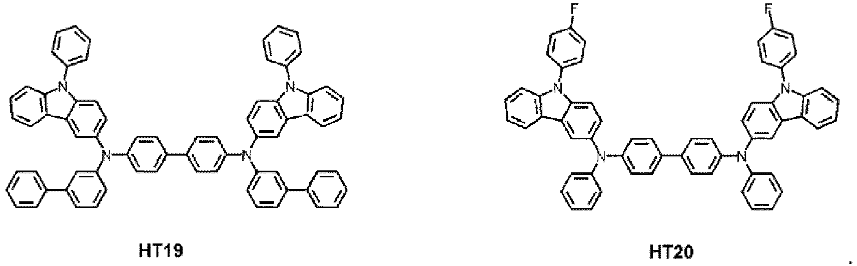

## Revendications

1. Dispositif luminescent organique comprenant :
une première électrode (110) ;
une deuxième électrode (190) ;
une couche d'émission (160) entre la première électrode (110) et la deuxième électrode (190) ; et
une région de transport de trous (130) entre la première électrode (110) et la couche d'émission (160), la région de transport de trous (130) comprenant une couche d'injection de trous, une couche de transport de trous et une couche auxiliaire (140), les couches étant empilées en séquence à partir de la première électrode (110) dans l'ordre indiqué, et la couche d'émission (160) et la couche auxiliaire (140) étant adjacentes l'une à l'autre, et
dans lequel la couche auxiliaire comprend (150) un composé à base d'amine représenté par la formule 1 : 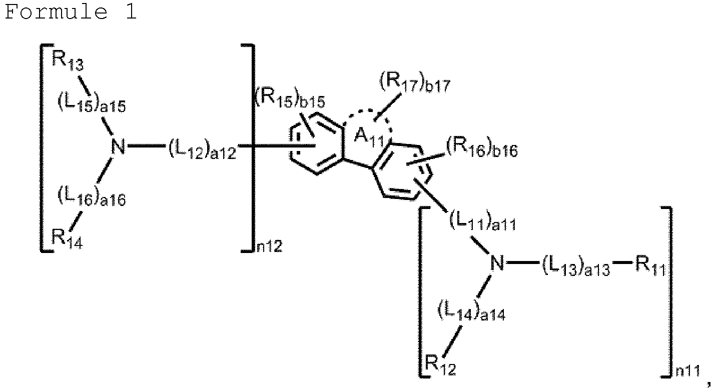
où, dans la formule 1,
A₁₁ est choisi parmi un cycloalcane en C₃ à C₁₀, un hétérocycloalcane en C₁ à C₁₀, un cycloalcène en C₃ à C₁₀, un hétérocycloalcène en C₁ à C₁₀, un arène en C₆ à C₆₀, un hétéroarène en C₁ à C₆₀, un groupe hydrocarboné polycyclique condensé non aromatique, et un groupe hydrocarboné hétéropolycyclique condensé non aromatique ;
chacun de L₁₁ à L₁₆ est indépendamment choisi parmi un groupe cycloalkylène en C₃ à C₁₀ substitué ou non substitué, un groupe hétérocycloalkylène en C₁ à C₁₀ substitué ou non substitué, un groupe cycloalcénylène en C₃ à C₁₀ substitué ou non substitué, un groupe hétérocycloalcénylène en C₁ à C₁₀ substitué ou non substitué, un groupe arylène en C₆ à C₆₀ substitué ou non substitué, un groupe hétéroarylène en C₁ à C₆₀ substitué ou non substitué, un groupe polycyclique condensé non aromatique divalent substitué ou non substitué, et un groupe hétéropolycyclique condensé non aromatique divalent substitué ou non substitué ;
chacun de a11 à a16 est indépendamment choisi parmi 0, 1, 2 et 3 ;
chacun de R₁₁ à R₁₄ est indépendamment choisi parmi un groupe aryle en C₆ à C₆₀ substitué ou non substitué, un groupe hétéroaryle en C₁ à C₆₀ substitué ou non substitué, un groupe polycyclique condensé non aromatique monovalent substitué ou non substitué, et un groupe hétéropolycyclique condensé non aromatique monovalent substitué ou non substitué ;
chacun de n11 et n12 est indépendamment choisi parmi 0, 1 et 2, et la somme de n11 et n12 est choisie parmi 1, 2, 3 et 4 ;
chacun de R₁₅ à R₁₇ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un de ses sels, un acide sulfonique ou un de ses sels, un acide phosphorique ou un de ses sels, un groupe alkyle en C₁ à C₆₀ substitué ou non substitué, un groupe alkoxy en C₁ à C₆₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), et -Si(Q₃)(Q₄)(Q₅) ;
chacun de b15 à b17 est indépendamment choisi parmi 1, 2, 3 et 4 ;
au moins un substituant du groupe cycloalkylène en C₃ à C₁₀ substitué, du groupe hétérocycloalkylène en C₁ à C₁₀ substitué, du groupe cycloalcénylène en C₃ à C₁₀ substitué, du groupe hétérocycloalcénylène en C₁ à C₁₀ substitué, du groupe arylène en C₆ à C₆₀ substitué, du groupe hétéroarylène en C₁ à C₆₀ substitué, du groupe polycyclique condensé non aromatique divalent substitué, du groupe hétéropolycyclique condensé non aromatique divalent substitué, du groupe alkyle en C₁ à C₆₀ substitué, du groupe alkoxy en C₁ à C₆₀ substitué, du groupe aryle en C₆ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe polycyclique condensé non aromatique monovalent substitué, et du groupe hétéropolycyclique condensé non aromatique monovalent substitué, est choisi parmi :
le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₆₀, un groupe alcényle en C₂ à C₆₀, un groupe alcynyle en C₂ à C₆₀, et un groupe alkoxy en C₁ à C₆₀ ;
un groupe alkyle en C₁ à C₆₀, un groupe alcényle en C₂ à C₆₀, un groupe alcynyle en C₂ à C₆₀, et un groupe alkoxy en C₁ à C₆₀, chacun substitué par au moins un substituant choisi parmi le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, un groupe hétéropolycyclique condensé non aromatique monovalent, -N(Q₁₁)(Q₁₂) et -Si (Q₁₃)(Q₁₄)(Q₁₅) ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent, chacun substitué par au moins un substituant choisi parmi le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₆₀, un groupe alcényle en C₂ à C₆₀, un groupe alcynyle en C₂ à C₆₀, un groupe alkoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, un groupe hétéropolycyclique condensé non aromatique monovalent, -N(Q₂₁)(Q₂₂), et -Si(Q₂₃)(Q₂₄)(Q₂₅) ; et
-N(Q₃₁)(Q₃₂) et -Si(Q₃₃)(Q₃₄)(Q₃₅) ;
où chacun de Q₁ à Q₅, Q₁₁ à Q₁₅, Q₂₁ à Q₂₅, et Q₃₁ à Q₃₅ est indépendamment choisi parmi :
un groupe alkyle en C₁ à C₆₀, un groupe aryle en C₆ à C₆₀, un groupe hétéroaryle en C₁ à C₆₀, un groupe polycyclique condensé non aromatique monovalent, et un groupe hétéropolycyclique condensé non aromatique monovalent ; et
un groupe aryle en C₆ à C₆₀, un groupe hétéroaryle en C₁ à C₆₀, un groupe polycyclique condensé non aromatique monovalent, et un groupe hétéropolycyclique condensé non aromatique monovalent, chacun substitué par un groupe aryle en C₆ à C₆₀ ; et
dans lequel la couche de transport de trous est constituée d'un premier composé de transport de trous représenté par l'une quelconque des formules 201A et 202A : 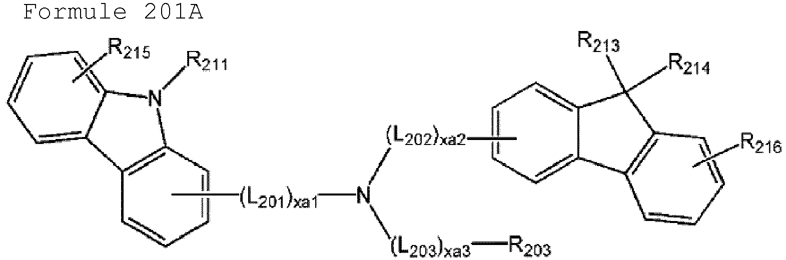 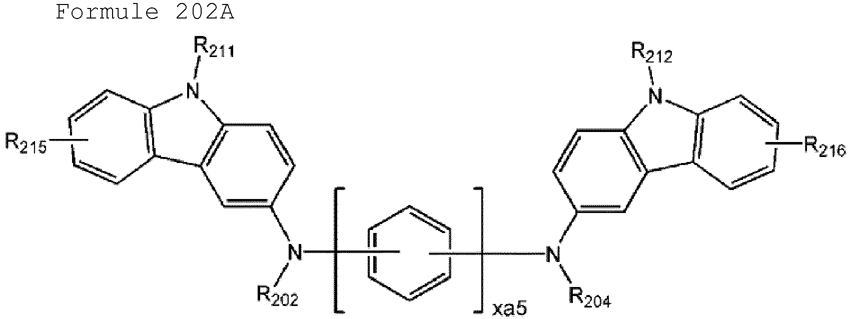
formules dans lesquelles :
L₂₀₁ à L₂₀₃ sont tels que définis en relation avec L₁₁ ; xa1 à xa3 sont indépendamment choisis parmi 0, 1, 2 et 3 ; xa5 est choisi parmi 1, 2, 3, 4 et 5 ; R₂₀₂ à R₂₀₄ ont la même définition que celle indiquée en relation avec R₁₁ ; chacun de R₂₁₁ et R₂₁₂ a indépendamment la même définition que celle indiquée en relation avec R₂₀₃ ci-dessus, et
chacun de R₂₁₃ à R₂₁₆ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazine, un groupe hydrazone, un groupe carboxyle ou un de ses sels, un acide sulfonique ou un de ses sels, un acide phosphorique ou un de ses sels, un groupe alkyle en C₁ à C₆₀, un groupe alcényle en C₂ à C₆₀, un groupe alcynyle en C₂ à C₆₀, un groupe alkoxy en C₁ à C₆₀, un groupe cycloalkyle en C₃ à C₁₀, un groupe hétérocycloalkyle en C₁ à C₁₀, un groupe cyclo-alcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent.

2. Dispositif luminescent organique selon la revendication 1, dans lequel A₁₁ est représenté par l'une quelconque des formules 9-1 à 9-5 : 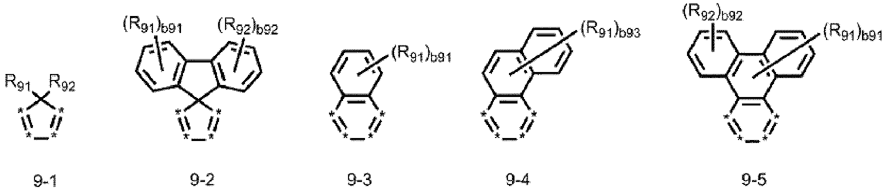
où, dans les formules 9-1 à 9-5,
* est un atome de carbone dans la formule 1 ;
chacun de R₉₁ et R₉₂ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₆₀ substitué ou non substitué, un groupe alkoxy en C₁ à C₆₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, et -N(Q₁)(Q₂),
où chacun de Q₁ et Q₂ est indépendamment choisi parmi :
un groupe aryle en C₆ à C₆₀ et un groupe polycyclique condensé non aromatique monovalent ; et
un groupe aryle en C₆ à C₆₀ et un groupe polycyclique condensé non aromatique monovalent, chacun substitué par un groupe aryle en C₆ à C₆₀ ;
chacun de b91 et b92 est indépendamment choisi parmi 1, 2, 3 et 4 ; et
b93 est choisi parmi 1, 2, 3, 4, 5 et 6.

3. Dispositif luminescent organique selon la revendication 1, dans lequel chacun de L₁₁ à L₁₆ est indépendamment choisi parmi :
un groupe phénylène, un groupe pentalénylène, un groupe indénylène, un groupe naphtylène, un groupe azulénylène, un groupe heptalénylène, un groupe indacénylène, un groupe acénaphtylène, un groupe fluorénylène, un groupe spirofluorénylène, un groupe benzofluorénylène, un groupe dibenzofluorénylène, un groupe phénalénylène, un groupe phénanthrénylène, un groupe anthracénylène, un groupe fluoranthénylène, un groupe triphénylénylène, un groupe pyrénylène, un groupe chrysénylène, un groupe naphtacénylène, un groupe picénylène, un groupe pérylénylène, un groupe pentaphénylène, un groupe hexacénylène, un groupe pentacénylène, un groupe rubicénylène, un groupe coronénylène, un groupe ovalénylène, un groupe pyrrolylène, un groupe thiophénylène, un groupe furanylène, un groupe imidazolylène, un groupe pyrazolylène, un groupe thiazolylène, un groupe isothiazolylène, un groupe oxazolylène, un groupe isoxazolylène, un groupe pyridinylène, un groupe pyrazinylène, un groupe pyrimidinylène, un groupe pyridazinylène, un groupe isoindolylène, un groupe indolylène, un groupe indazolylène, un groupe purinylène, un groupe quinolinylène, un groupe isoquinolinylène, un groupe benzoquinolinylène, un groupe phtalazinylène, un groupe naphtyridinylène, un groupe quinoxalinylène, un groupe quinazolinylène, un groupe cinnolinylène, un groupe carbazolylène, un groupe phénanthridinylène, un groupe acridinylène, un groupe phénanthrolinylène, un groupe phénazinylène, un groupe benzimidazolylène, un groupe benzofuranylène, un groupe benzothiophénylène, un groupe isobenzothiazolylène, un groupe benzoxazolylène, un groupe isobenzoxazolylène, un groupe triazolylène, un groupe tétrazolylène, un groupe oxadiazolylène, un groupe triazinylène, un groupe dibenzofuranylène, un groupe dibenzothiophénylène, un groupe benzocarbazolylène, et un groupe dibenzocarbazolylène ; et
un groupe phénylène, un groupe pentalénylène, un groupe indénylène, un groupe naphtylène, un groupe azulénylène, un groupe heptalénylène, un groupe indacénylène, un groupe acénaphtylène, un groupe fluorénylène, un groupe spirofluorénylène, un groupe benzofluorénylène, un groupe dibenzofluorénylène, un groupe phénalénylène, un groupe phénanthrénylène, un groupe anthracénylène, un groupe fluoranthénylène, un groupe triphénylénylène, un groupe pyrénylène, un groupe chrysénylène, un groupe naphtacénylène, un groupe picénylène, un groupe pérylénylène, un groupe pentaphénylène, un groupe hexacénylène, un groupe pentacénylène, un groupe rubicénylène, un groupe coronénylène, un groupe ovalénylène, un groupe pyrrolylène, un groupe thiophénylène, un groupe furanylène, un groupe imidazolylène, un groupe pyrazolylène, un groupe thiazolylène, un groupe isothiazolylène, un groupe oxazolylène, un groupe isoxazolylène, un groupe pyridinylène, un groupe pyrazinylène, un groupe pyrimidinylène, un groupe pyridazinylène, un groupe isoindolylène, un groupe indolylène, un groupe indazolylène, un groupe purinylène, un groupe quinolinylène, un groupe isoquinolinylène, un groupe benzoquinolinylène, un groupe phtalazinylène, un groupe naphtyridinylène, un groupe quinoxalinylène, un groupe quinazolinylène, un groupe cinnolinylène, un groupe carbazolylène, un groupe phénanthridinylène, un groupe acridinylène, un groupe phénanthrolinylène, un groupe phénazinylène, un groupe benzimidazolylène, un groupe benzofuranylène, un groupe benzothiophénylène, un groupe isobenzothiazolylène, un groupe benzoxazolylène, un groupe isobenzoxazolylène, un groupe triazolylène, un groupe tétrazolylène, un groupe oxadiazolylène, un groupe triazinylène, un groupe dibenzofuranylène, un groupe dibenzothiophénylène, un groupe benzocarbazolylène, et un groupe dibenzocarbazolylène, chacun substitué par au moins un substituant choisi parmi le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₂₀, un groupe alkoxy en C₁ à C₂₀, un groupe cyclopentyle, un groupe cyclohexyle, un groupe cycloheptyle, un groupe cyclopentényle, un groupe cyclohexényle, un groupe phényle, un groupe pentalényle, un groupe indényle, un groupe naphtyle, un groupe azulényle, un groupe heptalényle, un groupe indacényle, un groupe acénaphtyle, un groupe fluorényle, un groupe spirofluorényle, un groupe benzofluorényle, un groupe dibenzofluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe pérylényle, un groupe pentaphényle, un groupe hexacényle, un groupe pentacényle, un groupe rubicényle, un groupe coronényle, un groupe ovalényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe thiadiazolyle, et un groupe imidazopyridinyle.

4. Dispositif luminescent organique selon la revendication 1, dans lequel chacun de R₁₁ à R₁₄ est indépendamment choisi parmi :
un groupe phényle, un groupe pentalényle, un groupe indényle, un groupe naphtyle, un groupe azulényle, un groupe heptalényle, un groupe indacényle, un groupe acénaphtyle, un groupe fluorényle, un groupe spirofluorényle, un groupe benzofluorényle, un groupe dibenzofluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe pérylényle, un groupe pentaphényle, un groupe hexacényle, un groupe pentacényle, un groupe rubicényle, un groupe coronényle, un groupe ovalényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe carbazolyle, un groupe benzoquinolinyle, un groupe phtalazinyle, un groupe naphtyridinyle, un groupe quinoxalinyle, un groupe benzoquinoxalinyle, un groupe quinazolinyle, un groupe benzoquinazolinyle, un groupe cinnolinyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe thiadiazolyle, un groupe imidazopyridinyle, et un groupe imidazopyrimidinyle ; et
un groupe phényle, un groupe pentalényle, un groupe indényle, un groupe naphtyle, un groupe azulényle, un groupe heptalényle, un groupe indacényle, un groupe acénaphtyle, un groupe fluorényle, un groupe spirofluorényle, un groupe benzofluorényle, un groupe dibenzofluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe pérylényle, un groupe pentaphényle, un groupe hexacényle, un groupe pentacényle, un groupe rubicényle, un groupe coronényle, un groupe ovalényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe carbazolyle, un groupe benzoquinolinyle, un groupe phtalazinyle, un groupe naphtyridinyle, un groupe quinoxalinyle, un groupe benzoquinoxalinyle, un groupe quinazolinyle, un groupe benzoquinazolinyle, un groupe cinnolinyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe thiadiazolyle, un groupe imidazopyridinyle, et un groupe imidazopyrimidinyle, chacun substitué par au moins un substituant choisi parmi le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₂₀, un groupe alkoxy en C₁ à C₂₀, un groupe phényle, un groupe biphényle, un groupe pentalényle, un groupe indényle, un groupe naphtyle, un groupe azulényle, un groupe heptalényle, un groupe indacényle, un groupe acénaphtyle, un groupe fluorényle, un groupe spirofluorényle, un groupe benzofluorényle, un groupe dibenzofluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe pérylényle, un groupe pentaphényle, un groupe hexacényle, un groupe pentacényle, un groupe rubicényle, un groupe coronényle, un groupe ovalényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe carbazolyle, un groupe benzoquinolinyle, un groupe phtalazinyle, un groupe naphtyridinyle, un groupe quinoxalinyle, un groupe benzoquinoxalinyle, un groupe quinazolinyle, un groupe benzoquinazolinyle, un groupe cinnolinyle, un groupe carbazolyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe thiadiazolyle, un groupe imidazopyridinyle, et un groupe imidazopyrimidinyle.

5. Dispositif luminescent organique selon la revendication 1, dans lequel chacun de R₁₅ à R₁₇ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₆₀ substitué ou non substitué, un groupe alkoxy en C₁ à C₆₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, et -N(Q₁)(Q₂) ;
où chacun de Q₁ et Q₂ sont indépendamment choisis parmi un groupe aryle en C₆ à C₆₀ et un groupe polycyclique condensé non aromatique monovalent ; et
un groupe aryle en C₆ à C₆₀ et un groupe polycyclique condensé non aromatique monovalent, chacun substitué par un groupe aryle en C₆ à C₆₀.

6. Dispositif luminescent organique selon la revendication 1, dans lequel le composé à base d'amine est représenté par l'une quelconque des formules 1-1 à 1-5 : 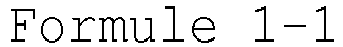 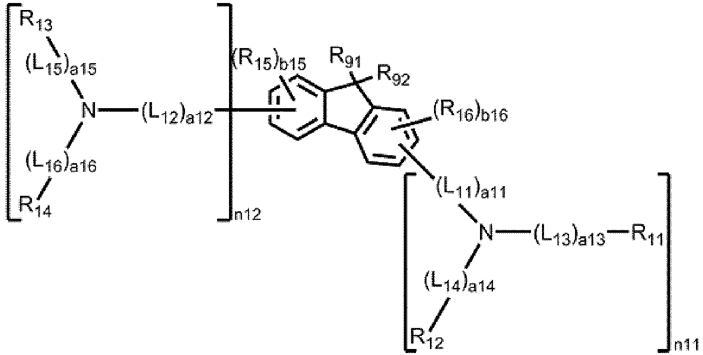 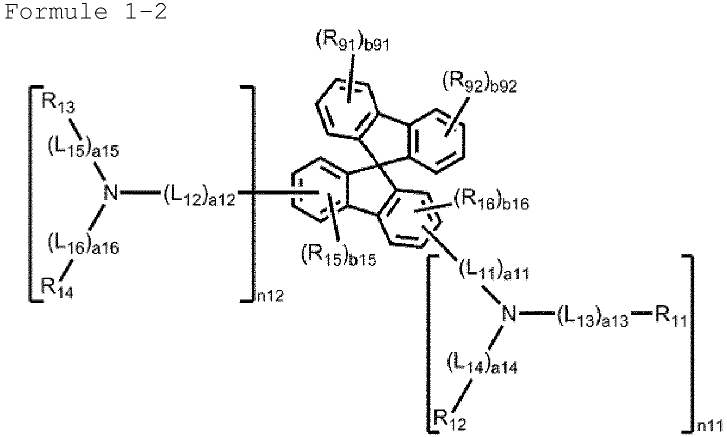 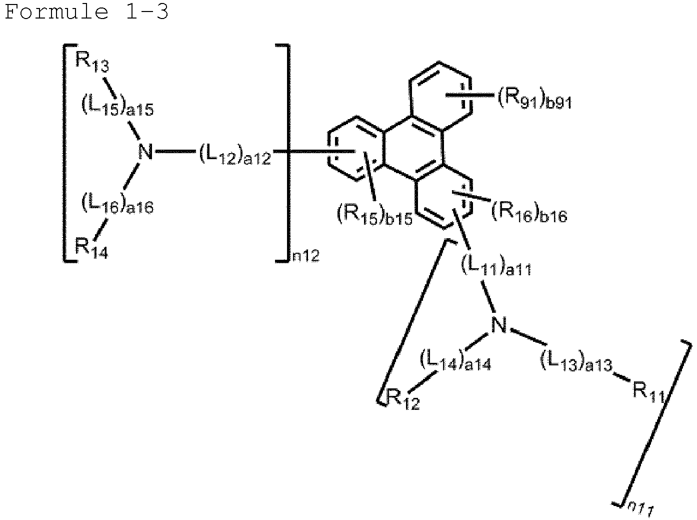 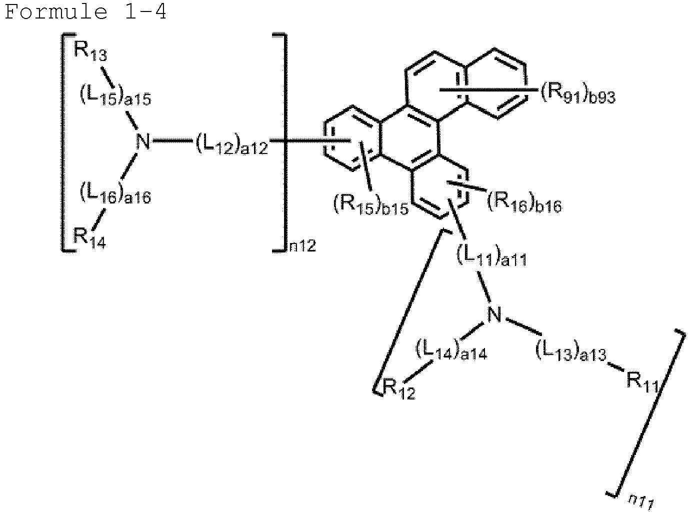 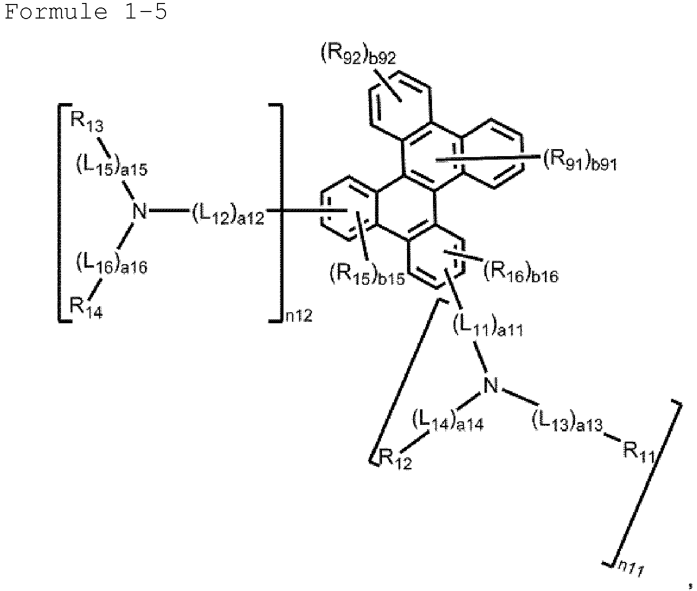
où, dans les formules 1-1 à 1,5,
chacun de L₁₁ à L₁₆ est indépendamment choisi parmi un groupe cycloalkylène en C₃ à C₁₀ substitué ou non substitué, un groupe hétérocycloalkylène en C₁ à C₁₀ substitué ou non substitué, un groupe cycloalcénylène en C₃ à C₁₀ substitué ou non substitué, un groupe hétérocycloalcénylène en C₁ à C₁₀ substitué ou non substitué, un groupe arylène en C₆ à C₆₀ substitué ou non substitué, un groupe hétéroarylène en C₁ à C₆₀ substitué ou non substitué, un groupe polycyclique condensé non aromatique divalent substitué ou non substitué, et un groupe hétéropolycyclique condensé non aromatique divalent substitué ou non substitué ;
chacun de a11 à a16 est indépendamment choisi parmi 0, 1, 2 et 3 ;
chacun de R₁₁ à R₁₄ est indépendamment choisi parmi un groupe aryle en C₆ à C₆₀ substitué ou non substitué, un groupe hétéroaryle en C₁ à C₆₀ substitué ou non substitué, un groupe polycyclique condensé non aromatique monovalent substitué ou non substitué, et un groupe hétéropolycyclique condensé non aromatique monovalent substitué ou non substitué ;
chacun de n11 et n12 est indépendamment choisi parmi 0, 1 et 2, où la somme de n11 et n12 est choisie parmi 1, 2, 3 et 4 ;
chacun de R₁₅, R₁₆, R₉₁ et R₉₂ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un de ses sels, un acide sulfonique ou un de ses sels, un acide phosphorique ou un de ses sels, un groupe alkyle en C₁ à C₆₀ substitué ou non substitué, un groupe alkoxy en C₁ à C₆₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, et -N(Q₁)(Q₂) ;
où chacun de Q₁ et Q₂ est indépendamment choisi parmi :
un groupe aryle en C₆ à C₆₀ et un groupe polycyclique condensé non aromatique monovalent ; et
un groupe aryle en C₆ à C₆₀ et un groupe polycyclique condensé non aromatique monovalent, chacun substitué par un groupe aryle en C₆ à C₆₀ ;
chacun de b15 et b16 est indépendamment choisi parmi 1, 2, 3 et 4 ;
chacun de b91 et b92 est indépendamment choisi parmi 1, 2, 3 et 4 ; et
b93 est choisi parmi 1, 2, 3, 4, 5 et 6.

7. Dispositif luminescent organique selon la revendication 1, dans lequel le composé à base d'amine est choisi parmi les composés 1 à 48 :

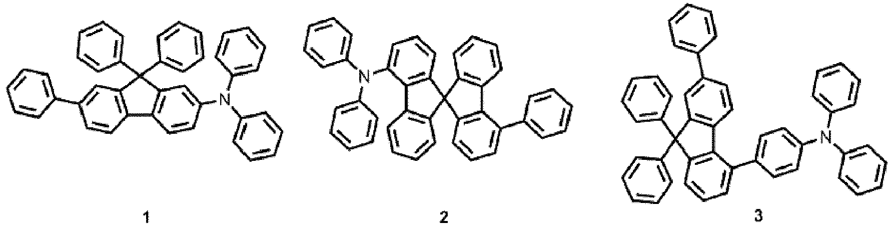

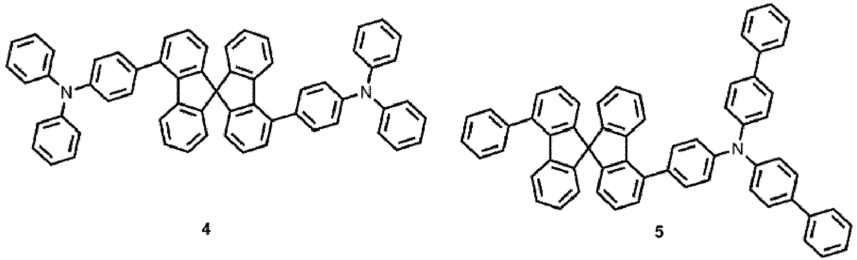

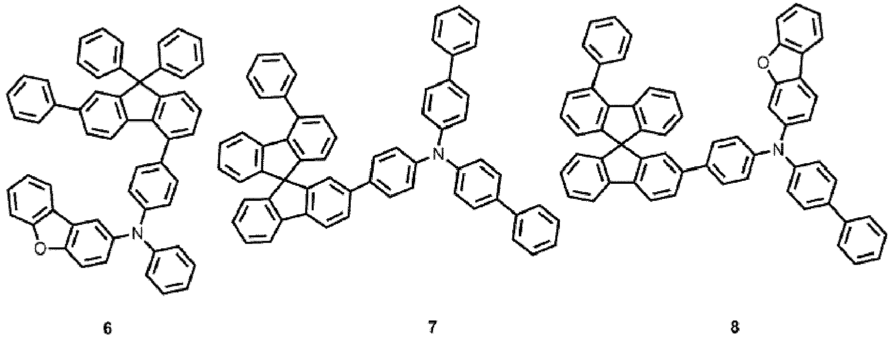

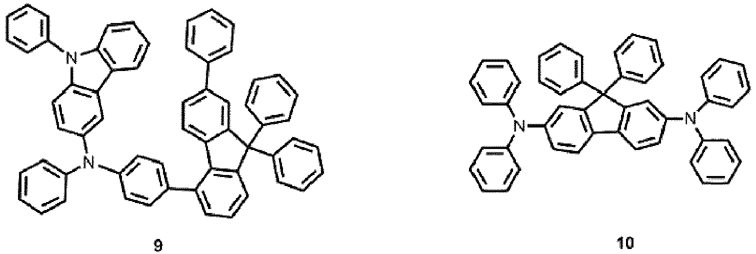

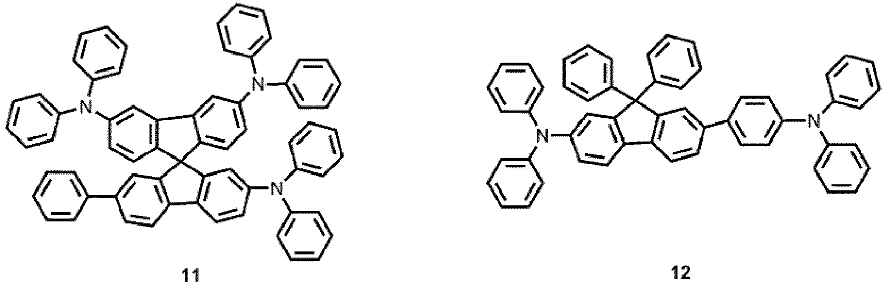

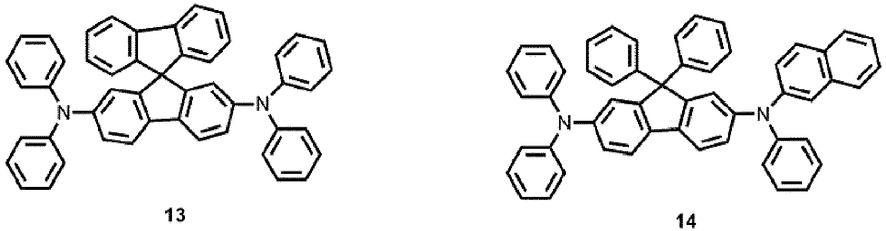

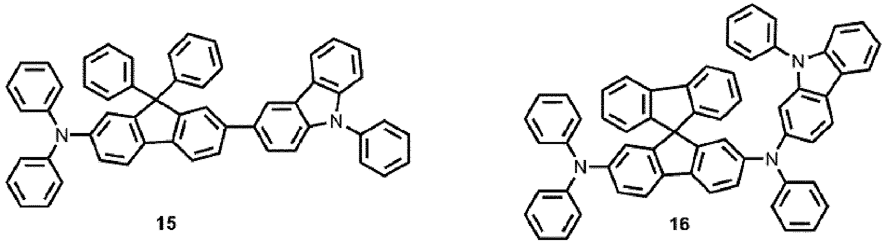

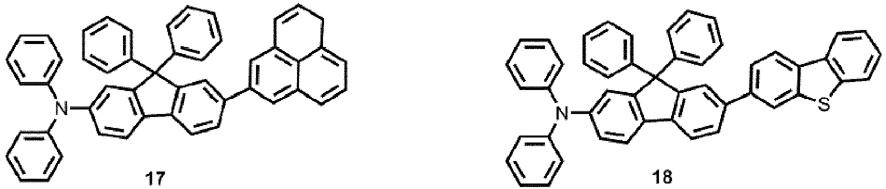

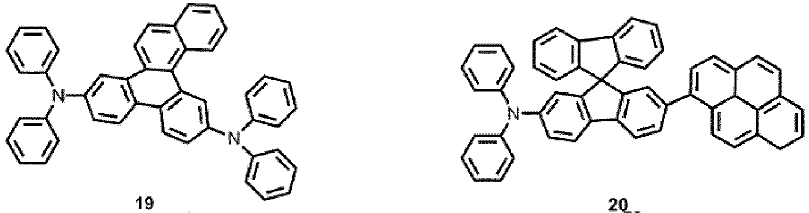

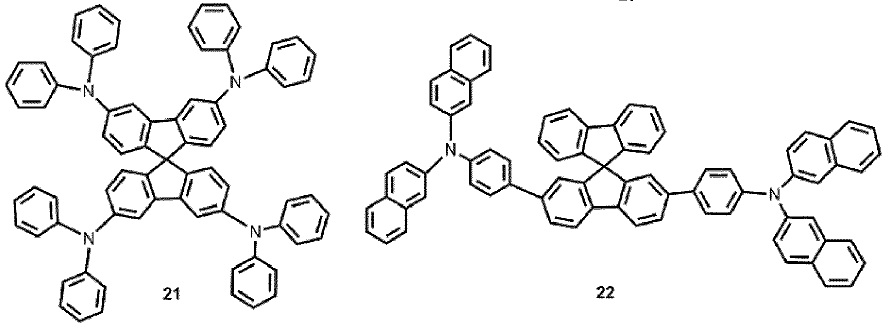

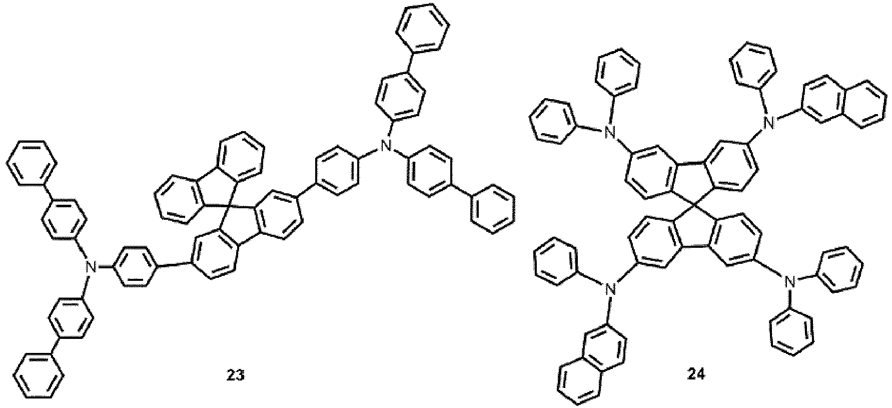

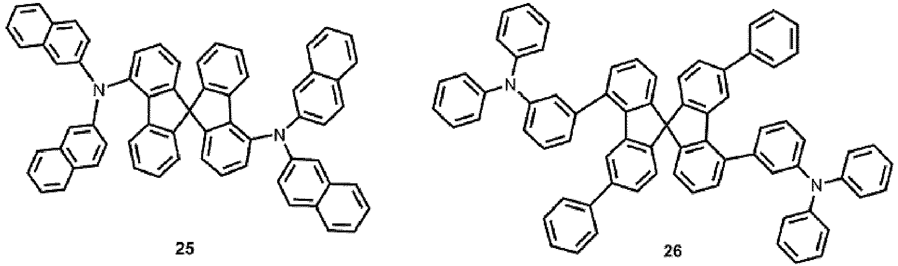

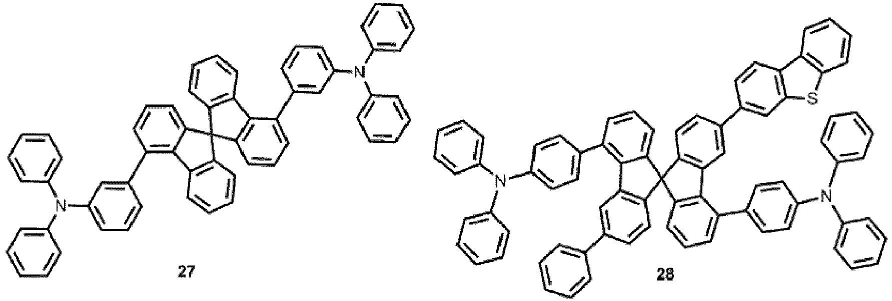

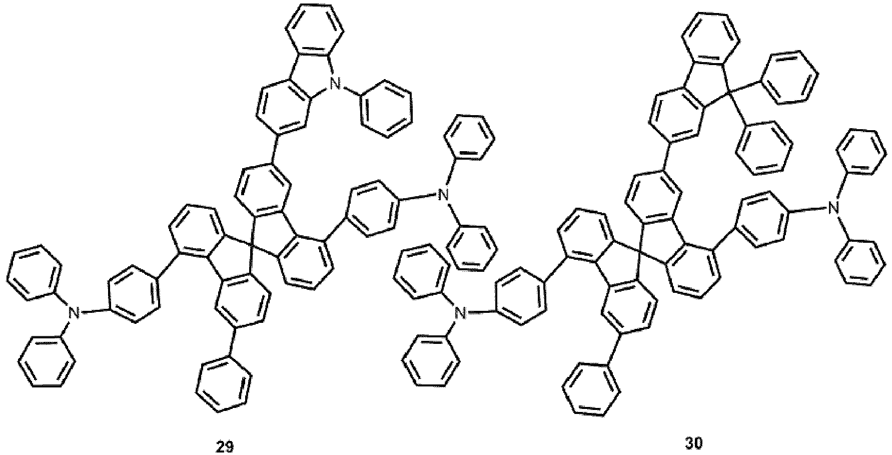

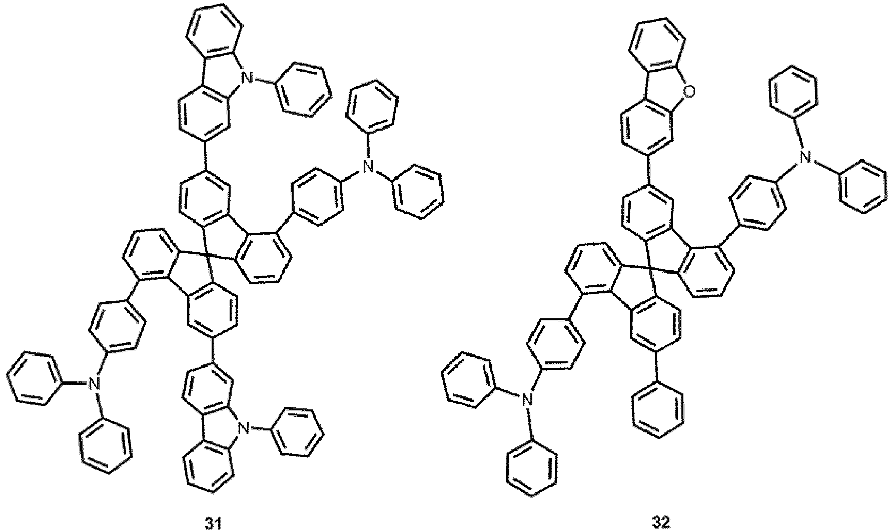

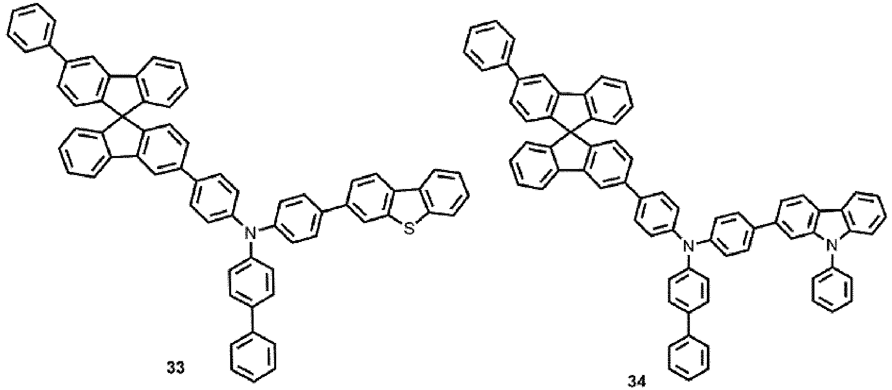

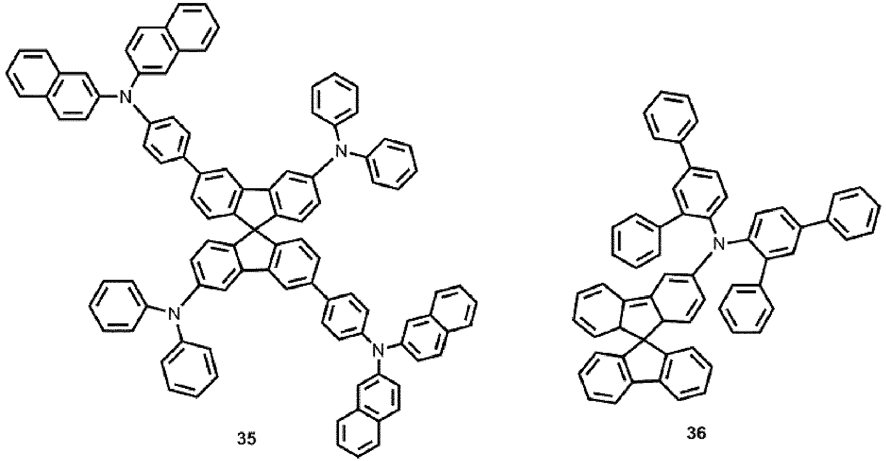

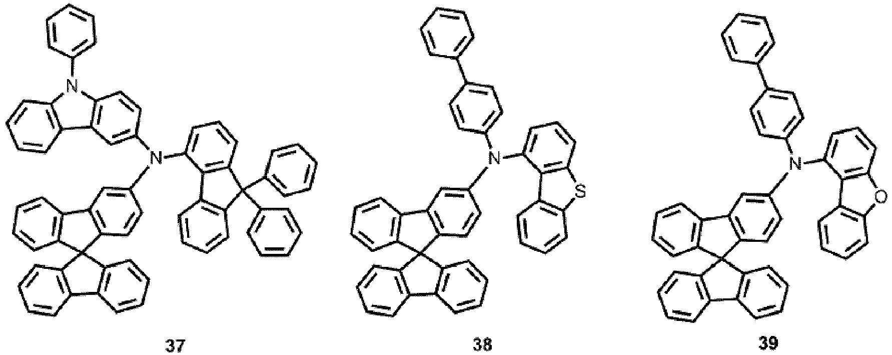

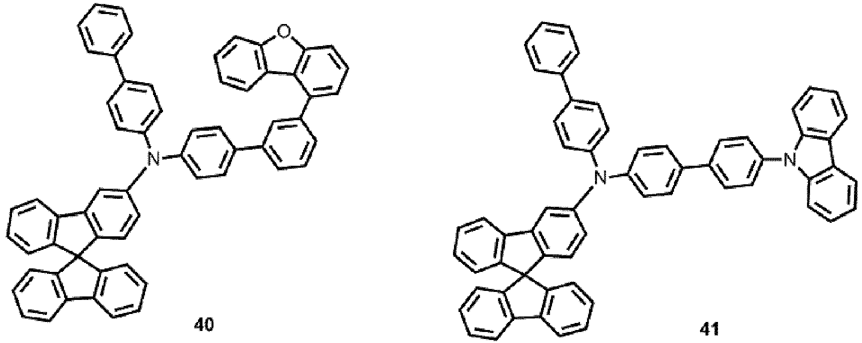

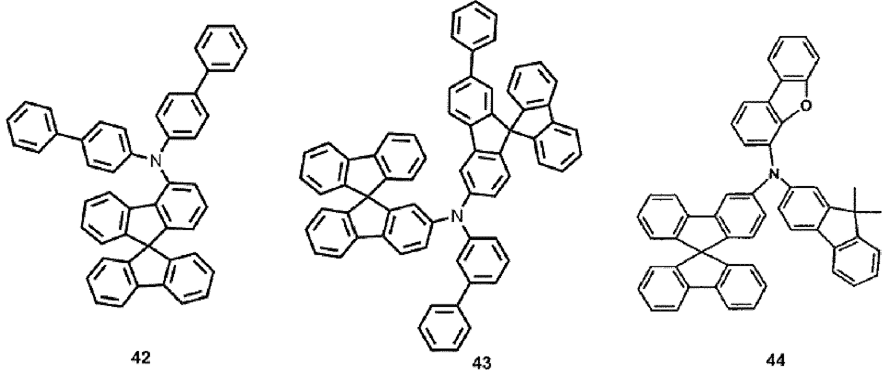

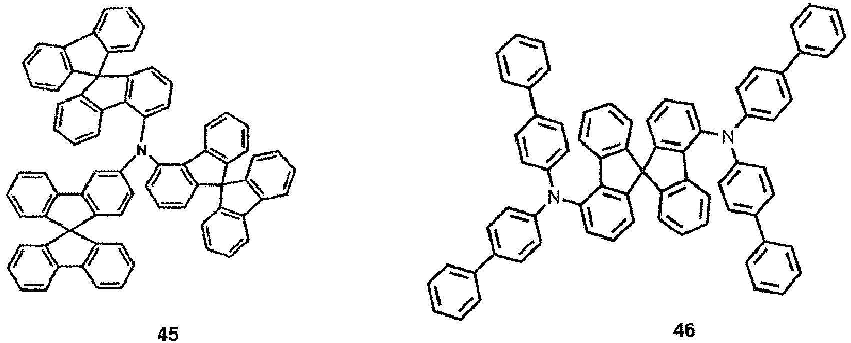

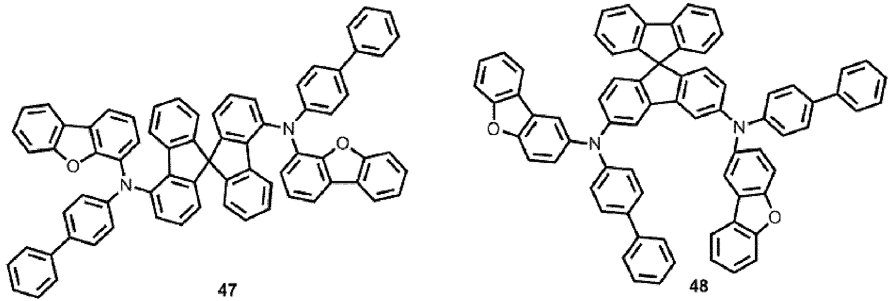

8. Dispositif luminescent organique selon la revendication 1, dans lequel le premier composé de transport de trous est représenté par l'un des composés HT1 à HT20 :
